(19) Europäisches Patentamt
European Patent Office
Office européen des brevets

(11) Publication number: **0 630 890 A2**

(12) # EUROPEAN PATENT APPLICATION

(21) Application number: **94109020.1**

(22) Date of filing: **13.06.94**

(51) Int. Cl.5: **C07D 239/60**, C07D 401/12, C07D 403/12, C07D 405/12, C07D 409/12, A01N 43/54

(30) Priority: **24.06.93 JP 175911/93**

(43) Date of publication of application: **28.12.94 Bulletin 94/52**

(84) Designated Contracting States: **BE CH DE FR GB IT LI NL**

(71) Applicant: **NIHON BAYER AGROCHEM K.K.**
**10-8, Takanawa 4-chome**
**Minato-ku**
**Tokyo 108 (JP)**

(72) Inventor: **Goto, Toshio**
**214-18, Koganei,**
**Kokubuji-machi**
**Shimotsuga-gun,**
**Tochigi (JP)**
Inventor: **Kitagawa, Yoshinori**
**1085, Ara-machi**
**Moka-shi,**
**Tochigi (JP)**
Inventor: **Ito, Seishi**
**1-39-1, Ekihigashidori**
**Oyama-shi,**
**Tochigi (JP)**
Inventor: **Shibuya, Katsuhiko**
**1425-2, Hitotonoya,**
**Oh-aza**
**Oyama-shi,**
**Tochigi (JP)**
Inventor: **Minegishi, Natsuko**
**1-9-31, Wakagi-cho**
**Oyama-shi,**
**Tochigi (JP)**
Inventor: **Ukawa, Kazuhiro**
**1-23-13, Ekihigashidori**
**Oyama-shi,**
**Tochigi (JP)**
Inventor: **Yamaoka, Tatsuya**
**934-7, Hitotonoy,**
**Oh-aza**
**Oyama-shi,**
**Tochigi (JP)**
Inventor: **Ueno, Chieko**
**934-7, Hitotonoya,**
**Oh-aza**
**Oyama-shi,**
**Tochigi (JP)**
Inventor: **Kyo, Yoshiko**
**934-7, Hitotonoya,**
**Oh-aza**
**Oyama-shi,**
**Tochigi (JP)**

(74) Representative: **Linkenheil, Dieter et al**
**Bayer AG**
**Patente Konzernzentrale RP**
**D-51368 Leverkusen (DE)**

(54) **Pyrimidine derivatives.**

(57) Novel pyrimidine derivatives containing a cyano group of the formula (I)

EP 0 630 890 A2

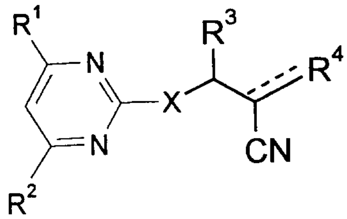

(I)

wherein

| | |
|---|---|
| X | represents oxygen or sulfur, |
| $R^1$ | represents halogen, $C_{1-4}$ alkyl, $C_{1-4}$ alkoxy, halogeno-$C_{1-4}$ alkyl, halogeno-$C_{1-4}$ alkoxy, |
| $R^2$ | represents halogen, $C_{1-4}$ alkoxy, halogeno-$C_{1-4}$ alkyl, halogeno-$C_{1-4}$ alkoxy, |
| $R^3$ | represents in each case optionally $C_{1-4}$ substituted or unsubstituted alkyl, or $C_{3-7}$ cycloalkyl, |
| ---- | represents a single bond or a double bond, |
| $R^4$ | represents hydroxy, oxo or one of the following groups: |

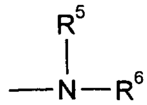

,

$-O-SO_2-R^7$ or

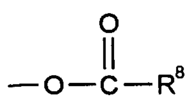

,

| | |
|---|---|
| $R^5$ and $R^6$ | each represent independently from each other hydrogen, or in each case optionally substituted or unsubstituted $C_{3-8}$ cycloalkyl or $C_{2-8}$ alkenyl or |
| $R^5$ and $R^6$ | together with the nitrogen atom to which they are bonded may form a 5- or 6-membered heterocyclic ring, |
| $R^7$ | represents in each case optionally substituted or unsubstituted $C_{1-8}$ alkyl, $C_{2-8}$ alkenyl, phenyl or a heterocyclic ring, |

and

$R^8$ represents hydrogen, or in each case optionally substituted or unsubstituted $C_{1-20}$ alkyl, $C_{2-20}$ alkenyl $C_{2-20}$ alkynyl, phenyl, a heterocyclic ring, a condensed heterocyclic ring or $C_{3-8}$ cycloalkyl,

and the use of the new compounds as herbicides.

The present invention relates to pyrimidine derivatives containing a cyano group, to processes for their preparation, and to their use as herbicides.

It has already been disclosed that a certain group of pyrimidine derivatives is useful as herbicides (see Japanese Patent Application Nos. 118540/1992, 307813/1992 and 47110/1993, and WO93/12094).

There have now been found novel pyrimidine derivatives containing a cyano group of the formula (I)

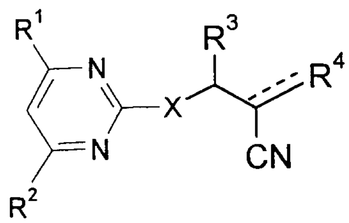

(I)

wherein

X represents oxygen or sulfur,

$R^1$ represents halogen, $C_{1-4}$ alkyl, $C_{1-4}$ alkoxy, halogeno-$C_{1-4}$ alkyl, halogeno-$C_{1-4}$ alkoxy,

$R^2$ represents halogen, $C_{1-4}$ alkoxy, halogeno-$C_{1-4}$ alkyl, halogeno-$C_{1-4}$ alkoxy,

$R^3$ represents in each case optionally $C_{1-4}$ substituted or unsubstituted alkyl, or $C_{3-7}$ cycloalkyl,

---- represents a single bond or a double bond,

$R^4$ represents hydroxy, oxo or one of the following groups:

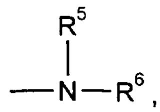

$-O-SO_2-R^7$ or

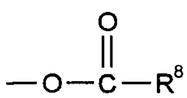

,

$R^5$ and $R^6$ each represent independently from each other hydrogen, or in each case optionally substituted or unsubstituted $C_{3-8}$ cycloalkyl or $C_{2-8}$ alkenyl or

$R^5$ and $R^6$ together with the nitrogen atom to which they are bonded may form a 5- or 6-membered heterocyclic ring,

$R^7$ represents in each case optionally substituted or unsubstituted $C_{1-8}$ alkyl, $C_{2-8}$ alkenyl, phenyl or a heterocyclic ring,

and

$R^8$ represents hydrogen, or in each case optionally substituted or unsubstituted $C_{1-20}$ alkyl, $C_{2-20}$ alkenyl, $C_{2-20}$ alkynyl, phenyl, a heterocyclic ring, a condensed heterocyclic ring or $C_{3-8}$ cycloalkyl.

Pyrimidine derivatives containing a cyano group of the formula (I) are obtained when

(a) in the case where $R^4$ represents hydroxy:

compounds of the formula (II)

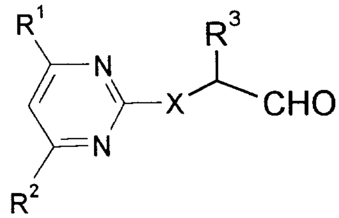

(II)

wherein
$R^1$, $R^2$ and $R^3$ have the above mentioned meanings,
are reacted with sodium cyanide or potassium cyanide,
in the presence of an acid, and if appropriate, in the presence of inert solvents,

or

(b) in the case where $R^4$ represents oxo:
compounds of the formula (Ia)

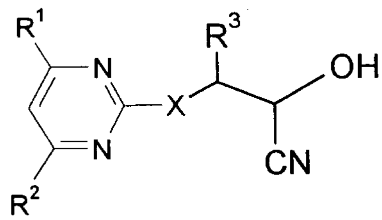

(Ia)

wherein $R^1$, $R^2$ and $R^3$ have the above mentioned meanings,
are reacted with an oxidizing agent,
if appropriate, in the presence of inert solvents,

or

(c) in the case where $R^4$ represents

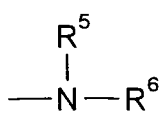:

the compounds of the formula (Ia)
are reacted with a compound of the following formula (III)

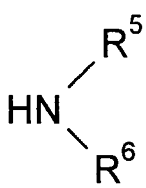

(III)

wherein $R^5$ and $R^6$ have the above mentioned meanings,
if appropriate, in the presence of inert solvents,

or

(d) in the case where $R^4$ represents $-O-SO_2-R^7$:
the compounds of the formula (Ia)
are reacted with a compound of the formula (IV)

$R^7-SO_2-Y$ (IV)

wherein $R^7$ represents the same meanings as mentioned above, and Y represents chlorine or bromine, if appropriate, in the presence of an acid binder, and if appropriate, in the presence of inert solvents,

or

(e) in the case where $R^4$ represents

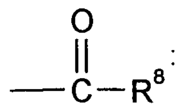

the compounds of the formula (Ia)

are reacted with the compound of the following formula (V)

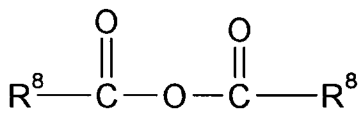

(V)

wherein $R^8$ has the same meanings as mentioned above,

or are reacted with the compounds of the following formula (VI)

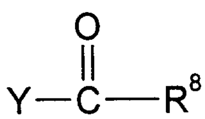

(VI)

wherein $R^8$ and Y represent the same meanings as mentioned above,

if appropriate, in the presence of an acid binder, and if appropriate, in the presence of inert solvents.

The novel pyrimidine derivatives containing a cyano group according to the invention exhibit powerful herbidical properties.

Surprisingsly, the pyrimidine derivatives containing a cyano group according to the invention exhibit a substantially higher selective herbicidal action than those known from the prior art, for instance, the aforementioned Japanese Laid Open Patent Application Nos. 118540/1992, 307813/1992 and 47110/1993, and WO93/12094.

In the compounds of the formula (I) and their intermediates, the $C_{1-4}$ alkyl and the $C_{1-4}$ alkoxy moiety represent straight-chain or branched alkyl such as methyl, ethyl, n-propyl, isopropyl, or n-(iso-, sec- or tert-)butyl.

The $C_{1-20}$ alkyl represents straight-chain or branched alkyl containing 1 to 20 carbon atoms such as those exemplified by the above mentioned $C_{1-4}$ alkyl, n-(sec-, iso-)pentyl, hexyl, heptyl, nonyl, decyl, undecyl, dodecyl, tridecyl, tetradecyl, pentadecyl, hexadecyl, heptadecyl, octadecyl, nonadecyl, icosyl, 1-ethyl-1-methylpropane, 1,1-dimethylpropane and so on.

The halogen or halogen moiety of halogeno-$C_{1-4}$ alkyl or halogeno-$C_{1-4}$ alkoxy represent fluorine, chlorine, bromine or iodine, preferably fluorine or chlorine.

The $C_{3-7}$ cycloalkyl represents cyclopropyl, cyclobutyl, cyclopentyl, cyclohexyl and so on, preferably cyclopropyl, cyclopentyl, cyclohexyl.

The $C_{2-20}$ alkenyl represents straight-chain or branched alkenyl such as vinyl, 1-(2-)propenyl, isopropenyl, 1-(2-, or 3-)butenyl, 1-(2-, 3-, 4-)pentenyl, 9-decenyl, 8-tridecenyl, 10-nonadecenyl, 8-heptadecenyl, 8-pentadecenyl, 2-methyl-1-propenyl, 1-methyl-1-butenyl, 1,1-dimethyl-3-butenyl and so on.

The five- or six-membered heterocyclic ring may have one to four hetero atoms selected from the group consisting of nitrogen, sulfur and oxygen, such as thiadiazolyl, furyl, thiazolyl, imidazolyl, pyridyl, pyrimidinyl, thienyl, pyrazinyl, pyridazinyl, isoxazolyl and pyrazolyl and the like.

The condensed heterocyclic ring represents a nine- or ten-membered bicyclic group that has been formed by the above mentioned five- or six-membered heterocyclic ring being condensed with phenyl, such as quinolyl, benzoxazolyl, benzothiazolyl, benzisoxazolyl, benzimidazolyl, phthalazinyl and the like.

Among the pyrimidine derivatives containing a cyano group according to the invention of the formula (I), the preferred compounds are those wherein

X represents oxygen or sulfur,

$R^1$ represents halogen, $C_{1-3}$ alkyl, $C_{1-3}$ alkoxy, halogeno-$C_{1-3}$ alkyl or halogeno-$C_{1-3}$ alkoxy,

$R^2$ represents halogen, $C_{1-3}$ alkoxy, halogeno-$C_{1-3}$ alkyl or halogeno-$C_{1-3}$ alkoxy,

$R^3$ represents $C_{2-4}$ alkyl, or $C_{3-6}$ cycloalkyl which may be substituted by $C_{1-3}$ alkyl,

---- represents a single bond or double bond,

$R^4$ represents hydroxy, oxo or one of the following groups:

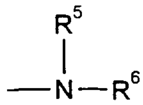, 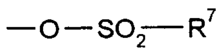 or 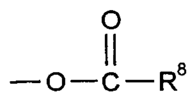,

$R^5$ and $R^6$ each represent independently from each other hydrogen, and in each case optionally substituted or unsubstituted $C_{1-6}$ alkyl, $C_{1-4}$ alkoxy-$C_{1-4}$ alkyl, $C_{3-8}$ cycloalkyl-$C_{1-4}$ alkyl, $C_{1-4}$ alkyl-$C_{3-8}$ cycloalkyl-$C_{1-4}$ alkyl, $C_{3-8}$ cycloalkyl, $C_{3-8}$ cycloalkyl-$C_{1-4}$ alkyl, $C_{2-6}$ alkenyl, or phenyl-$C_{1-4}$ alkyl; the substituent(s) being selected from the group consisting of halogen, $C_{1-4}$ alkyl, halogeno-$C_{1-4}$ alkyl and halogeno-$C_{1-4}$ alkoxy, or

$R^5$ and $R^6$ may form a pyrrolidine, piperidine or morpholine together with the nitrogen to which they are bonded,

$R^7$ represents in each case optionally substituted or unsubstituted $C_{1-4}$ alkyl, halogeno-$C_{1-4}$ alkyl, $C_{1-4}$ alkyl-carbonyl-$C_{1-4}$ alkyl, $C_{2-8}$ alkenyl, phenyl, the substituent(s) being selected from the group consisting of halogen, nitro, $C_{1-4}$ alkyl, halogeno-$C_{1-4}$ alkyl, $C_{1-4}$ alkoxy, halogeno-$C_{1-4}$ alkoxy and $C_{1-4}$ alkoxycarbonyl,

$R^7$ further represents an optionally substituted 5- or 6-membered heterocyclic ring, the hetero atom(s) of said heterocyclic ring being selected from the group consisting of oxygen, sulfur and nitrogen, and the substituent(s) being selected from the group consisting of halogen, $C_{1-4}$ alkyl, $C_{1-4}$ alkoxy or $C_{1-4}$ alkoxycarbonyl,

and

$R^8$ represents hydrogen, $C_{1-6}$ alkyl, halogeno-$C_{1-6}$ alkyl, cyano-$C_{1-6}$ alkyl, carboxyl-$C_{1-6}$ alkyl, $C_{1-4}$ alkoxy-$C_{1-4}$ alkyl, $C_{1-4}$ alkyl-carbonyl-$C_{1-4}$ alkyl, or $C_{1-4}$ alkoxycarbonyl-$C_{1-4}$ alkyl,

$R^8$ further represents in each case optionally substituted or unsubstituted phenyl-$C_{1-4}$ alkyl, phenoxy-$C_{1-4}$ alkyl or phenylthio-$C_{1-4}$ alkyl, the substituent(s) being selected from the group consisting of halogen, $C_{1-4}$ alkyl and halogeno-$C_{1-4}$ alkyl,

$R^8$ further represents optionally substituted or unsubstituted $C_{2-6}$ alkenyl, the substituent(s) being selected from the group consisting of halogen, carboxyl or its salt, phenyl halogeno-phenyl and $C_{1-4}$ alkyl-phenyl,

$R^8$ further represents in each case optionally substituted or unsubstituted $C_{2-6}$ alkynyl or phenyl, the substituent(s) being selected from the group consisting of nitro, halogen, $C_{1-4}$ alkyl, $C_{1-4}$ alkoxy, $C_{1-4}$ alkylthio, halogeno-$C_{1-4}$ alkyl and halogeno-$C_{1-4}$ alkylthio,

$R^8$ further represents in each case an optionally substituted 5- or 6-membered heterocyclic ring or 5.6- or 6.6-condensed heterocyclic ring, the hetero atom(s) of said heterocyclic ring(s) being selected from the group consisting of oxygen, sulfur and nitrogen, the substituent(s) being selected from the group consisting of halogen, $C_{1-4}$ alkyl, $C_{1-4}$ alkoxy, $C_{1-4}$ alkylthio, halogeno-$C_{1-4}$ alkyl, phenyl, halogeno-phenyl, $C_{1-4}$ alkyl-phenyl and carboxyl or its salts, or

$R^8$ further represents $C_{3-8}$ cycloalkyl which may optionally be substituted by $C_{1-4}$ alkyl.

Very particularly preferred pyrimidine derivatives containing a cyano group according to the invention of the formula (I), are those wherein

X represents oxygen or sulfur,

$R^1$ represents methoxy,

$R^2$ represents methoxy,

$R^3$ represents $C_{2-4}$ alkyl or $C_{3-6}$ cycloalkyl which may be substituted by methyl,

---- represents single bond or double bond,

$R^4$ represents hydroxy, oxo or one of the following groups:

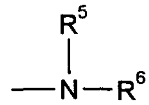,

$-O-SO_2-R^7$ or

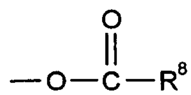,

$R^5$ and $R^6$ each represent independently from each other hydrogen, or in each case optionally substituted or unsubstituted $C_{1-4}$ alkyl, methoxy-$C_{1-3}$ alkyl, cyclopropyl-$C_{1-3}$ alkyl, methyl-cyclopropyl-$C_{1-3}$ alkyl, cyclopentyl-$C_{1-3}$ alkyl, methyl-cyclopentyl-$C_{1-3}$ alkyl, cyclohexyl-$C_{1-3}$ alkyl, methyl-cyclohexyl-$C_{1-3}$ alkyl, cyclopropyl, methyl-cyclopropyl, cyclopentyl, methyl-cyclopentyl, cyclohexyl, methyl-cyclohexyl, $C_{2-4}$ alkenyl, or phenyl-$C_{1-3}$ alkyl, the subsitutent(s) being selected from the group consisting of fluoro, chloro, bromo, methyl, butyl, trifluoromethyl, methoxy and trifluoromethoxy, or

$R^5$ and $R^6$ may form a pyrrolidine, piperidine or morpholine together with the nitrogen to which they are bonded,

$R^7$ represents in each case optionally substituted or unsubstituted $C_{1-4}$ alkyl, or $C_{2-8}$ alkenyl, the substituent(s) being selected from the group consisting of fluoro, chloro, bromo and methoxy-carbonyl, or phenyl being optionally substituted by one of the group consisting of fluoro, chloro, bromo, nitro, $C_{1-4}$ alkyl, halogeno-$C_{1-4}$ alkyl, methoxy, trifluoromethoxy and methoxy-carbonyl,

$R^7$ further represents in each case an optionally substituted thienyl, thiazolyl, imidazolyl, pyrazolyl, furyl, pyridyl, pyrimidinyl, pyridazinyl, pyrazinyl, isoxazolyl and oxazolyl, the substitutent(s) being selected from the group consisting of fluoro, chloro, bromo, methyl and methoxy-carbonyl,

and

$R^8$ represents hydrogen, $C_{1-4}$ alkyl, halogeno-$C_{1-4}$ alkyl, cyano-$C_{1-4}$ alkyl, carboxyl-$C_{1-4}$ alkyl, methoxy-$C_{1-4}$ alkyl, methylcarbonyl-$C_{1-4}$ alkyl, or methoxycarbonyl-$C_{1-4}$ alkyl,

$R^8$ further represents in each case optionally substituted phenyl-$C_{1-4}$ alkyl, phenoxy-$C_{1-4}$ alkyl or phenylthio-$C_{1-4}$ alkyl, the subsitutent(s) of those phenyl rings being selected from the group consisting of fluoro, chloro, bromo, $C_{1-4}$ alkyl and trifluoromethyl,

$R^8$ further represents optionally substituted $C_{2-6}$ alkenyl, the substituent(s) being selected from the group consisting of fluoro, chloro, bromo, carboxyl or its sodium salt and phenyl which may be substituted by fluoro, chloro, bromo or methyl,

$R^8$ further represents $C_{2-6}$ alkynyl, optionally substituted phenyl, the substituent(s) being selected from the group consisting of nitro, fluoro, chloro, bromo, methyl, ethyl, methoxy, methylthio, trifluoromethyl and trifluoromethoxy,

$R^8$ further represents in each case an optionally substituted thienyl, thiazolyl, imidazolyl, pyrazolyl, furyl, pyridyl, pyrimidinyl, pyridazinyl, pyrazinyl, isoxalyl, oxazolyl, quinolyl and indolinyl, the substituent(s) being selected from the group consisting of fluoro, chloro, bromo, methoxy, methylthio, trifluoromethyl, carboxyl, phenyl, halogeno-phenyl and $C_{1-4}$ alkyl-phenyl,

$R^8$ further represents cyclopropyl, methyl-cyclopropyl, cyclopenthyl, methyl-cyclopenthyl, cyclohexyl, methyl-cyclohexyl.

As examples of the compounds of the formula (I) according to the invention, there may be mentioned those in the following Table 1, in addition to the compounds that will be enumerated in the Examples hereinafter.

Table 1

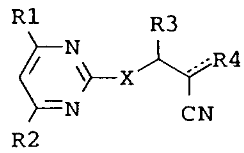

| R1 | R2 | X | R3 | R4 |
|---|---|---|---|---|
| $OCH_3$ | $OCH_3$ | O | $CH_2CH_2CH_3$ | O |
| $OCH_3$ | $OCH_3$ | O | $CH(CH_3)_2$ | O |
| $OCH_3$ | $OCH_3$ | O | $CH_2CH_2CH_2CH_3$ | O |
| $OCH_3$ | $OCH_3$ | O | $CH(CH_3)C_2H_5$ | O |
| $OCH_3$ | $OCH_3$ | O | $CH_2CH(CH_3)_2$ | O |
| $OCH_3$ | $OCH_3$ | O | $C(CH_3)_3$ | O |
| $OCH_3$ | $OCH_3$ | O | $CH(CH_3)(CH_2)_2CH_3$ | O |
| $OCH_3$ | $OCH_3$ | O | $CH(C_2H_5)_2$ | O |
| $OCH_3$ | $OCH_3$ | O | $C(CH_3)_2C_2H_5$ | O |
| $OCH_3$ | $OCH_3$ | O | 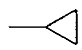 | O |
| $OCH_3$ | $OCH_3$ | O | 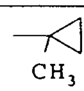 | O |
| $OCH_3$ | $OCH_3$ | O | 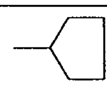 | O |
| $OCH_3$ | $OCH_3$ | O | 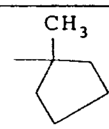 | O |
| $OCH_3$ | $OCH_3$ | S | $CH_2CH_2CH_3$ | O |
| $OCH_3$ | $OCH_3$ | S | $CH(CH_3)_2$ | O |
| $OCH_3$ | $OCH_3$ | S | $CH_2CH_2CH_2CH_3$ | O |
| $OCH_3$ | $OCH_3$ | S | $CH(CH_3)C_2H_5$ | O |
| $OCH_3$ | $OCH_3$ | S | $CH_2CH(CH_3)_2$ | O |
| $OCH_3$ | $OCH_3$ | S | $C(CH_3)_3$ | O |
| $OCH_3$ | $OCH_3$ | S | $CH(CH_3)(CH_2)_2CH_3$ | O |
| $OCH_3$ | $OCH_3$ | S | $CH(C_2H_5)_2$ | O |
| $OCH_3$ | $OCH_3$ | S | $C(CH_3)_2C_2H_5$ | O |
| $OCH_3$ | $OCH_3$ | S | 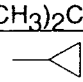 | O |
| $OCH_3$ | $CH_3$ | O | $C(CH_3)_3$ | O |
| $OCH_3$ | $CH_3$ | S | $C(CH_3)_3$ | O |
| $OCH_3$ | $OCH_3$ | S | 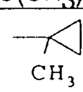 | O |
| $OCH_3$ | $OCH_3$ | S | 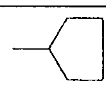 | O |

Table 1 (continued)

| R1 | R2 | X | R3 | R4 |
|---|---|---|---|---|
| $OCH_3$ | $OCH_3$ | S | 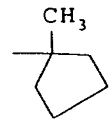 | O |
| $OCF_3$ | $OCF_3$ | O | $C(CH_3)_3$ | O |
| $OCF_3$ | $OCF_3$ | S | $C(CH_3)_3$ | O |
| OCHF2 | OCHF2 | O | $C(CH_3)_3$ | O |
| OCHF2 | OCHF2 | S | $C(CH_3)_3$ | O |
| $OCF_3$ | $OCH_3$ | O | $CH(CH_3)_2$ | O |
| $OCF_3$ | $OCH_3$ | S | $CH(CH_3)_2$ | O |
| $OCF_3$ | $OCH_3$ | O | $C(CH_3)_3$ | O |
| $OCF_3$ | $OCH_3$ | S | $C(CH_3)_3$ | O |
| $OCH_3$ | $OCH_3$ | O | $CH_2CH_2CH_3$ | OH |
| $OCH_3$ | $OCH_3$ | O | $CH(CH_3)_2$ | OH |
| $OCH_3$ | $OCH_3$ | O | $CH_2CH_2CH_2CH_3$ | OH |
| $OCH_3$ | $OCH_3$ | O | $CH(CH_3)C_2H_5$ | OH |
| $OCH_3$ | $OCH_3$ | O | $CH_2CH(CH_3)_2$ | OH |
| $OCH_3$ | $OCH_3$ | O | $C(CH_3)_3$ | OH |
| $OCH_3$ | $OCH_3$ | O | $CH(CH_3)(CH_2)_2CH_3$ | OH |
| $OCH_3$ | $OCH_3$ | O | $CH(C_2H_5)_2$ | OH |
| $OCH_3$ | $OCH_3$ | O | $C(CH_3)_2C_2H_5$ | OH |
| $OCH_3$ | $OCH_3$ | O | 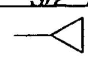 | OH |
| $OCH_3$ | $OCH_3$ | O | 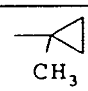 | OH |
| $OCH_3$ | $OCH_3$ | O | 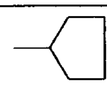 | OH |
| $OCH_3$ | $OCH_3$ | O | 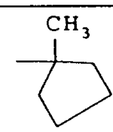 | OH |
| $OCH_3$ | $OCH_3$ | S | $CH_2CH_2CH_3$ | OH |
| $OCH_3$ | $OCH_3$ | S | $CH(CH_3)_2$ | OH |
| $OCH_3$ | $OCH_3$ | S | $CH_2CH_2CH_2CH_3$ | OH |
| $OCH_3$ | $OCH_3$ | S | $CH(CH_3)C_2H_5$ | OH |
| $OCH_3$ | $OCH_3$ | S | $CH_2CH(CH_3)_2$ | OH |
| $OCH_3$ | $OCH_3$ | S | $C(CH_3)_3$ | OH |
| $OCH_3$ | $OCH_3$ | S | $CH(CH_3)(CH_2)_2CH_3$ | OH |
| $OCH_3$ | $OCH_3$ | S | $CH(C_2H_5)_2$ | OH |
| $OCH_3$ | $OCH_3$ | O | 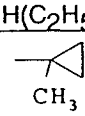 | $NH_2$ |
| $OCH_3$ | $OCH_3$ | O | 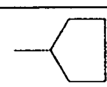 | $NH_2$ |

Table 1 (continued)

| R1 | R2 | X | R3 | R4 |
|---|---|---|---|---|
| $OCH_3$ | $OCH_3$ | O | 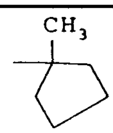 | $NH_2$ |
| $OCH_3$ | $OCH_3$ | S | $C(CH_3)_2C_2H_5$ | OH |
| $OCH_3$ | $OCH_3$ | S | 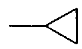 | OH |
| $OCH_3$ | $OCH_3$ | S | 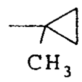 | OH |
| $OCH_3$ | $OCH_3$ | S | 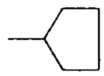 | OH |
| $OCH_3$ | $OCH_3$ | S | 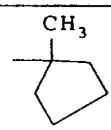 | OH |
| $OCH_3$ | $CH_3$ | O | $C(CH_3)_3$ | OH |
| $OCH_3$ | $CH_3$ | S | $C(CH_3)_3$ | OH |
| $OCF_3$ | $OCF_3$ | O | $C(CH_3)_3$ | OH |
| $OCF_3$ | $OCF_3$ | S | $C(CH_3)_3$ | OH |
| OCHF2 | OCHF2 | O | $C(CH_3)_3$ | OH |
| OCHF2 | OCHF2 | S | $C(CH_3)_3$ | OH |
| $OCF_3$ | $OCH_3$ | O | $CH(CH_3)_2$ | OH |
| $OCF_3$ | $OCH_3$ | S | $CH(CH_3)_2$ | OH |
| $OCF_3$ | $OCH_3$ | O | $C(CH_3)_3$ | OH |
| $OCF_3$ | $OCH_3$ | S | $C(CH_3)_3$ | OH |
| $OCH_3$ | $OCH_3$ | O | $CH_2CH_2CH_3$ | $NH_2$ |
| $OCH_3$ | $OCH_3$ | O | $CH(CH_3)_2$ | $NH_2$ |
| $OCH_3$ | $OCH_3$ | O | $CH_2CH_2CH_2CH_3$ | $NH_2$ |
| $OCH_3$ | $OCH_3$ | O | $CH(CH_3)C_2H_5$ | $NH_2$ |
| $OCH_3$ | $OCH_3$ | O | $CH_2CH(CH_3)_2$ | $NH_2$ |
| $OCH_3$ | $OCH_3$ | O | $C(CH_3)_3$ | $NH_2$ |
| $OCH_3$ | $OCH_3$ | O | $CH(CH_3)(CH_2)_2CH_3$ | $NH_2$ |
| $OCH_3$ | $OCH_3$ | O | $CH(C_2H_5)_2$ | $NH_2$ |
| $OCH_3$ | $OCH_3$ | O | $C(CH_3)_2C_2H_5$ | $NH_2$ |
| $OCH_3$ | $OCH_3$ | O | 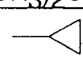 | $NH_2$ |
| $OCH_3$ | $OCH_3$ | S | $CH_2CH_2CH_3$ | $NH_2$ |
| $OCH_3$ | $OCH_3$ | S | $CH(CH_3)_2$ | $NH_2$ |
| $OCH_3$ | $OCH_3$ | S | $C(CH_3)_3$ | $NH_2$ |
| $OCH_3$ | $OCH_3$ | S | $CH(CH_3)(CH_2)_2CH_3$ | $NH_2$ |
| $OCH_3$ | $OCH_3$ | S | $CH(C_2H_5)_2$ | $NH_2$ |
| $OCH_3$ | $OCH_3$ | S | $CH_2CH_2CH_2CH_3$ | $NH_2$ |
| $OCH_3$ | $OCH_3$ | S | $CH(CH_3)C_2H_5$ | $NH_2$ |
| $OCH_3$ | $OCH_3$ | S | $CH_2CH(CH_3)_2$ | $NH_2$ |

Table I (continued)

| R1 | R2 | X | R3 | R4 |
|---|---|---|---|---|
| $OCH_3$ | $OCH_3$ | S | $C(CH_3)_2C_2H_5$ | $NH_2$ |
| $OCH_3$ | $OCH_3$ | S | 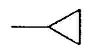 | $NH_2$ |
| $OCH_3$ | $OCH_3$ | S | 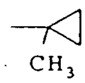 | $NH_2$ |
| $OCH_3$ | $OCH_3$ | S | 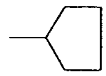 | $NH_2$ |
| $OCH_3$ | $OCH_3$ | S | 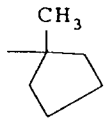 | $NH_2$ |
| $OCH_3$ | $CH_3$ | O | $C(CH_3)_3$ | $NH_2$ |
| $OCH_3$ | $CH_3$ | S | $C(CH_3)_3$ | $NH_2$ |
| $OCF_3$ | $OCF_3$ | O | $C(CH_3)_3$ | $NH_2$ |
| $OCF_3$ | $OCF_3$ | S | $C(CH_3)_3$ | $NH_2$ |
| OCHF2 | OCHF2 | O | $C(CH_3)_3$ | $NH_2$ |
| OCHF2 | OCHF2 | S | $C(CH_3)_3$ | $NH_2$ |
| $OCF_3$ | $OCH_3$ | O | $CH(CH_3)_2$ | $NH_2$ |
| $OCF_3$ | $OCH_3$ | S | $CH(CH_3)_2$ | $NH_2$ |
| $OCF_3$ | $OCH_3$ | O | $C(CH_3)_3$ | $NH_2$ |
| $OCF_3$ | $OCH_3$ | S | $C(CH_3)_3$ | $NH_2$ |
| $OCH_3$ | $OCH_3$ | O | $CH(CH_3)_2$ | 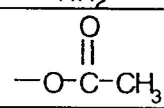 |
| $OCH_3$ | $OCH_3$ | O | $CH(CH_3)_2$ | 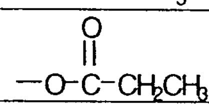 |
| $OCH_3$ | $OCH_3$ | O | $CH(CH_3)_2$ | 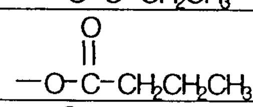 |
| $OCH_3$ | $OCH_3$ | S | $C(CH_3)_3$ | 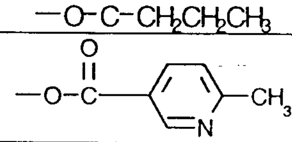 |
| $OCH_3$ | $OCH_3$ | S | $C(CH_3)_3$ | 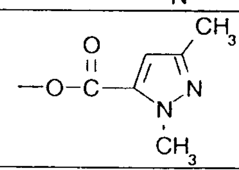 |
| $OCH_3$ | $OCH_3$ | O | $CH(CH_3)_2$ | 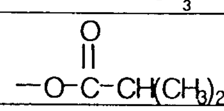 |
| $OCH_3$ | $OCH_3$ | O | $CH(CH_3)_2$ | 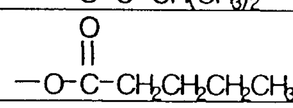 |

Table 1 (continued)

| R1 | R2 | X | R3 | R4 |
|---|---|---|---|---|
| $OCH_3$ | $OCH_3$ | O | $CH(CH_3)_2$ | 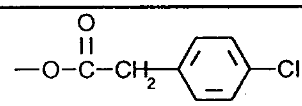 |
| $OCH_3$ | $OCH_3$ | O | $CH(CH_3)_2$ | 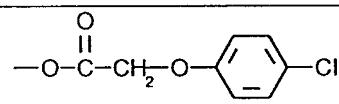 |
| $OCH_3$ | $OCH_3$ | O | $CH(CH_3)_2$ | 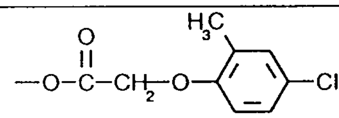 |
| $OCH_3$ | $OCH_3$ | O | $CH(CH_3)_2$ | 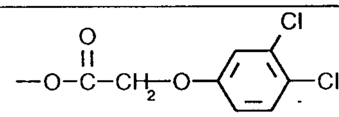 |
| $OCH_3$ | $OCH_3$ | O | $CH(CH_3)_2$ | 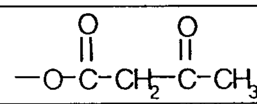 |
| $OCH_3$ | $OCH_3$ | O | $CH(CH_3)_2$ | 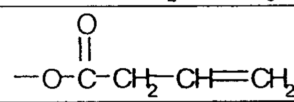 |
| $OCH_3$ | $OCH_3$ | O | $CH(CH_3)_2$ | 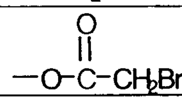 |
| $OCH_3$ | $OCH_3$ | O | $CH(CH_3)_2$ | 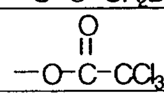 |
| $OCH_3$ | $OCH_3$ | O | $CH(CH_3)_2$ | 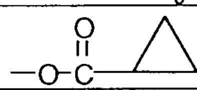 |
| $OCH_3$ | $OCH_3$ | O | $CH(CH_3)_2$ | 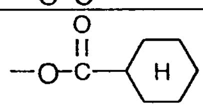 |
| $OCH_3$ | $OCH_3$ | O | $CH(CH_3)_2$ | 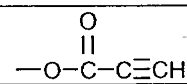 |
| $OCH_3$ | $OCH_3$ | O | $CH(CH_3)_2$ | 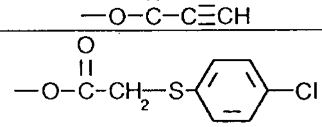 |
| $OCH_3$ | $OCH_3$ | O | $CH(CH_3)_2$ | 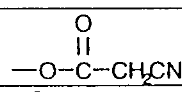 |
| $OCH_3$ | $OCH_3$ | O | $CH(CH_3)_2$ | 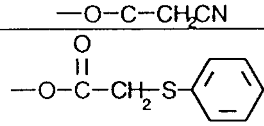 |
| $OCH_3$ | $OCH_3$ | O | $CH(CH_3)_2$ | 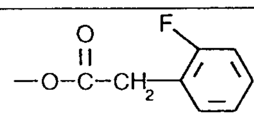 |

Table 1 (continued)

| R1 | R2 | X | R3 | R4 |
|---|---|---|---|---|
| $OCH_3$ | $OCH_3$ | O | $CH(CH_3)_2$ | 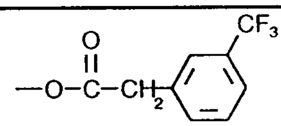 |
| $OCH_3$ | $OCH_3$ | O | $CH(CH_3)_2$ | 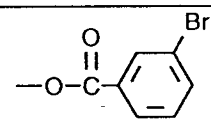 |
| $OCH_3$ | $OCH_3$ | O | $CH(CH_3)_2$ | 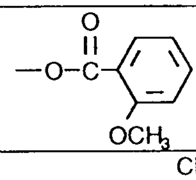 |
| $OCH_3$ | $OCH_3$ | O | $CH(CH_3)_2$ | 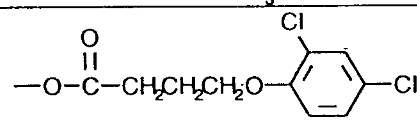 |
| $OCH_3$ | $OCH_3$ | O | $CH(CH_3)_2$ | 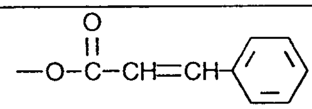 |
| $OCH_3$ | $OCH_3$ | O | $CH(CH_3)_2$ | 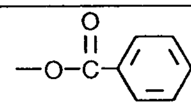 |
| $OCH_3$ | $OCH_3$ | O | $CH(CH_3)_2$ | 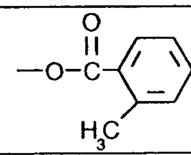 |
| $OCH_3$ | $OCH_3$ | O | $CH(CH_3)_2$ | 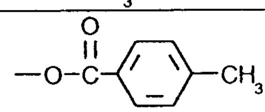 |
| $OCH_3$ | $OCH_3$ | O | $CH(CH_3)_2$ | 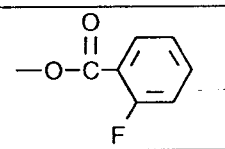 |
| $OCH_3$ | $OCH_3$ | O | $CH(CH_3)_2$ | 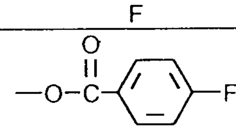 |
| $OCH_3$ | $OCH_3$ | O | $CH(CH_3)_2$ | 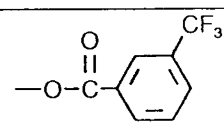 |
| $OCH_3$ | $OCH_3$ | O | $CH(CH_3)_2$ | 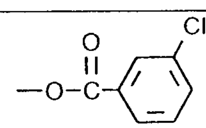 |

Table 1 (continued)

| R1 | R2 | X | R3 | R4 |
|---|---|---|---|---|
| $OCH_3$ | $OCH_3$ | O | $CH(CH_3)_2$ | 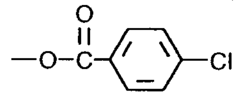 |
| $OCH_3$ | $OCH_3$ | O | $CH(CH_3)_2$ | 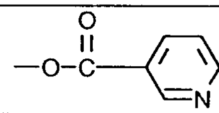 |
| $OCH_3$ | $OCH_3$ | O | $CH(CH_3)_2$ | 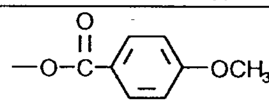 |
| $OCH_3$ | $OCH_3$ | O | $CH(CH_3)_2$ | 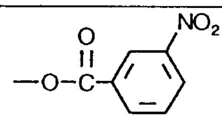 |
| $OCH_3$ | $OCH_3$ | O | $CH(CH_3)_2$ | 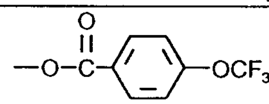 |
| $OCH_3$ | $OCH_3$ | O | $CH(CH_3)_2$ | 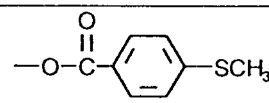 |
| $OCH_3$ | $OCH_3$ | O | $CH(CH_3)_2$ | 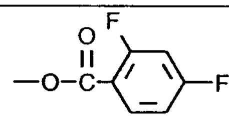 |
| $OCH_3$ | $OCH_3$ | O | $CH(CH_3)_2$ | 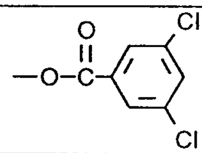 |
| $OCH_3$ | $OCH_3$ | O | $CH(CH_3)_2$ | 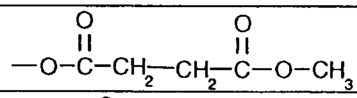 |
| $OCH_3$ | $OCH_3$ | O | $CH(CH_3)_2$ | 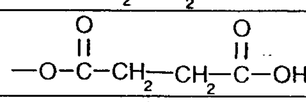 |
| $OCH_3$ | $OCH_3$ | O | $CH(CH_3)_2$ | 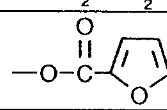 |
| $OCH_3$ | $OCH_3$ | O | $CH(CH_3)_2$ | 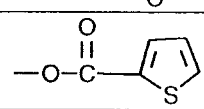 |
| $OCH_3$ | $OCH_3$ | O | $CH(CH_3)_2$ | 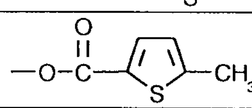 |
| $OCH_3$ | $OCH_3$ | O | $CH(CH_3)_2$ | 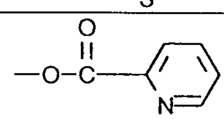 |

Table 1 (continued)

| R1 | R2 | X | R3 | R4 |
|---|---|---|---|---|
| $OCH_3$ | $OCH_3$ | O | $CH(CH_3)_2$ | 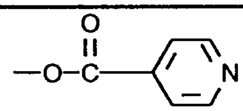 |
| $OCH_3$ | $OCH_3$ | O | $CH(CH_3)_2$ | 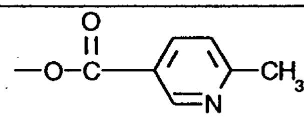 |
| $OCH_3$ | $OCH_3$ | O | $CH(CH_3)_2$ | 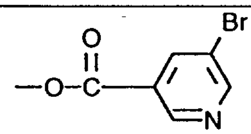 |
| $OCH_3$ | $OCH_3$ | O | $CH(CH_3)_2$ | 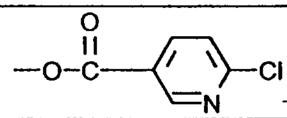 |
| $OCH_3$ | $OCH_3$ | O | $CH(CH_3)_2$ | 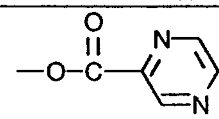 |
| $OCH_3$ | $OCH_3$ | O | $CH(CH_3)_2$ | 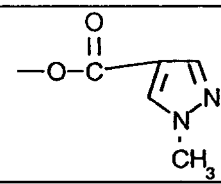 |
| $OCH_3$ | $OCH_3$ | O | $CH(CH_3)_2$ | 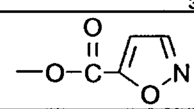 |
| $OCH_3$ | $OCH_3$ | O | $CH(CH_3)_2$ | 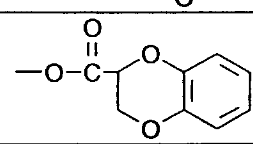 |
| $OCH_3$ | $OCH_3$ | O | $CH(CH_3)_2$ | 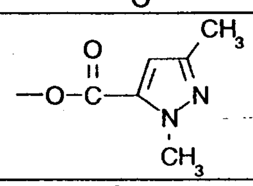 |
| $OCH_3$ | $OCH_3$ | O | $CH(CH_3)_2$ | 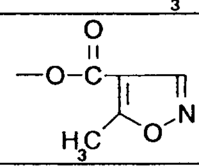 |
| $OCH_3$ | $OCH_3$ | O | $CH(CH_3)_2$ | 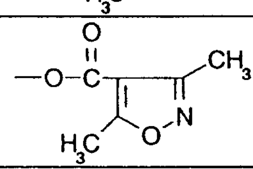 |

Table 1 (continued)

| R1 | R2 | X | R3 | R4 |
|---|---|---|---|---|
| $OCH_3$ | $OCH_3$ | O | $CH(CH_3)_2$ | 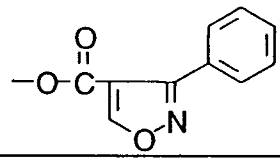 |
| $OCH_3$ | $OCH_3$ | O | $CH(CH_3)_2$ | 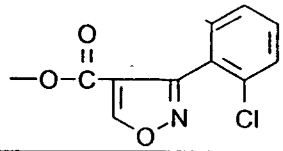 |
| $OCH_3$ | $OCH_3$ | O | $CH(CH_3)_2$ | 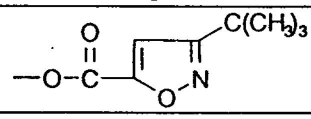 |
| $OCH_3$ | $OCH_3$ | O | $CH(CH_3)_2$ | 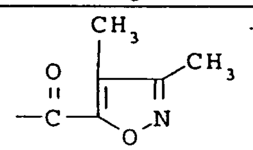 |
| $OCH_3$ | $OCH_3$ | O | $CH(CH_3)_2$ | 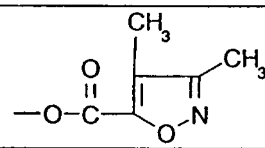 |
| $OCH_3$ | $OCH_3$ | O | $CH(CH_3)_2$ | 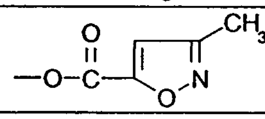 |
| $OCH_3$ | $OCH_3$ | O | $CH(CH_3)_2$ | 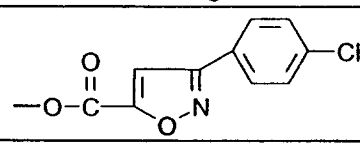 |
| $OCH_3$ | $OCH_3$ | O | $CH(CH_3)_2$ | 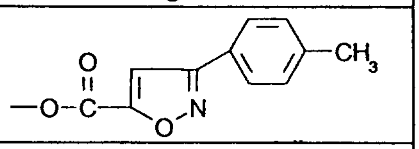 |
| $OCH_3$ | $OCH_3$ | O | $CH(CH_3)_2$ | 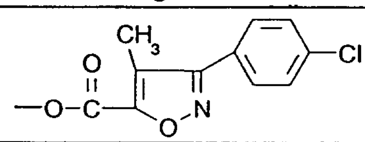 |
| $OCH_3$ | $OCH_3$ | O | $CH(CH_3)_2$ | 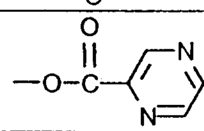 |
| $OCH_3$ | $OCH_3$ | O | $CH(CH_3)_2$ | 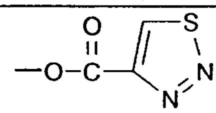 |

Table I (continued)

| R1 | R2 | X | R3 | R4 |
|---|---|---|---|---|
| $OCH_3$ | $OCH_3$ | O | $CH(CH_3)_2$ | 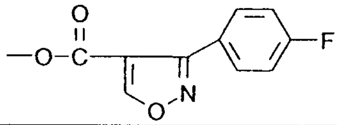 |
| $OCH_3$ | $OCH_3$ | O | $CH(CH_3)_2$ | 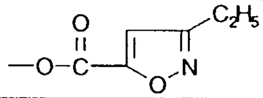 |
| $OCH_3$ | $OCH_3$ | O | $CH(CH_3)_2$ | 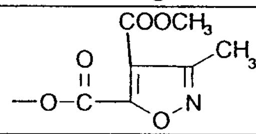 |
| $OCH_3$ | $OCH_3$ | O | $CH(CH_3)_2$ | 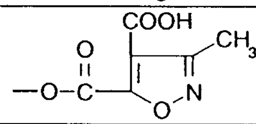 |
| $OCH_3$ | $OCH_3$ | O | $C(CH_3)_3$ | 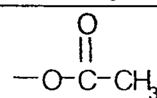 |
| $OCH_3$ | $OCH_3$ | O | $C(CH_3)_3$ | 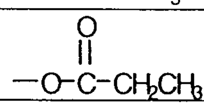 |
| $OCH_3$ | $OCH_3$ | O | $C(CH_3)_3$ | 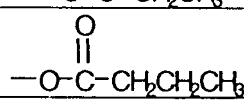 |
| $OCH_3$ | $OCH_3$ | O | $C(CH_3)_3$ | 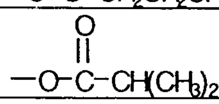 |
| $OCH_3$ | $OCH_3$ | O | $C(CH_3)_3$ | 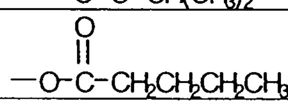 |
| $OCH_3$ | $OCH_3$ | O | $C(CH_3)_3$ | 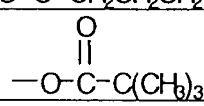 |
| $OCH_3$ | $OCH_3$ | O | $C(CH_3)_3$ | 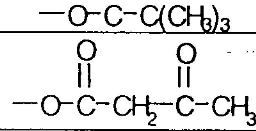 |
| $OCH_3$ | $OCH_3$ | O | $C(CH_3)_3$ | 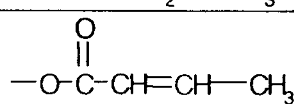 |
| $OCH_3$ | $OCH_3$ | O | $C(CH_3)_3$ | 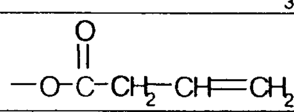 |
| $OCH_3$ | $OCH_3$ | O | $C(CH_3)_3$ | 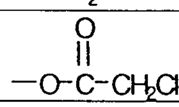 |
| $OCH_3$ | $OCH_3$ | O | $C(CH_3)_3$ | 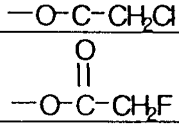 |

Table 1 (continued)

| R1 | R2 | X | R3 | R4 |
|---|---|---|---|---|
| $OCH_3$ | $OCH_3$ | O | $C(CH_3)_3$ | 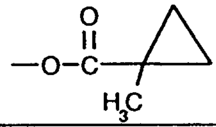 |
| $OCH_3$ | $OCH_3$ | O | $C(CH_3)_3$ | 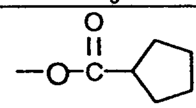 |
| $OCH_3$ | $OCH_3$ | O | $C(CH_3)_3$ | 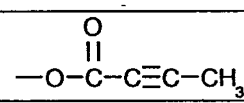 |
| $OCH_3$ | $OCH_3$ | O | $C(CH_3)_3$ | 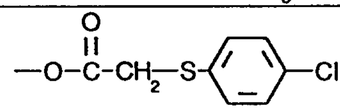 |
| $OCH_3$ | $OCH_3$ | O | $C(CH_3)_3$ | 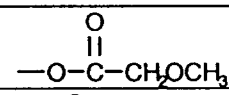 |
| $OCH_3$ | $OCH_3$ | O | $C(CH_3)_3$ | 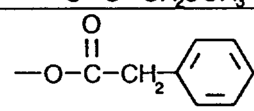 |
| $OCH_3$ | $OCH_3$ | O | $C(CH_3)_3$ | 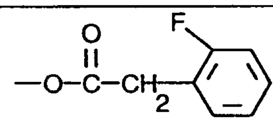 |
| $OCH_3$ | $OCH_3$ | O | $C(CH_3)_3$ | 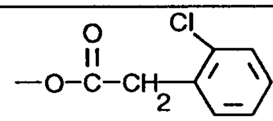 |
| $OCH_3$ | $OCH_3$ | O | $C(CH_3)_3$ | 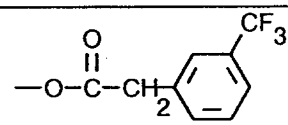 |
| $OCH_3$ | $OCH_3$ | O | $C(CH_3)_3$ | 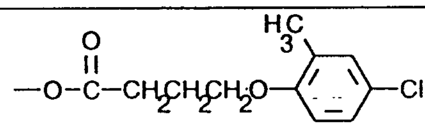 |
| $OCH_3$ | $OCH_3$ | O | $C(CH_3)_3$ | 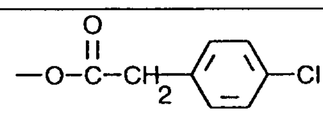 |
| $OCH_3$ | $OCH_3$ | O | $C(CH_3)_3$ | 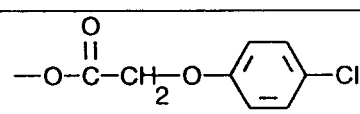 |
| $OCH_3$ | $OCH_3$ | O | $C(CH_3)_3$ | 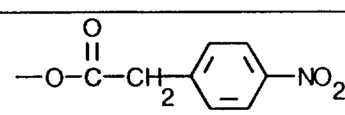 |

Table 1 (continued)

| R1 | R2 | X | R3 | R4 |
|---|---|---|---|---|
| $OCH_3$ | $OCH_3$ | O | $C(CH_3)_3$ | 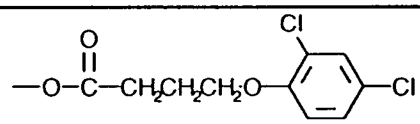 |
| $OCH_3$ | $OCH_3$ | O | $C(CH_3)_3$ | 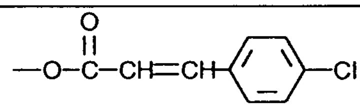 |
| $OCH_3$ | $OCH_3$ | O | $C(CH_3)_3$ | 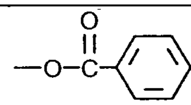 |
| $OCH_3$ | $OCH_3$ | O | $C(CH_3)_3$ | 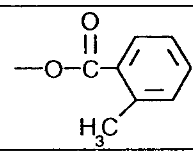 |
| $OCH_3$ | $OCH_3$ | O | $C(CH_3)_3$ | 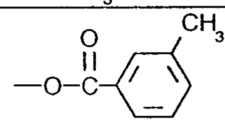 |
| $OCH_3$ | $OCH_3$ | O | $C(CH_3)_3$ | 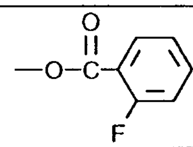 |
| $OCH_3$ | $OCH_3$ | O | $C(CH_3)_3$ | 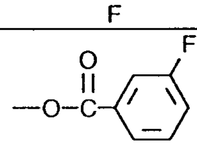 |
| $OCH_3$ | $OCH_3$ | O | $C(CH_3)_3$ | 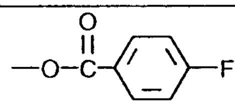 |
| $OCH_3$ | $OCH_3$ | O | $C(CH_3)_3$ | 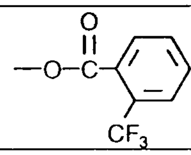 |
| $OCH_3$ | $OCH_3$ | O | $C(CH_3)_3$ | 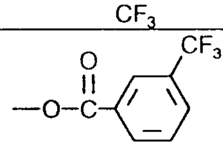 |
| $OCH_3$ | $OCH_3$ | O | $C(CH_3)_3$ | 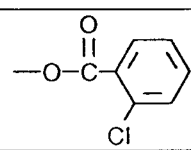 |
| $OCH_3$ | $OCH_3$ | O | $C(CH_3)_3$ | 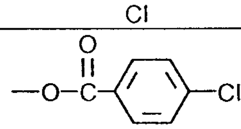 |

Table I (continued)

| R1 | R2 | X | R3 | R4 |
|---|---|---|---|---|
| $OCH_3$ | $OCH_3$ | O | $C(CH_3)_3$ | 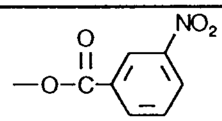 |
| $OCH_3$ | $OCH_3$ | O | $C(CH_3)_3$ | 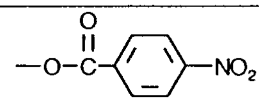 |
| $OCH_3$ | $OCH_3$ | O | $C(CH_3)_3$ | 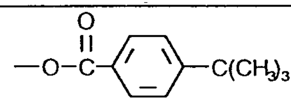 |
| $OCH_3$ | $OCH_3$ | O | $C(CH_3)_3$ | 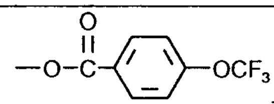 |
| $OCH_3$ | $OCH_3$ | O | $C(CH_3)_3$ | 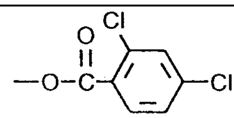 |
| $OCH_3$ | $OCH_3$ | O | $C(CH_3)_3$ | 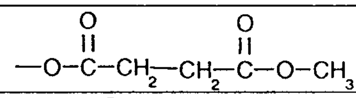 |
| $OCH_3$ | $OCH_3$ | O | $C(CH_3)_3$ | 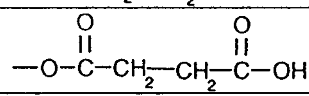 |
| $OCH_3$ | $OCH_3$ | O | $C(CH_3)_3$ | 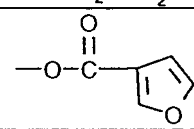 |
| $OCH_3$ | $OCH_3$ | O | $C(CH_3)_3$ | 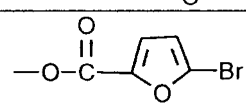 |
| $OCH_3$ | $OCH_3$ | O | $C(CH_3)_3$ | 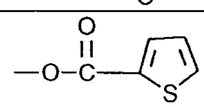 |
| $OCH_3$ | $OCH_3$ | O | $C(CH_3)_3$ | 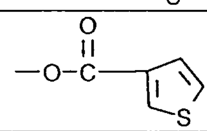 |
| $OCH_3$ | $OCH_3$ | O | $C(CH_3)_3$ | 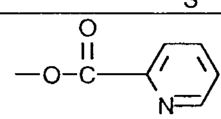 |
| $OCH_3$ | $OCH_3$ | O | $C(CH_3)_3$ | 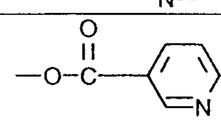 |
| $OCH_3$ | $OCH_3$ | O | $C(CH_3)_3$ | 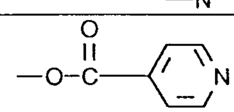 |

Table 1 (continued)

| R1 | R2 | X | R3 | R4 |
|---|---|---|---|---|
| $OCH_3$ | $OCH_3$ | O | $C(CH_3)_3$ | 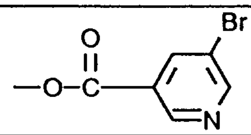 |
| $OCH_3$ | $OCH_3$ | O | $C(CH_3)_3$ | 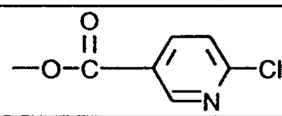 |
| $OCH_3$ | $OCH_3$ | O | $C(CH_3)_3$ | 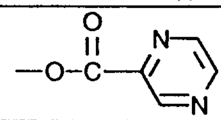 |
| $OCH_3$ | $OCH_3$ | O | $C(CH_3)_3$ | 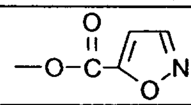 |
| $OCH_3$ | $OCH_3$ | O | $C(CH_3)_3$ | 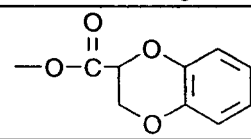 |
| $OCH_3$ | $OCH_3$ | O | $C(CH_3)_3$ | 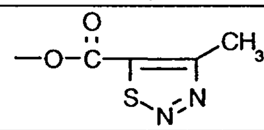 |
| $OCH_3$ | $OCH_3$ | O | $C(CH_3)_3$ | 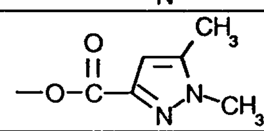 |
| $OCH_3$ | $OCH_3$ | O | $C(CH_3)_3$ | 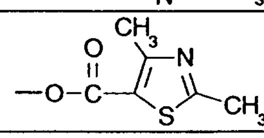 |
| $OCH_3$ | $OCH_3$ | O | $C(CH_3)_3$ | 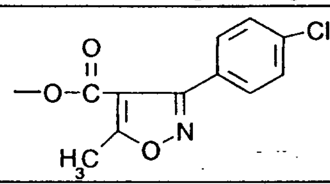 |
| $OCH_3$ | $OCH_3$ | O | $C(CH_3)_3$ | 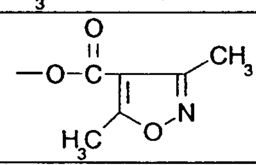 |
| $OCH_3$ | $OCH_3$ | O | $C(CH_3)_3$ | 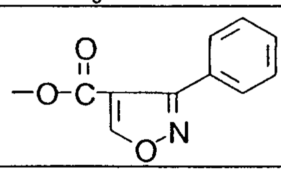 |

Table 1 (continued)

| R1 | R2 | X | R3 | R4 |
| --- | --- | --- | --- | --- |
| $OCH_3$ | $OCH_3$ | O | $C(CH_3)_3$ | 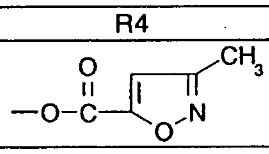 |
| $OCH_3$ | $OCH_3$ | O | $C(CH_3)_3$ | 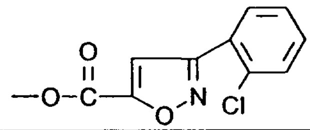 |
| $OCH_3$ | $OCH_3$ | O | $C(CH_3)_3$ | 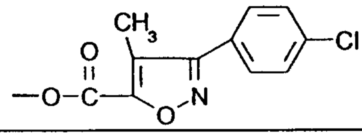 |
| $OCH_3$ | $OCH_3$ | O | $C(CH_3)_3$ | 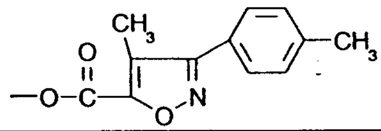 |
| $OCH_3$ | $OCH_3$ | O | $C(CH_3)_3$ | 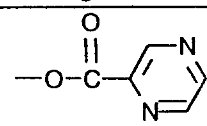 |
| $OCH_3$ | $OCH_3$ | O | $C(CH_3)_3$ | 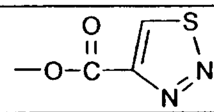 |
| $OCH_3$ | $OCH_3$ | O | $C(CH_3)_3$ | 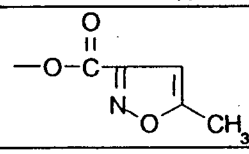 |
| $OCH_3$ | $OCH_3$ | O | $C(CH_3)_3$ | 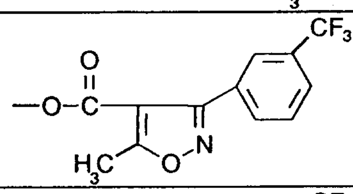 |
| $OCH_3$ | $OCH_3$ | O | $C(CH_3)_3$ | 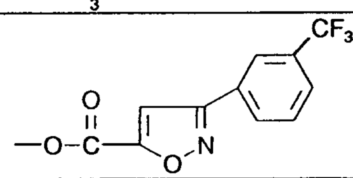 |
| $OCH_3$ | $OCH_3$ | O | $C(CH_3)_3$ | 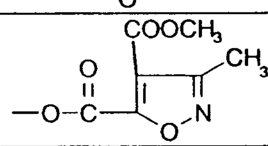 |
| $OCH_3$ | $OCH_3$ | O | $C(CH_3)_3$ | 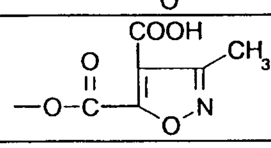 |

Table 1 (continued)

| R1 | R2 | X | R3 | R4 |
|---|---|---|---|---|
| $OCH_3$ | $OCH_3$ | S | $CH(CH_3)_2$ | 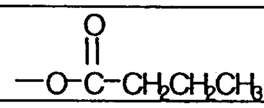 |
| $OCH_3$ | $OCH_3$ | S | $CH(CH_3)_2$ | 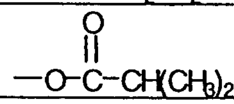 |
| $OCH_3$ | $OCH_3$ | S | $CH(CH_3)_2$ | 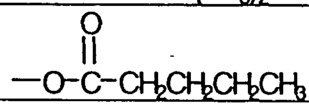 |
| $OCH_3$ | $OCH_3$ | S | $CH(CH_3)_2$ | 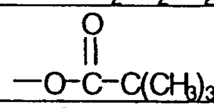 |
| $OCH_3$ | $OCH_3$ | S | $CH(CH_3)_2$ | 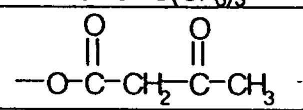 |
| $OCH_3$ | $OCH_3$ | S | $CH(CH_3)_2$ | 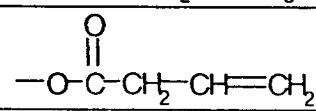 |
| $OCH_3$ | $OCH_3$ | S | $CH(CH_3)_2$ | 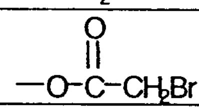 |
| $OCH_3$ | $OCH_3$ | S | $CH(CH_3)_2$ | 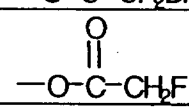 |
| $OCH_3$ | $OCH_3$ | S | $CH(CH_3)_2$ | 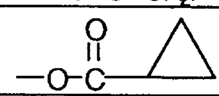 |
| $OCH_3$ | $OCH_3$ | S | $CH(CH_3)_2$ | 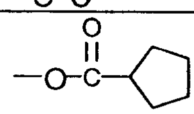 |
| $OCH_3$ | $OCH_3$ | S | $CH(CH_3)_2$ | 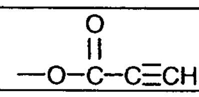 |
| $OCH_3$ | $OCH_3$ | S | $CH(CH_3)_2$ | 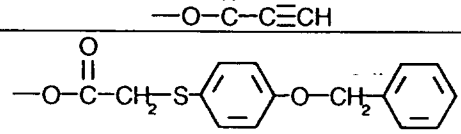 |
| $OCH_3$ | $OCH_3$ | S | $CH(CH_3)_2$ | 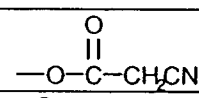 |
| $OCH_3$ | $OCH_3$ | S | $CH(CH_3)_2$ | 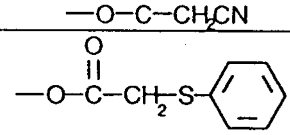 |
| $OCH_3$ | $OCH_3$ | S | $CH(CH_3)_2$ | 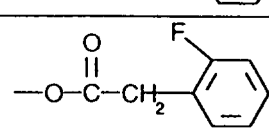 |

Table 1 (continued)

| R1 | R2 | X | R3 | R4 |
|---|---|---|---|---|
| $OCH_3$ | $OCH_3$ | S | $CH(CH_3)_2$ | 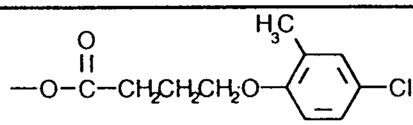 |
| $OCH_3$ | $OCH_3$ | S | $CH(CH_3)_2$ | 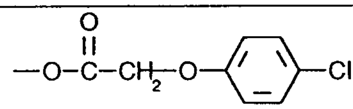 |
| $OCH_3$ | $OCH_3$ | S | $CH(CH_3)_2$ | 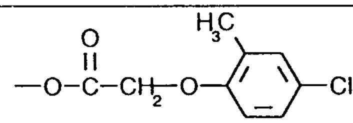 |
| $OCH_3$ | $OCH_3$ | S | $CH(CH_3)_2$ | 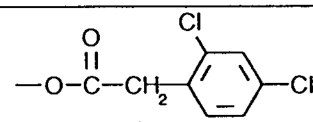 |
| $OCH_3$ | $OCH_3$ | S | $CH(CH_3)_2$ | 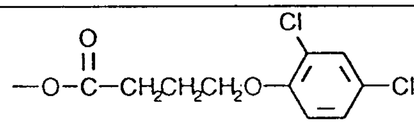 |
| $OCH_3$ | $OCH_3$ | S | $CH(CH_3)_2$ | 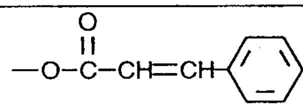 |
| $OCH_3$ | $OCH_3$ | S | $CH(CH_3)_2$ | 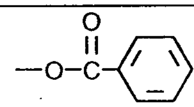 |
| $OCH_3$ | $OCH_3$ | S | $CH(CH_3)_2$ | 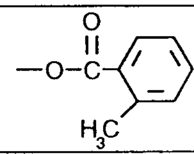 |
| $OCH_3$ | $OCH_3$ | S | $CH(CH_3)_2$ | 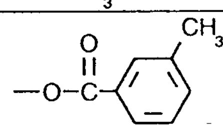 |
| $OCH_3$ | $OCH_3$ | S | $CH(CH_3)_2$ | 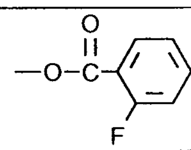 |
| $OCH_3$ | $OCH_3$ | S | $CH(CH_3)_2$ | 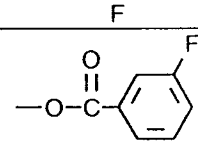 |
| $OCH_3$ | $OCH_3$ | S | $CH(CH_3)_2$ | 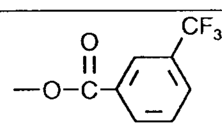 |

Table 1 (continued)

| R1 | R2 | X | R3 | R4 |
|---|---|---|---|---|
| $OCH_3$ | $OCH_3$ | S | $CH(CH_3)_2$ | 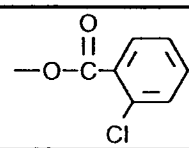 |
| $OCH_3$ | $OCH_3$ | S | $CH(CH_3)_2$ | 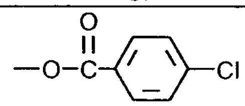 |
| $OCH_3$ | $OCH_3$ | S | $CH(CH_3)_2$ | 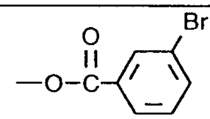 |
| $OCH_3$ | $OCH_3$ | S | $CH(CH_3)_2$ | 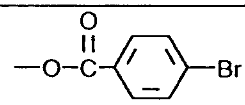 |
| $OCH_3$ | $OCH_3$ | S | $CH(CH_3)_2$ | 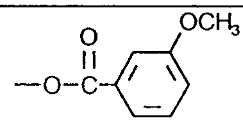 |
| $OCH_3$ | $OCH_3$ | S | $CH(CH_3)_2$ | 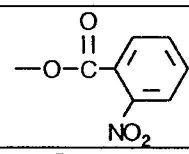 |
| $OCH_3$ | $OCH_3$ | S | $CH(CH_3)_2$ | 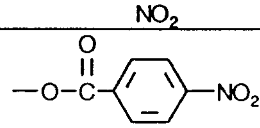 |
| $OCH_3$ | $OCH_3$ | S | $CH(CH_3)_2$ | 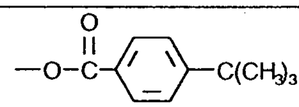 |
| $OCH_3$ | $OCH_3$ | S | $CH(CH_3)_2$ | 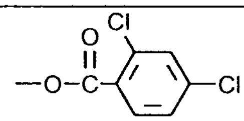 |
| $OCH_3$ | $OCH_3$ | S | $CH(CH_3)_2$ | 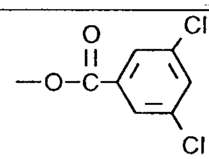 |
| $OCH_3$ | $OCH_3$ | S | $CH(CH_3)_2$ | 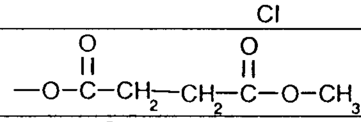 |
| $OCH_3$ | $OCH_3$ | S | $CH(CH_3)_2$ | 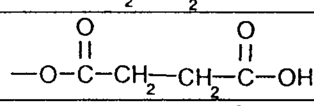 |
| $OCH_3$ | $OCH_3$ | S | $CH(CH_3)_2$ | 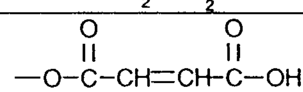 |

Table 1 (continued)

| R1 | R2 | X | R3 | R4 |
|---|---|---|---|---|
| $OCH_3$ | $OCH_3$ | S | $CH(CH_3)_2$ | 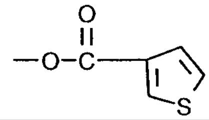 |
| $OCH_3$ | $OCH_3$ | S | $CH(CH_3)_2$ | 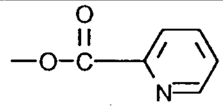 |
| $OCH_3$ | $OCH_3$ | S | $CH(CH_3)_2$ | 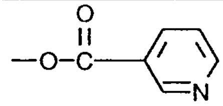 |
| $OCH_3$ | $OCH_3$ | S | $CH(CH_3)_2$ | 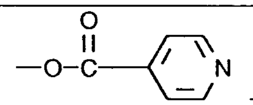 |
| $OCH_3$ | $OCH_3$ | S | $CH(CH_3)_2$ | 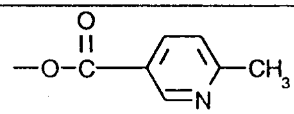 |
| $OCH_3$ | $OCH_3$ | S | $CH(CH_3)_2$ | 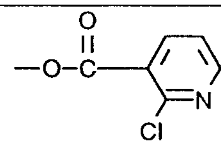 |
| $OCH_3$ | $OCH_3$ | S | $CH(CH_3)_2$ | 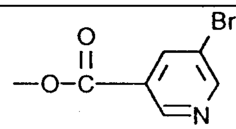 |
| $OCH_3$ | $OCH_3$ | S | $CH(CH_3)_2$ | 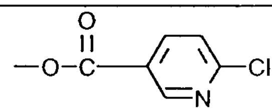 |
| $OCH_3$ | $OCH_3$ | S | $CH(CH_3)_2$ | 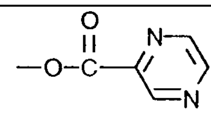 |
| $OCH_3$ | $OCH_3$ | S | $CH(CH_3)_2$ | 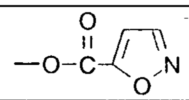 |
| $OCH_3$ | $OCH_3$ | S | $CH(CH_3)_2$ | 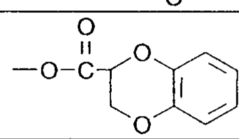 |
| $OCH_3$ | $OCH_3$ | S | $CH(CH_3)_2$ | 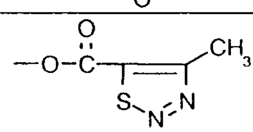 |
| $OCH_3$ | $OCH_3$ | S | $CH(CH_3)_2$ | 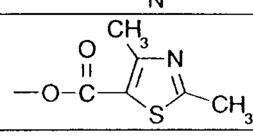 |

Table 1 (continued)

| R1 | R2 | X | R3 | R4 |
|---|---|---|---|---|
| $OCH_3$ | $OCH_3$ | S | $CH(CH_3)_2$ | 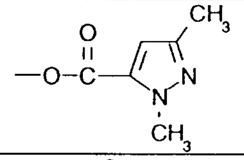 |
| $OCH_3$ | $OCH_3$ | | $CH(CH_3)_2$ | 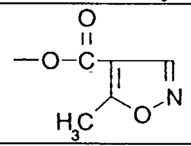 |
| $OCH_3$ | $OCH_3$ | S | $CH(CH_3)_2$ | 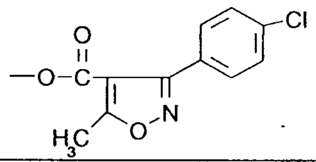 |
| $OCH_3$ | $OCH_3$ | S | $CH(CH_3)_2$ | 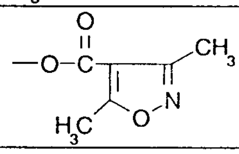 |
| $OCH_3$ | $OCH_3$ | S | $CH(CH_3)_2$ | 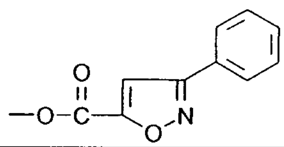 |
| $OCH_3$ | $OCH_3$ | S | $CH(CH_3)_2$ | 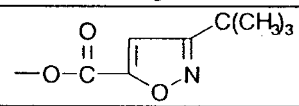 |
| $OCH_3$ | $OCH_3$ | S | $CH(CH_3)_2$ | 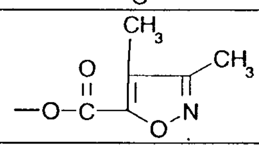 |
| $OCH_3$ | $OCH_3$ | S | $CH(CH_3)_2$ | 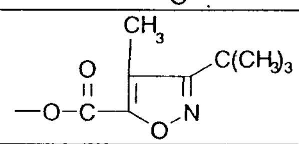 |
| $OCH_3$ | $OCH_3$ | S | $CH(CH_3)_2$ | 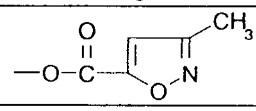 |
| $OCH_3$ | $OCH_3$ | S | $CH(CH_3)_2$ | 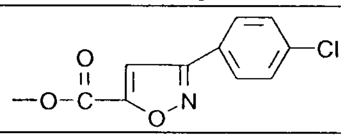 |
| $OCH_3$ | $OCH_3$ | S | $CH(CH_3)_2$ | 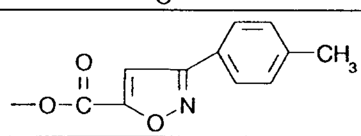 |

Table 1 (continued)

| R1 | R2 | X | R3 | R4 |
|---|---|---|---|---|
| $OCH_3$ | $OCH_3$ | S | $CH(CH_3)_2$ | 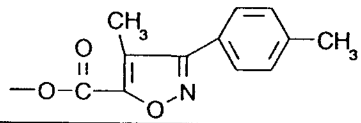 |
| $OCH_3$ | $OCH_3$ | S | $CH(CH_3)_2$ | 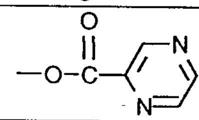 |
| $OCH_3$ | $OCH_3$ | S | $CH(CH_3)_2$ | 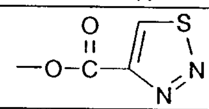 |
| $OCH_3$ | $OCH_3$ | S | $CH(CH_3)_2$ | 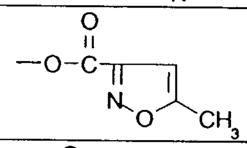 |
| $OCH_3$ | $OCH_3$ | S | $CH(CH_3)_2$ | 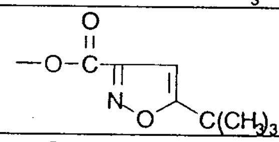 |
| $OCH_3$ | $OCH_3$ | S | $CH(CH_3)_2$ | 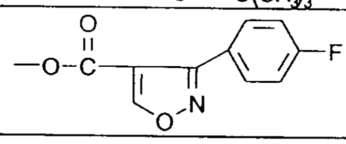 |
| $OCH_3$ | $OCH_3$ | S | $CH(CH_3)_2$ | 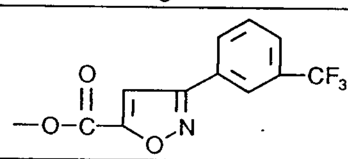 |
| $OCH_3$ | $OCH_3$ | S | $CH(CH_3)_2$ | 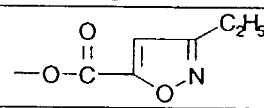 |
| $OCH_3$ | $OCH_3$ | S | $CH(CH_3)_2$ | 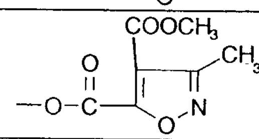 |
| $OCH_3$ | $OCH_3$ | S | $CH(CH_3)_2$ | 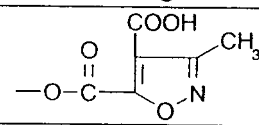 |
| $OCH_3$ | $OCH_3$ | S | $CH(CH_3)_2$ | 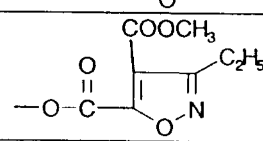 |
| $OCH_3$ | $OCH_3$ | S | $CH(CH_3)_2$ | 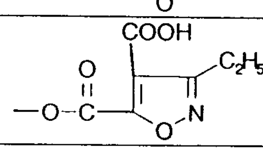 |

Table 1 (continued)

| R1 | R2 | X | R3 | R4 |
|---|---|---|---|---|
| $OCH_3$ | $OCH_3$ | S | $C(CH_3)_3$ | 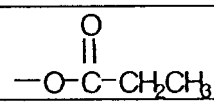 |
| $OCH_3$ | $OCH_3$ | S | $C(CH_3)_3$ | 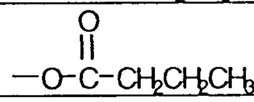 |
| $OCH_3$ | $OCH_3$ | S | $C(CH_3)_3$ | 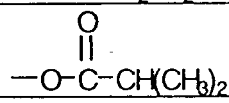 |
| $OCH_3$ | $OCH_3$ | S | $C(CH_3)_3$ | 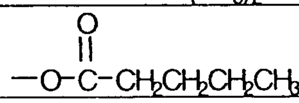 |
| $OCH_3$ | $OCH_3$ | S | $C(CH_3)_3$ | 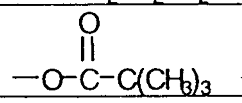 |
| $OCH_3$ | $OCH_3$ | S | $C(CH_3)_3$ | 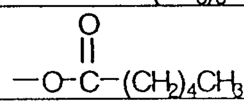 |
| $OCH_3$ | $OCH_3$ | S | $C(CH_3)_3$ | 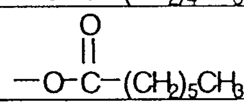 |
| $OCH_3$ | $OCH_3$ | S | $C(CH_3)_3$ | 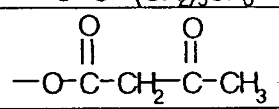 |
| $OCH_3$ | $OCH_3$ | S | $C(CH_3)_3$ | 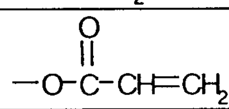 |
| $OCH_3$ | $OCH_3$ | S | $C(CH_3)_3$ | 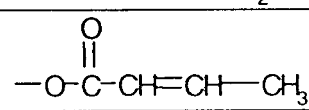 |
| $OCH_3$ | $OCH_3$ | S | $C(CH_3)_3$ | 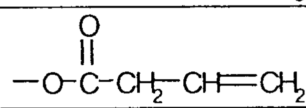 |
| $OCH_3$ | $OCH_3$ | S | $C(CH_3)_3$ | 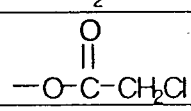 |
| $OCH_3$ | $OCH_3$ | S | $C(CH_3)_3$ | 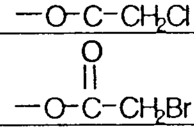 |
| $OCH_3$ | $OCH_3$ | S | $C(CH_3)_3$ | 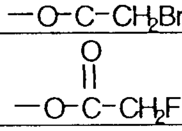 |
| $OCH_3$ | $OCH_3$ | S | $C(CH_3)_3$ | 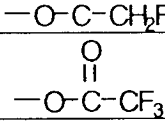 |
| $OCH_3$ | $OCH_3$ | S | $C(CH_3)_3$ | 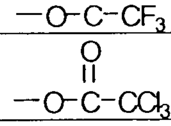 |

Table 1 (continued)

| R1 | R2 | X | R3 | R4 |
|---|---|---|---|---|
| $OCH_3$ | $OCH_3$ | S | $C(CH_3)_3$ | 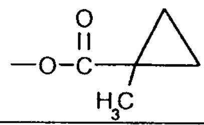 |
| $OCH_3$ | $OCH_3$ | S | $C(CH_3)_3$ | 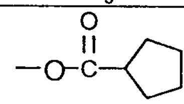 |
| $OCH_3$ | $OCH_3$ | S | $C(CH_3)_3$ | 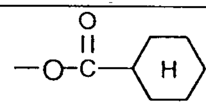 |
| $OCH_3$ | $OCH_3$ | S | $C(CH_3)_3$ | 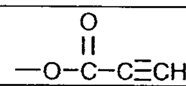 |
| $OCH_3$ | $OCH_3$ | S | $C(CH_3)_3$ | 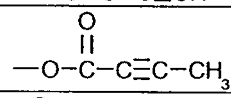 |
| $OCH_3$ | $OCH_3$ | S | $C(CH_3)_3$ | 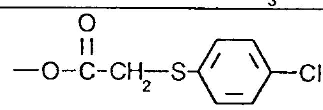 |
| $OCH_3$ | $OCH_3$ | S | $C(CH_3)_3$ | 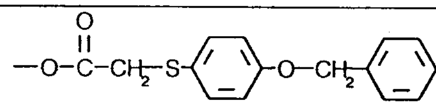 |
| $OCH_3$ | $OCH_3$ | S | $C(CH_3)_3$ | 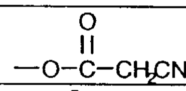 |
| $OCH_3$ | $OCH_3$ | S | $C(CH_3)_3$ | 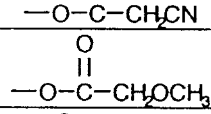 |
| $OCH_3$ | $OCH_3$ | S | $C(CH_3)_3$ | 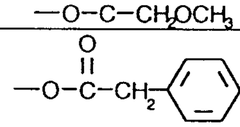 |
| $OCH_3$ | $OCH_3$ | S | $C(CH_3)_3$ | 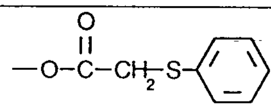 |
| $OCH_3$ | $OCH_3$ | S | $C(CH_3)_3$ | 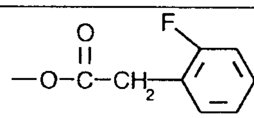 |
| $OCH_3$ | $OCH_3$ | S | $C(CH_3)_3$ | 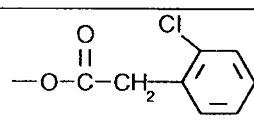 |
| $OCH_3$ | $OCH_3$ | S | $C(CH_3)_3$ | 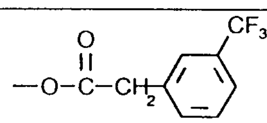 |

Table 1 (continued)

| R1 | R2 | X | R3 | R4 |
|---|---|---|---|---|
| $OCH_3$ | $OCH_3$ | S | $C(CH_3)_3$ | 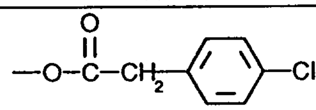 |
| $OCH_3$ | $OCH_3$ | S | $C(CH_3)_3$ | 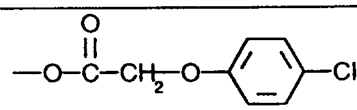 |
| $OCH_3$ | $OCH_3$ | S | $C(CH_3)_3$ | 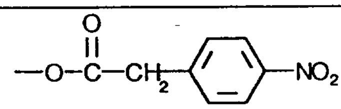 |
| $OCH_3$ | $OCH_3$ | S | $C(CH_3)_3$ | 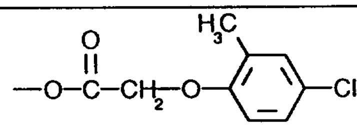 |
| $OCH_3$ | $OCH_3$ | S | $C(CH_3)_3$ | 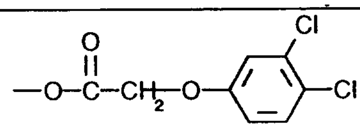 |
| $OCH_3$ | $OCH_3$ | S | $C(CH_3)_3$ | 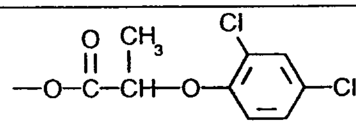 |
| $OCH_3$ | $OCH_3$ | S | $C(CH_3)_3$ | 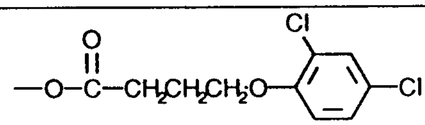 |
| $OCH_3$ | $OCH_3$ | S | $C(CH_3)_3$ | 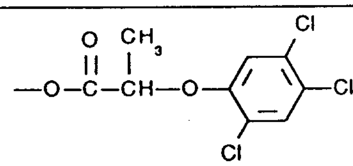 |
| $OCH_3$ | $OCH_3$ | S | $C(CH_3)_3$ | 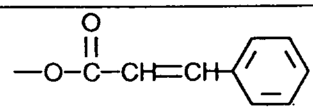 |
| $OCH_3$ | $OCH_3$ | S | $C(CH_3)_3$ | 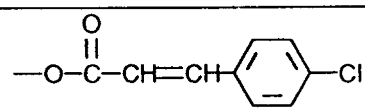 |
| $OCH_3$ | $OCH_3$ | S | $C(CH_3)_3$ | 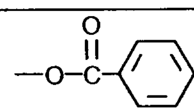 |
| $OCH_3$ | $OCH_3$ | S | $C(CH_3)_3$ | 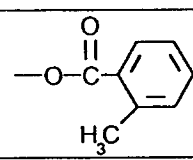 |

Table 1 (continued)

| R1 | R2 | X | R3 | R4 |
|---|---|---|---|---|
| $OCH_3$ | $OCH_3$ | S | $C(CH_3)_3$ | 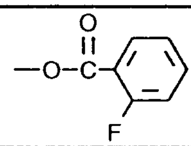 |
| $OCH_3$ | $OCH_3$ | S | $C(CH_3)_3$ | 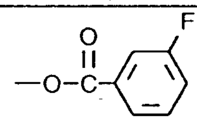 |
| $OCH_3$ | $OCH_3$ | S | $C(CH_3)_3$ | 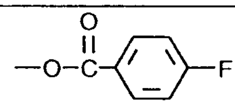 |
| $OCH_3$ | $OCH_3$ | S | $C(CH_3)_3$ | 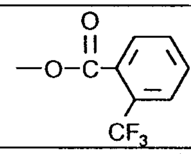 |
| $OCH_3$ | $OCH_3$ | S | $C(CH_3)_3$ | 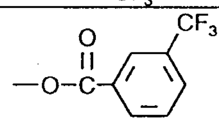 |
| $OCH_3$ | $OCH_3$ | S | $C(CH_3)_3$ | 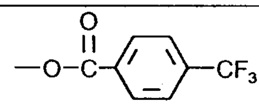 |
| $OCH_3$ | $OCH_3$ | S | $C(CH_3)_3$ | 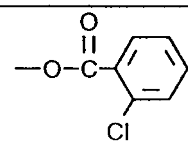 |
| $OCH_3$ | $OCH_3$ | S | $C(CH_3)_3$ | 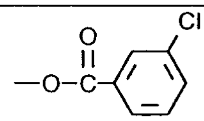 |
| $OCH_3$ | $OCH_3$ | S | $C(CH_3)_3$ | 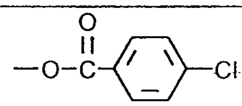 |
| $OCH_3$ | $OCH_3$ | S | $C(CH_3)_3$ | 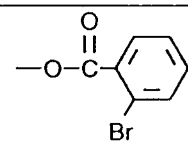 |
| $OCH_3$ | $OCH_3$ | S | $C(CH_3)_3$ | 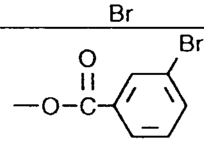 |
| $OCH_3$ | $OCH_3$ | S | $C(CH_3)_3$ | 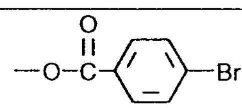 |

Table 1 (continued)

| R1 | R2 | X | R3 | R4 |
|---|---|---|---|---|
| $OCH_3$ | $OCH_3$ | S | $C(CH_3)_3$ | 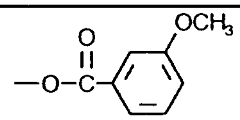 |
| $OCH_3$ | $OCH_3$ | S | $C(CH_3)_3$ | 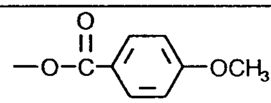 |
| $OCH_3$ | $OCH_3$ | S | $C(CH_3)_3$ | 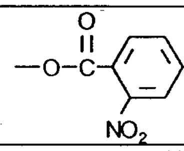 |
| $OCH_3$ | $OCH_3$ | S | $C(CH_3)_3$ | 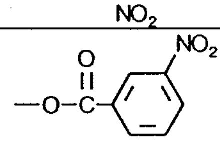 |
| $OCH_3$ | $OCH_3$ | S | $C(CH_3)_3$ | 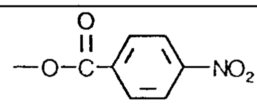 |
| $OCH_3$ | $OCH_3$ | S | $C(CH_3)_3$ | 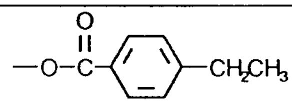 |
| $OCH_3$ | $OCH_3$ | S | $C(CH_3)_3$ | 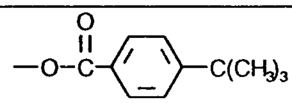 |
| $OCH_3$ | $OCH_3$ | S | $C(CH_3)_3$ | 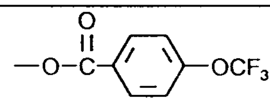 |
| $OCH_3$ | $OCH_3$ | S | $C(CH_3)_3$ | 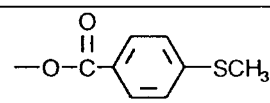 |
| $OCH_3$ | $OCH_3$ | S | $C(CH_3)_3$ | 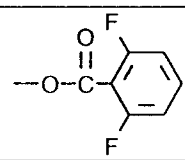 |
| $OCH_3$ | $OCH_3$ | S | $C(CH_3)_3$ | 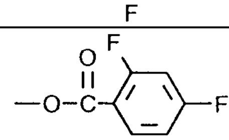 |
| $OCH_3$ | $OCH_3$ | S | $C(CH_3)_3$ | 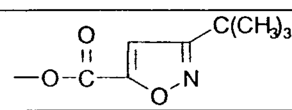 |
| $OCH_3$ | $OCH_3$ | S | $C(CH_3)_3$ | 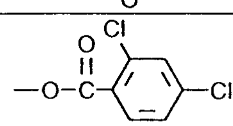 |

Table 1 (continued)

| R1 | R2 | X | R3 | R4 |
|---|---|---|---|---|
| $OCH_3$ | $OCH_3$ | S | $C(CH_3)_3$ | 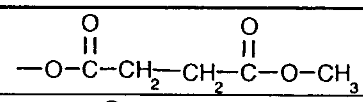 |
| $OCH_3$ | $OCH_3$ | S | $C(CH_3)_3$ | 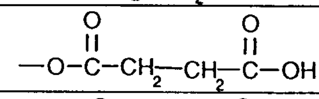 |
| $OCH_3$ | $OCH_3$ | S | $C(CH_3)_3$ | 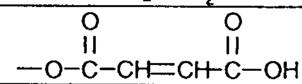 |
| $OCH_3$ | $OCH_3$ | S | $C(CH_3)_3$ | 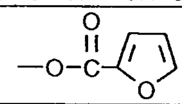 |
| $OCH_3$ | $OCH_3$ | S | $C(CH_3)_3$ | 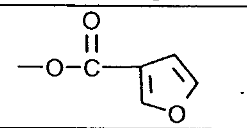 |
| $OCH_3$ | $OCH_3$ | S | $C(CH_3)_3$ | 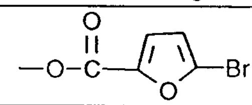 |
| $OCH_3$ | $OCH_3$ | S | $C(CH_3)_3$ | 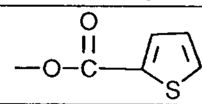 |
| $OCH_3$ | $OCH_3$ | S | $C(CH_3)_3$ | 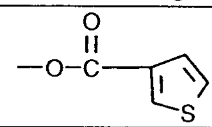 |
| $OCH_3$ | $OCH_3$ | S | $C(CH_3)_3$ | 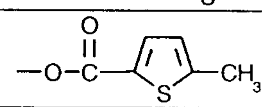 |
| $OCH_3$ | $OCH_3$ | S | $C(CH_3)_3$ | 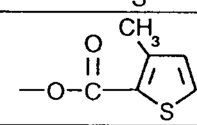 |
| $OCH_3$ | $OCH_3$ | S | $C(CH_3)_3$ | 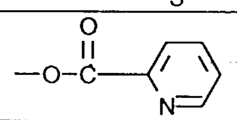 |
| $OCH_3$ | $OCH_3$ | S | $C(CH_3)_3$ | 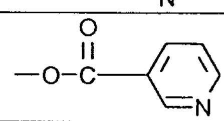 |
| $OCH_3$ | $OCH_3$ | S | $C(CH_3)_3$ | 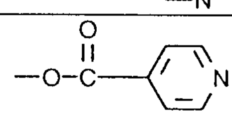 |
| $OCH_3$ | $OCH_3$ | S | $C(CH_3)_3$ | 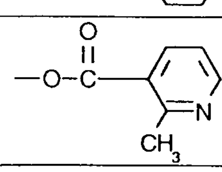 |

Table 1 (continued)

| R1 | R2 | X | R3 | R4 |
|---|---|---|---|---|
| $OCH_3$ | $OCH_3$ | S | $C(CH_3)_3$ | 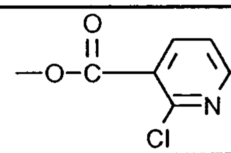 |
| $OCH_3$ | $OCH_3$ | S | $C(CH_3)_3$ | 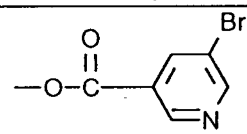 |
| $OCH_3$ | $OCH_3$ | S | $C(CH_3)_3$ | 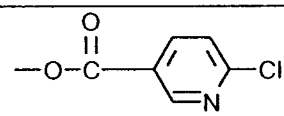 |
| $OCH_3$ | $OCH_3$ | S | $C(CH_3)_3$ | 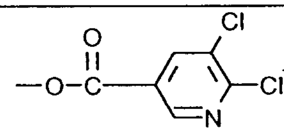 |
| $OCH_3$ | $OCH_3$ | S | $C(CH_3)_3$ | 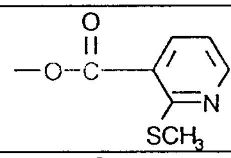 |
| $OCH_3$ | $OCH_3$ | S | $C(CH_3)_3$ | 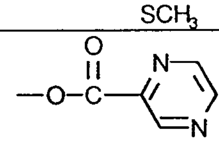 |
| $OCH_3$ | $OCH_3$ | S | $C(CH_3)_3$ | 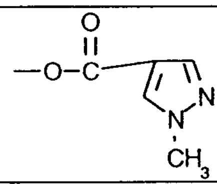 |
| $OCH_3$ | $OCH_3$ | S | $C(CH_3)_3$ | 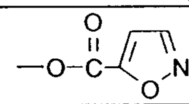 |
| $OCH_3$ | $OCH_3$ | S | $C(CH_3)_3$ | 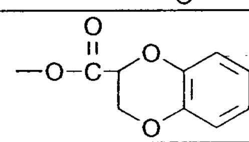 |
| $OCH_3$ | $OCH_3$ | S | $C(CH_3)_3$ | 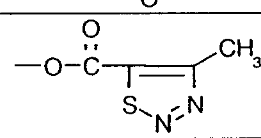 |
| $OCH_3$ | $OCH_3$ | S | $C(CH_3)_3$ | 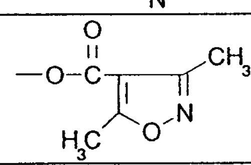 |

Table 1 (continued)

| R1 | R2 | X | R3 | R4 |
|---|---|---|---|---|
| $OCH_3$ | $OCH_3$ | S | $C(CH_3)_3$ | 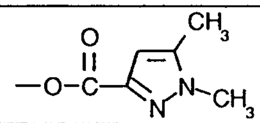 |
| $OCH_3$ | $OCH_3$ | S | $C(CH_3)_3$ | 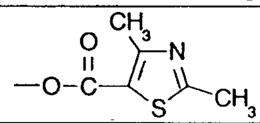 |
| $OCH_3$ | $OCH_3$ | S | $C(CH_3)_3$ | 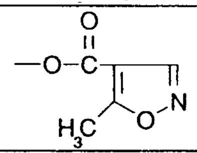 |
| $OCH_3$ | $OCH_3$ | S | $C(CH_3)_3$ | 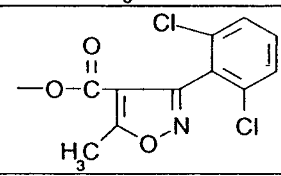 |
| $OCH_3$ | $OCH_3$ | S | $C(CH_3)_3$ | 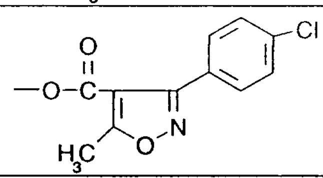 |
| $OCH_3$ | $OCH_3$ | S | $C(CH_3)_3$ | 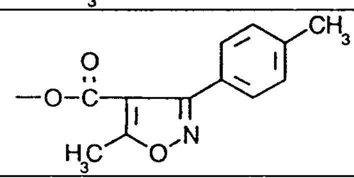 |
| $OCH_3$ | $OCH_3$ | S | $C(CH_3)_3$ | 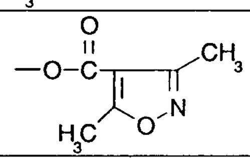 |
| $OCH_3$ | $OCH_3$ | S | $C(CH_3)_3$ | 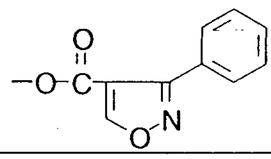 |
| $OCH_3$ | $OCH_3$ | S | $C(CH_3)_3$ | 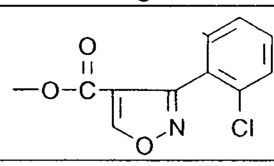 |
| $OCH_3$ | $OCH_3$ | S | $C(CH_3)_3$ | 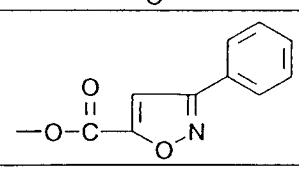 |

Table 1 (continued)

| R1 | R2 | X | R3 | R4 |
|---|---|---|---|---|
| $OCH_3$ | $OCH_3$ | S | $C(CH_3)_3$ | 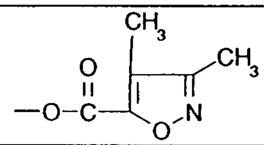 |
| $OCH_3$ | $OCH_3$ | S | $C(CH_3)_3$ | 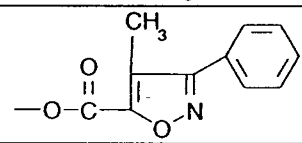 |
| $OCH_3$ | $OCH_3$ | S | $C(CH_3)_3$ | 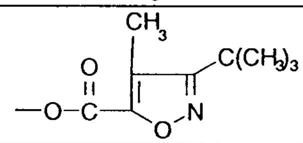 |
| $OCH_3$ | $OCH_3$ | S | $C(CH_3)_3$ | 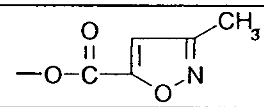 |
| $OCH_3$ | $OCH_3$ | S | $C(CH_3)_3$ | 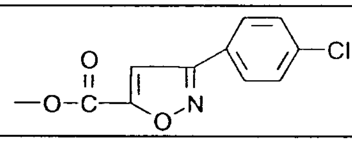 |
| $OCH_3$ | $OCH_3$ | S | $C(CH_3)_3$ | 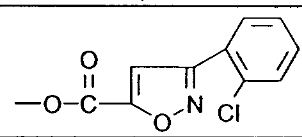 |
| $OCH_3$ | $OCH_3$ | S | $C(CH_3)_3$ | 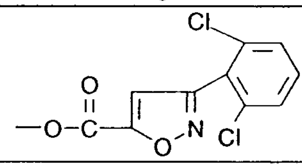 |
| $OCH_3$ | $OCH_3$ | S | $C(CH_3)_3$ | 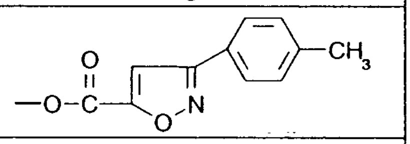 |
| $OCH_3$ | $OCH_3$ | S | $C(CH_3)_3$ | 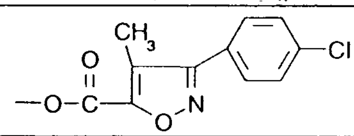 |
| $OCH_3$ | $OCH_3$ | S | $C(CH_3)_3$ | 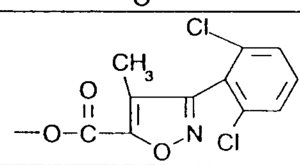 |
| $OCH_3$ | $OCH_3$ | S | $C(CH_3)_3$ | 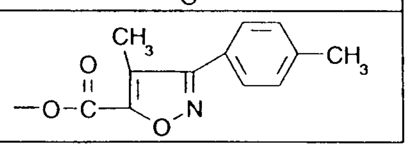 |

Table 1 (continued)

| R1 | R2 | X | R3 | R4 |
|---|---|---|---|---|
| $OCH_3$ | $OCH_3$ | S | $C(CH_3)_3$ | 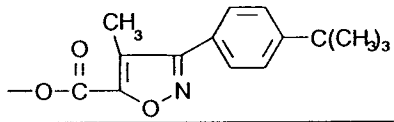 |
| $OCH_3$ | $OCH_3$ | S | $C(CH_3)_3$ | 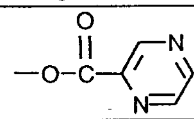 |
| $OCH_3$ | $OCH_3$ | S | $C(CH_3)_3$ | 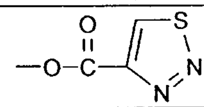 |
| $OCH_3$ | $OCH_3$ | S | $C(CH_3)_3$ | 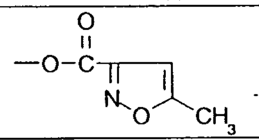 |
| $OCH_3$ | $OCH_3$ | S | $C(CH_3)_3$ | 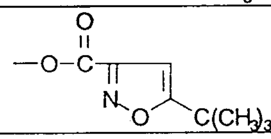 |
| $OCH_3$ | $OCH_3$ | S | $C(CH_3)_3$ | 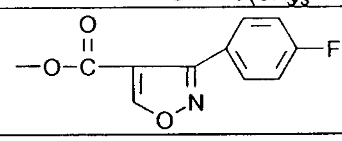 |
| $OCH_3$ | $OCH_3$ | S | $C(CH_3)_3$ | 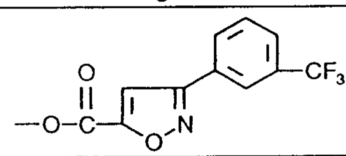 |
| $OCH_3$ | $OCH_3$ | S | $C(CH_3)_3$ | 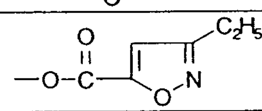 |
| $OCH_3$ | $OCH_3$ | S | $C(CH_3)_3$ | 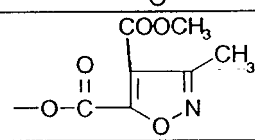 |
| $OCH_3$ | $OCH_3$ | S | $C(CH_3)_3$ | 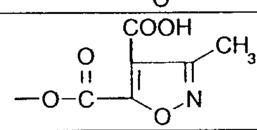 |
| $OCH_3$ | $OCH_3$ | S | $C(CH_3)_3$ | 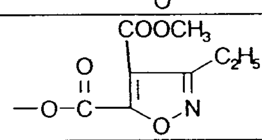 |
| $OCH_3$ | $OCH_3$ | S | $C(CH_3)_3$ | 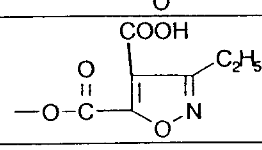 |

Table 1 (continued)

| R1 | R2 | X | R3 | R4 |
|---|---|---|---|---|
| $OCH_3$ | $OCH_3$ | O | $CH(CH_3)_2$ | $—O—SO_2—CH_3$ |
| $OCH_3$ | $OCH_3$ | O | $CH(CH_3)_2$ | $—O—SO_2—C_2H_5$ |
| $OCH_3$ | $OCH_3$ | O | $CH(CH_3)_2$ | $—O—SO_2—CH_2CH_2CH_3$ |
| $OCH_3$ | $OCH_3$ | O | $CH(CH_3)_2$ | $—O—SO_2—CH(CH_3)_2$ |
| $OCH_3$ | $OCH_3$ | O | $CH(CH_3)_2$ | $—O—SO_2—CH_2CH_2CH_2CH_3$ |
| $OCH_3$ | $OCH_3$ | O | $CH(CH_3)_2$ | $—O—SO_2—C(CH_3)_3$ |
| $OCH_3$ | $OCH_3$ | O | $CH(CH_3)_2$ | $—O—SO_2—CF_3$ |
| $OCH_3$ | $OCH_3$ | O | $CH(CH_3)_2$ | $—O—SO_2—CH=CH_2$ |
| $OCH_3$ | $OCH_3$ | O | $CH(CH_3)_2$ | $—O—SO_2—CH_2CF_3$ |
| $OCH_3$ | $OCH_3$ | O | $CH(CH_3)_2$ | 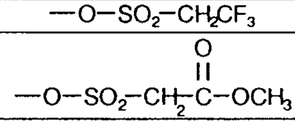 |
| $OCH_3$ | $OCH_3$ | O | $CH(CH_3)_2$ | 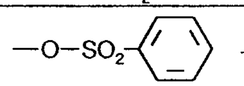 |
| $OCH_3$ | $OCH_3$ | O | $CH(CH_3)_2$ | 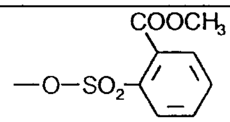 |
| $OCH_3$ | $OCH_3$ | O | $CH(CH_3)_2$ | 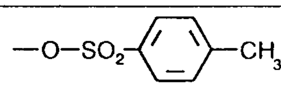 |
| $OCH_3$ | $OCH_3$ | O | $CH(CH_3)_2$ | 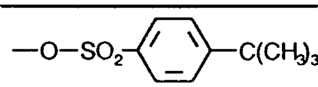 |
| $OCH_3$ | $OCH_3$ | O | $CH(CH_3)_2$ | 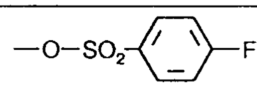 |
| $OCH_3$ | $OCH_3$ | O | $CH(CH_3)_2$ | 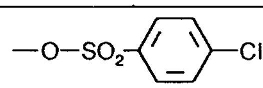 |
| $OCH_3$ | $OCH_3$ | O | $CH(CH_3)_2$ | 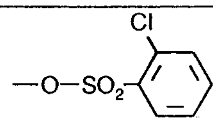 |
| $OCH_3$ | $OCH_3$ | O | $CH(CH_3)_2$ | 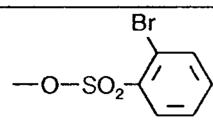 |
| $OCH_3$ | $OCH_3$ | O | $CH(CH_3)_2$ | 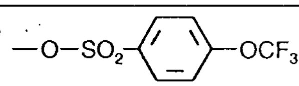 |
| $OCH_3$ | $OCH_3$ | O | $CH(CH_3)_2$ | 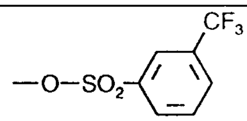 |
| $OCH_3$ | $OCH_3$ | O | $CH(CH_3)_2$ | 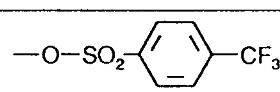 |

Table 1 (continued)

| R1 | R2 | X | R3 | R4 |
|---|---|---|---|---|
| $OCH_3$ | $OCH_3$ | O | $CH(CH_3)_2$ | 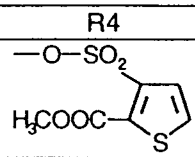 |
| $OCH_3$ | $OCH_3$ | O | $C(CH_3)_3$ | $-O-SO_2-CH_3$ |
| $OCH_3$ | $OCH_3$ | O | $C(CH_3)_3$ | $-O-SO_2-C_2H_5$ |
| $OCH_3$ | $OCH_3$ | O | $C(CH_3)_3$ | $-O-SO_2-CH_2CH_2CH_3$ |
| $OCH_3$ | $OCH_3$ | O | $C(CH_3)_3$ | $-O-SO_2-CH(CH_3)_2$ |
| $OCH_3$ | $OCH_3$ | O | $C(CH_3)_3$ | $-O-SO_2-CH_2CH_2CH_2CH_3$ |
| $OCH_3$ | $OCH_3$ | O | $C(CH_3)_3$ | $-O-SO_2-C(CH_3)_3$ |
| $OCH_3$ | $OCH_3$ | O | $C(CH_3)_3$ | $-O-SO_2-CF_3$ |
| $OCH_3$ | $OCH_3$ | O | $C(CH_3)_3$ | $-O-SO_2-CH=CH_2$ |
| $OCH_3$ | $OCH_3$ | O | $C(CH_3)_3$ | $-O-SO_2-CH_2CF_3$ |
| $OCH_3$ | $OCH_3$ | O | $C(CH_3)_3$ | 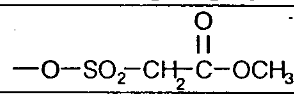 |
| $OCH_3$ | $OCH_3$ | O | $C(CH_3)_3$ | 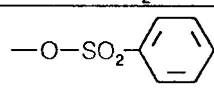 |
| $OCH_3$ | $OCH_3$ | O | $C(CH_3)_3$ | 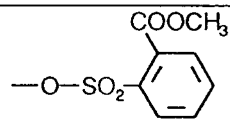 |
| $OCH_3$ | $OCH_3$ | O | $C(CH_3)_3$ | 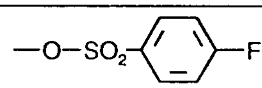 |
| $OCH_3$ | $OCH_3$ | O | $C(CH_3)_3$ | 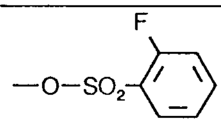 |
| $OCH_3$ | $OCH_3$ | O | $C(CH_3)_3$ | 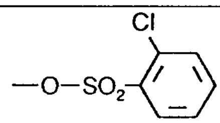 |
| $OCH_3$ | $OCH_3$ | O | $C(CH_3)_3$ | 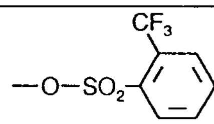 |
| $OCH_3$ | $OCH_3$ | O | $C(CH_3)_3$ | 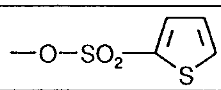 |
| $OCH_3$ | $OCH_3$ | O | $C(CH_3)_3$ | 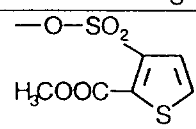 |
| $OCH_3$ | $OCH_3$ | S | $CH(CH_3)_2$ | $-O-SO_2-CH_3$ |
| $OCH_3$ | $OCH_3$ | S | $CH(CH_3)_2$ | $-O-SO_2-C_2H_5$ |
| $OCH_3$ | $OCH_3$ | S | $CH(CH_3)_2$ | $-O-SO_2-CH_2CH_2CH_3$ |

Table 1 (continued)

| R1 | R2 | X | R3 | R4 |
|---|---|---|---|---|
| $OCH_3$ | $OCH_3$ | S | $CH(CH_3)_2$ | $—O—SO_2—CH(CH_3)_2$ |
| $OCH_3$ | $OCH_3$ | S | $CH(CH_3)_2$ | $—O—SO_2—CH_2CH_2CH_2CH_3$ |
| $OCH_3$ | $OCH_3$ | S | $CH(CH_3)_2$ | $—O—SO_2—CF_3$ |
| $OCH_3$ | $OCH_3$ | S | $CH(CH_3)_2$ | $—O—SO_2—CH{=}CH_2$ |
| $OCH_3$ | $OCH_3$ | S | $CH(CH_3)_2$ | $—O—SO_2—CH_2CF_3$ |
| $OCH_3$ | $OCH_3$ | S | $CH(CH_3)_2$ | 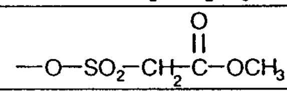 |
| $OCH_3$ | $OCH_3$ | S | $CH(CH_3)_2$ | 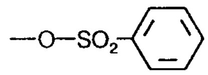 |
| $OCH_3$ | $OCH_3$ | S | $CH(CH_3)_2$ | 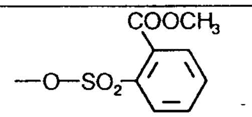 |
| $OCH_3$ | $OCH_3$ | S | $CH(CH_3)_2$ | 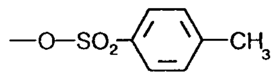 |
| $OCH_3$ | $OCH_3$ | S | $CH(CH_3)_2$ | 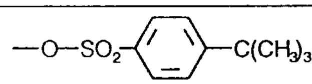 |
| $OCH_3$ | $OCH_3$ | S | $CH(CH_3)_2$ | 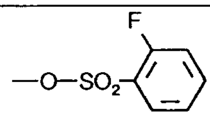 |
| $OCH_3$ | $OCH_3$ | S | $CH(CH_3)_2$ | 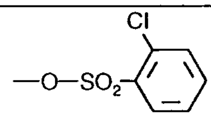 |
| $OCH_3$ | $OCH_3$ | S | $CH(CH_3)_2$ | 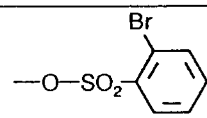 |
| $OCH_3$ | $OCH_3$ | S | $CH(CH_3)_2$ | 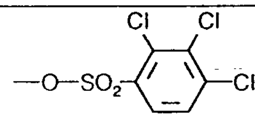 |
| $OCH_3$ | $OCH_3$ | S | $CH(CH_3)_2$ | 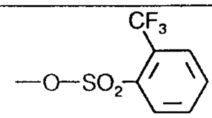 |
| $OCH_3$ | $OCH_3$ | S | $CH(CH_3)_2$ | 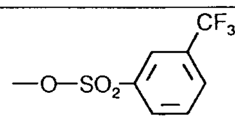 |
| $OCH_3$ | $OCH_3$ | S | $CH(CH_3)_2$ | 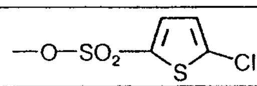 |
| $OCH_3$ | $OCH_3$ | S | $C(CH_3)_3$ | $—O—SO_2—CH_3$ |
| $OCH_3$ | $OCH_3$ | S | $C(CH_3)_3$ | $—O—SO_2—C_2H_5$ |

Table 1 (continued)

| R1 | R2 | X | R3 | R4 |
|---|---|---|---|---|
| $OCH_3$ | $OCH_3$ | S | $C(CH_3)_3$ | $-O-SO_2-CH_2CH_2CH_3$ |
| $OCH_3$ | $OCH_3$ | S | $C(CH_3)_3$ | $-O-SO_2-CH(CH_3)_2$ |
| $OCH_3$ | $OCH_3$ | S | $C(CH_3)_3$ | $-O-SO_2-CH_2CH_2CH_2CH_3$ |
| $OCH_3$ | $OCH_3$ | S | $C(CH_3)_3$ | $-O-SO_2-C(CH_3)_3$ |
| $OCH_3$ | $OCH_3$ | S | $C(CH_3)_3$ | $-O-SO_2-CF_3$ |
| $OCH_3$ | $OCH_3$ | S | $C(CH_3)_3$ | $-O-SO_2-CH=CH_2$ |
| $OCH_3$ | $OCH_3$ | S | $C(CH_3)_3$ | 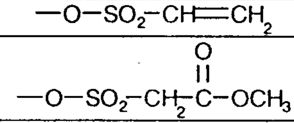 |
| $OCH_3$ | $OCH_3$ | S | $C(CH_3)_3$ | 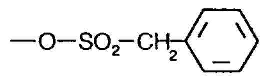 |
| $OCH_3$ | $OCH_3$ | S | $C(CH_3)_3$ | 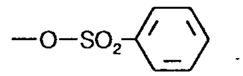 |
| $OCH_3$ | $OCH_3$ | S | $C(CH_3)_3$ | 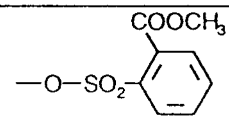 |
| $OCH_3$ | $OCH_3$ | S | $C(CH_3)_3$ | 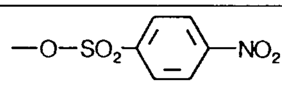 |
| $OCH_3$ | $OCH_3$ | S | $C(CH_3)_3$ | 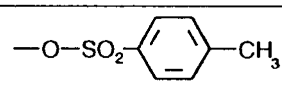 |
| $OCH_3$ | $OCH_3$ | S | $C(CH_3)_3$ | 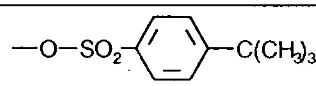 |
| $OCH_3$ | $OCH_3$ | S | $C(CH_3)_3$ | 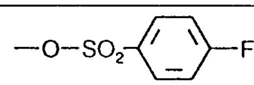 |
| $OCH_3$ | $OCH_3$ | S | $C(CH_3)_3$ | 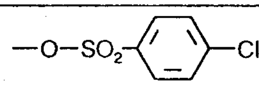 |
| $OCH_3$ | $OCH_3$ | S | $C(CH_3)_3$ | 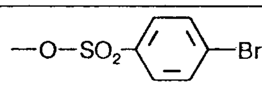 |
| $OCH_3$ | $OCH_3$ | S | $C(CH_3)_3$ | 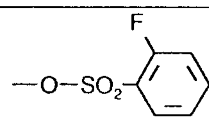 |
| $OCH_3$ | $OCH_3$ | S | $C(CH_3)_3$ | 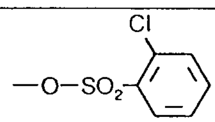 |
| $OCH_3$ | $OCH_3$ | S | $C(CH_3)_3$ | 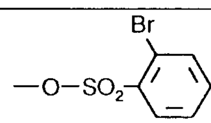 |

Table 1 (continued)

| R1 | R2 | X | R3 | R4 |
|---|---|---|---|---|
| $OCH_3$ | $OCH_3$ | S | $C(CH_3)_3$ | 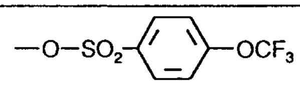 |
| $OCH_3$ | $OCH_3$ | S | $C(CH_3)_3$ | 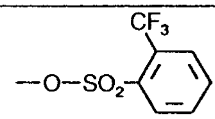 |
| $OCH_3$ | $OCH_3$ | S | $C(CH_3)_3$ | 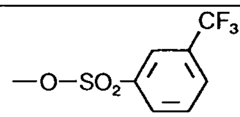 |
| $OCH_3$ | $OCH_3$ | S | $C(CH_3)_3$ | 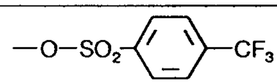 |
| $OCH_3$ | $OCH_3$ | S | $C(CH_3)_3$ | 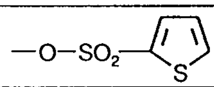 |
| $OCH_3$ | $OCH_3$ | S | $C(CH_3)_3$ | 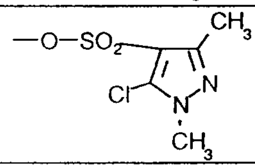 |
| $OCH_3$ | $OCH_3$ | S | $C(CH_3)_3$ | 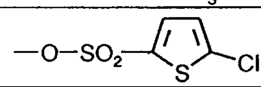 |
| $OCH_3$ | $OCH_3$ | S | $C(CH_3)_3$ | 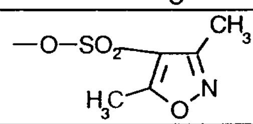 |
| $OCH_3$ | $OCH_3$ | S | $C(CH_3)_3$ | 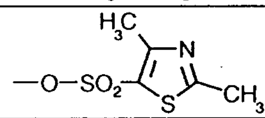 |
| $OCH_3$ | $OCH_3$ | S | $C(CH_3)_3$ | 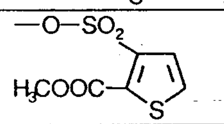 |
| $OCH_3$ | $OCH_3$ | O | $CH(CH_3)_2$ | $-NHCH_3$ |
| $OCH_3$ | $OCH_3$ | O | $CH(CH_3)_2$ | $-NHC_2H_5$ |
| $OCH_3$ | $OCH_3$ | O | $CH(CH_3)_2$ | $-NHCH_2CH_2CH_3$ |
| $OCH_3$ | $OCH_3$ | O | $CH(CH_3)_2$ | $-NHCH(CH_3)_2$ |
| $OCH_3$ | $OCH_3$ | O | $CH(CH_3)_2$ | $-NHCH_2CH_2CH_2CH_3$ |
| $OCH_3$ | $OCH_3$ | O | $CH(CH_3)_2$ | $-NHC(CH_3)_3$ |
| $OCH_3$ | $OCH_3$ | O | $CH(CH_3)_2$ | 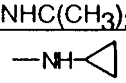 |
| $OCH_3$ | $OCH_3$ | O | $CH(CH_3)_2$ | 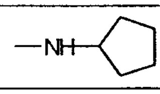 |
| $OCH_3$ | $OCH_3$ | O | $CH(CH_3)_2$ | $-NH-CH_2CH=CH_2$ |
| $OCH_3$ | $OCH_3$ | O | $CH(CH_3)_2$ | $-NH-CH_2CH_2OCH_3$ |

Table 1 (continued)

| R1 | R2 | X | R3 | R4 |
|---|---|---|---|---|
| $OCH_3$ | $OCH_3$ | O | $CH(CH_3)_2$ | $-N(CH_3)_2$ |
| $OCH_3$ | $OCH_3$ | O | $CH(CH_3)_2$ | $-N(CH_3)C_2H_5$ |
| $OCH_3$ | $OCH_3$ | O | $CH(CH_3)_2$ | $-N(C_2H_5)_2$ |
| $OCH_3$ | $OCH_3$ | O | $CH(CH_3)_2$ | $-N(CH_3)CH_2CH_2CH_3$ |
| $OCH_3$ | $OCH_3$ | O | $CH(CH_3)_2$ | $-N(CH_3)CH(CH_3)_2$ |
| $OCH_3$ | $OCH_3$ | O | $CH(CH_3)_2$ | $-N(CH_3)CH_2CH_2CH_2CH_3$ |
| $OCH_3$ | $OCH_3$ | O | $CH(CH_3)_2$ | 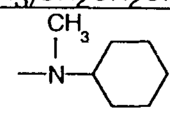 |
| $OCH_3$ | $OCH_3$ | O | $CH(CH_3)_2$ | $-N(CH_3)CH_2CH=CH_2$ |
| $OCH_3$ | $OCH_3$ | O | $CH(CH_3)_2$ | $-N(CH_3)CH_2CH_2OCH_3$ |
| $OCH_3$ | $OCH_3$ | O | $CH(CH_3)_2$ | 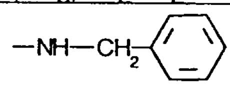 |
| $OCH_3$ | $OCH_3$ | O | $CH(CH_3)_2$ | 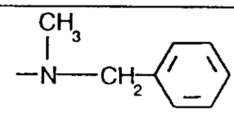 |
| $OCH_3$ | $OCH_3$ | O | $CH(CH_3)_2$ | 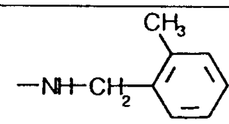 |
| $OCH_3$ | $OCH_3$ | O | $CH(CH_3)_2$ | 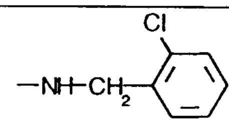 |
| $OCH_3$ | $OCH_3$ | O | $CH(CH_3)_2$ | 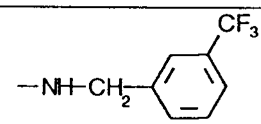 |
| $OCH_3$ | $OCH_3$ | O | $CH(CH_3)_2$ | 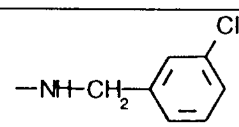 |
| $OCH_3$ | $OCH_3$ | O | $CH(CH_3)_2$ | 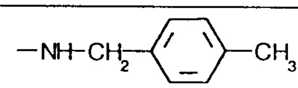 |
| $OCH_3$ | $OCH_3$ | O | $CH(CH_3)_2$ | 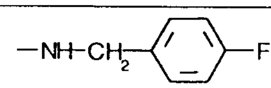 |
| $OCH_3$ | $OCH_3$ | O | $CH(CH_3)_2$ | 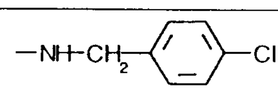 |
| $OCH_3$ | $OCH_3$ | O | $CH(CH_3)_2$ | 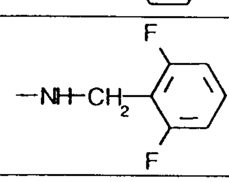 |

Table 1 (continued)

| R1 | R2 | X | R3 | R4 |
|---|---|---|---|---|
| $OCH_3$ | $OCH_3$ | O | $CH(CH_3)_2$ | 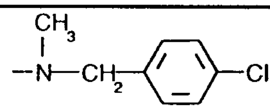 |
| $OCH_3$ | $OCH_3$ | O | $CH(CH_3)_2$ | 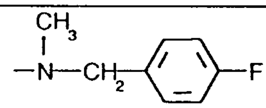 |
| $OCH_3$ | $OCH_3$ | O | $CH(CH_3)_2$ | 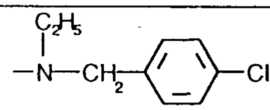 |
| $OCH_3$ | $OCH_3$ | O | $CH(CH_3)_2$ | 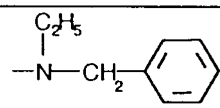 |
| $OCH_3$ | $OCH_3$ | O | $CH(CH_3)_2$ | 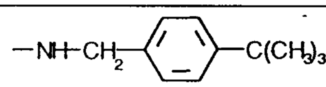 |
| $OCH_3$ | $OCH_3$ | O | $C(CH_3)_3$ | $-NHCH_3$ |
| $OCH_3$ | $OCH_3$ | O | $C(CH_3)_3$ | $-NHC_2H_5$ |
| $OCH_3$ | $OCH_3$ | O | $C(CH_3)_3$ | $-NHCH_2CH_2CH_3$ |
| $OCH_3$ | $OCH_3$ | O | $C(CH_3)_3$ | $-NHCH(CH_3)_2$ |
| $OCH_3$ | $OCH_3$ | O | $C(CH_3)_3$ | $-NHCH_2CH_2CH_2CH_3$ |
| $OCH_3$ | $OCH_3$ | O | $C(CH_3)_3$ | $-NHCH_2CH(CH_3)_2$ |
| $OCH_3$ | $OCH_3$ | O | $C(CH_3)_3$ | $-NHCH(CH_3)CH_2CH_3$ |
| $OCH_3$ | $OCH_3$ | O | $C(CH_3)_3$ | $-NHC(CH_3)_3$ |
| $OCH_3$ | $OCH_3$ | O | $C(CH_3)_3$ | $-NH(CH_2)_4CH_3$ |
| $OCH_3$ | $OCH_3$ | O | $C(CH_3)_3$ | 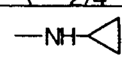 |
| $OCH_3$ | $OCH_3$ | O | $C(CH_3)_3$ | 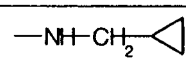 |
| $OCH_3$ | $OCH_3$ | O | $C(CH_3)_3$ | 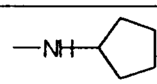 |
| $OCH_3$ | $OCH_3$ | O | $C(CH_3)_3$ | 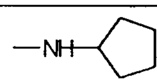 |
| $OCH_3$ | $OCH_3$ | O | $C(CH_3)_3$ | 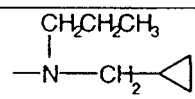 |
| $OCH_3$ | $OCH_3$ | O | $C(CH_3)_3$ | 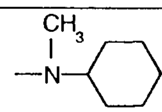 |
| $OCH_3$ | $OCH_3$ | O | $C(CH_3)_3$ | 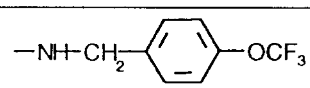 |
| $OCH_3$ | $OCH_3$ | O | $C(CH_3)_3$ | 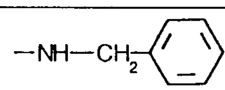 |

Table 1 (continued)

| R1 | R2 | X | R3 | R4 |
|---|---|---|---|---|
| $OCH_3$ | $OCH_3$ | O | $C(CH_3)_3$ | 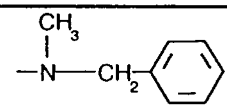 |
| $OCH_3$ | $OCH_3$ | O | $C(CH_3)_3$ | 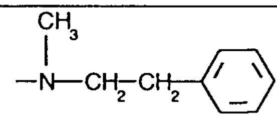 |
| $OCH_3$ | $OCH_3$ | O | $C(CH_3)_3$ | 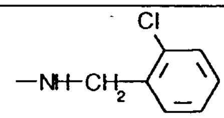 |
| $OCH_3$ | $OCH_3$ | O | $C(CH_3)_3$ | 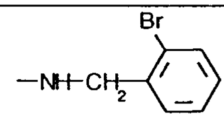 |
| $OCH_3$ | $OCH_3$ | O | $C(CH_3)_3$ | 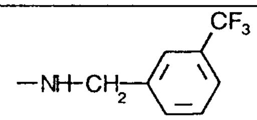 |
| $OCH_3$ | $OCH_3$ | O | $C(CH_3)_3$ | 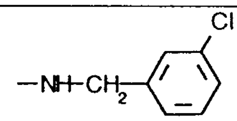 |
| $OCH_3$ | $OCH_3$ | O | $C(CH_3)_3$ | 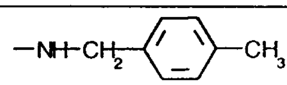 |
| $OCH_3$ | $OCH_3$ | O | $C(CH_3)_3$ | 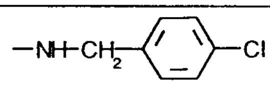 |
| $OCH_3$ | $OCH_3$ | S | $CH(CH_3)_2$ | $-NHCH_2CH_2CH_2CH_3$ |
| $OCH_3$ | $OCH_3$ | O | $C(CH_3)_3$ | 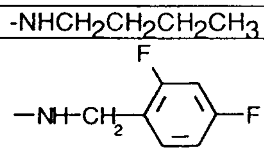 |
| $OCH_3$ | $OCH_3$ | O | $C(CH_3)_3$ | 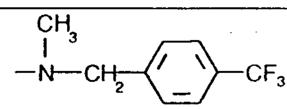 |
| $OCH_3$ | $OCH_3$ | O | $C(CH_3)_3$ | 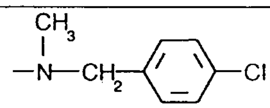 |
| $OCH_3$ | $OCH_3$ | O | $C(CH_3)_3$ | 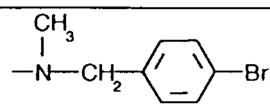 |
| $OCH_3$ | $OCH_3$ | S | $CH(CH_3)_2$ | $-NHCH_3$ |
| $OCH_3$ | $OCH_3$ | S | $CH(CH_3)_2$ | $-NHC_2H_5$ |
| $OCH_3$ | $OCH_3$ | S | $CH(CH_3)_2$ | $-NHCH_2CH_2CH_3$ |
| $OCH_3$ | $OCH_3$ | S | $CH(CH_3)_2$ | $-NHCH(CH_3)_2$ |

Table 1 (continued)

| R1 | R2 | X | R3 | R4 |
|---|---|---|---|---|
| $OCH_3$ | $OCH_3$ | S | $CH(CH_3)_2$ | 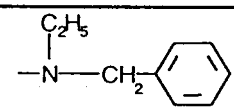 |
| $OCH_3$ | $OCH_3$ | S | $CH(CH_3)_2$ | $-NHCH_2CH(CH_3)_2$ |
| $OCH_3$ | $OCH_3$ | S | $CH(CH_3)_2$ | $-NHCH(CH_3)CH_2CH_3$ |
| $OCH_3$ | $OCH_3$ | S | $CH(CH_3)_2$ | $-NHC(CH_3)_3$ |
| $OCH_3$ | $OCH_3$ | S | $CH(CH_3)_2$ | $-NH(CH_2)_4CH_3$ |
| $OCH_3$ | $OCH_3$ | S | $CH(CH_3)_2$ | 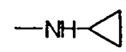 |
| $OCH_3$ | $OCH_3$ | S | $CH(CH_3)_2$ | 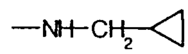 |
| $OCH_3$ | $OCH_3$ | S | $CH(CH_3)_2$ | 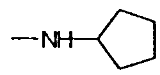 |
| $OCH_3$ | $OCH_3$ | S | $CH(CH_3)_2$ | 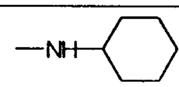 |
| $OCH_3$ | $OCH_3$ | S | $CH(CH_3)_2$ | 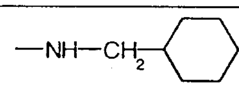 |
| $OCH_3$ | $OCH_3$ | S | $CH(CH_3)_2$ | $-NH-CH_2CH=CH_2$ |
| $OCH_3$ | $OCH_3$ | S | $CH(CH_3)_2$ | $-NH-CH_2CH_2OCH_3$ |
| $OCH_3$ | $OCH_3$ | S | $CH(CH_3)_2$ | $-N(CH_3)_2$ |
| $OCH_3$ | $OCH_3$ | S | $CH(CH_3)_2$ | $-N(CH_3)C_2H_5$ |
| $OCH_3$ | $OCH_3$ | S | $CH(CH_3)_2$ | $-N(C_2H_5)_2$ |
| $OCH_3$ | $OCH_3$ | S | $CH(CH_3)_2$ | $-N(CH_3)CH_2CH_2CH_3$ |
| $OCH_3$ | $OCH_3$ | S | $CH(CH_3)_2$ | $-N(CH_3)CH(CH_3)_2$ |
| $OCH_3$ | $OCH_3$ | S | $CH(CH_3)_2$ | $-N(CH_3)CH_2CH_2CH_2CH_3$ |
| $OCH_3$ | $OCH_3$ | S | $CH(CH_3)_2$ | 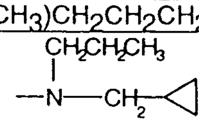 |
| $OCH_3$ | $OCH_3$ | S | $CH(CH_3)_2$ | 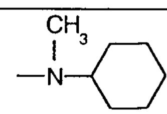 |
| $OCH_3$ | $OCH_3$ | S | $CH(CH_3)_2$ | $-N(CH_3)CH_2CH=CH_2$ |
| $OCH_3$ | $OCH_3$ | S | $CH(CH_3)_2$ | $-N(CH_3)CH_2CH_2OCH_3$ |
| $OCH_3$ | $OCH_3$ | S | $CH(CH_3)_2$ | 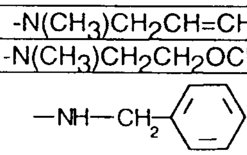 |
| $OCH_3$ | $OCH_3$ | S | $CH(CH_3)_2$ | 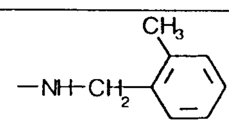 |
| $OCH_3$ | $OCH_3$ | S | $CH(CH_3)_2$ | 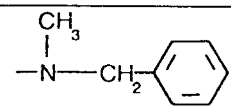 |

Table 1 (continued)

| R1 | R2 | X | R3 | R4 |
|---|---|---|---|---|
| $OCH_3$ | $OCH_3$ | S | $C(CH_3)_3$ | 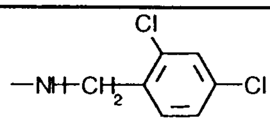 |
| $OCH_3$ | $OCH_3$ | S | $C(CH_3)_3$ | 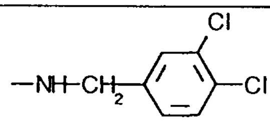 |
| $OCH_3$ | $OCH_3$ | S | $CH(CH_3)_2$ | 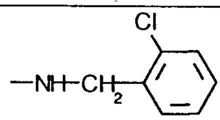 |
| $OCH_3$ | $OCH_3$ | S | $CH(CH_3)_2$ | 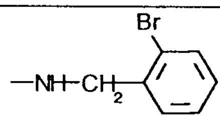 |
| $OCH_3$ | $OCH_3$ | S | $CH(CH_3)_2$ | 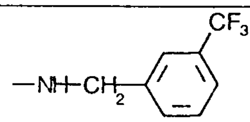 |
| $OCH_3$ | $OCH_3$ | S | $CH(CH_3)_2$ | 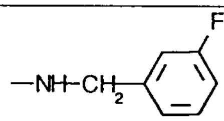 |
| $OCH_3$ | $OCH_3$ | S | $CH(CH_3)_2$ | 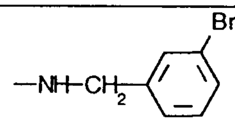 |
| $OCH_3$ | $OCH_3$ | S | $CH(CH_3)_2$ | 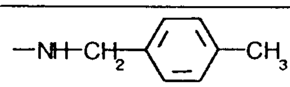 |
| $OCH_3$ | $OCH_3$ | S | $CH(CH_3)_2$ | 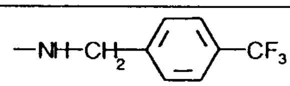 |
| $OCH_3$ | $OCH_3$ | S | $CH(CH_3)_2$ | 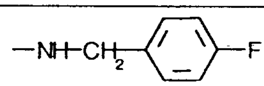 |
| $OCH_3$ | $OCH_3$ | S | $CH(CH_3)_2$ | 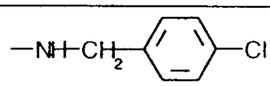 |
| $OCH_3$ | $OCH_3$ | S | $CH(CH_3)_2$ | 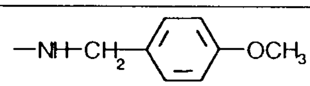 |
| $OCH_3$ | $OCH_3$ | S | $CH(CH_3)_2$ | 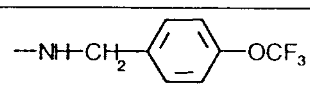 |
| $OCH_3$ | $OCH_3$ | S | $CH(CH_3)_2$ | 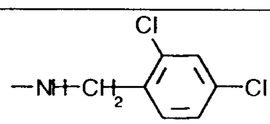 |

Table 1 (continued)

| R1 | R2 | X | R3 | R4 |
|---|---|---|---|---|
| $OCH_3$ | $OCH_3$ | S | $CH(CH_3)_2$ | 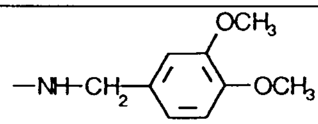 |
| $OCH_3$ | $OCH_3$ | S | $CH(CH_3)_2$ | 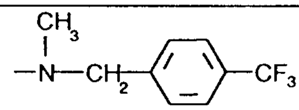 |
| $OCH_3$ | $OCH_3$ | S | $CH(CH_3)_2$ | 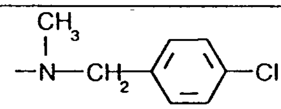 |
| $OCH_3$ | $OCH_3$ | S | $CH(CH_3)_2$ | 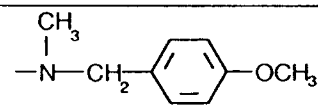 |
| $OCH_3$ | $OCH_3$ | S | $C(CH_3)_3$ | $-NHCH_3$ |
| $OCH_3$ | $OCH_3$ | S | $CH(CH_3)_2$ | 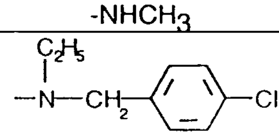 |
| $OCH_3$ | $OCH_3$ | S | $CH(CH_3)_2$ | 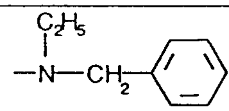 |
| $OCH_3$ | $OCH_3$ | S | $CH(CH_3)_2$ | 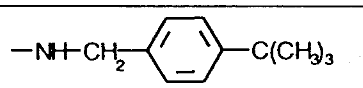 |
| $OCH_3$ | $OCH_3$ | S | $C(CH_3)_3$ | $-NHC_2H_5$ |
| $OCH_3$ | $OCH_3$ | S | $C(CH_3)_3$ | $-NHCH_2CH_2CH_3$ |
| $OCH_3$ | $OCH_3$ | S | $C(CH_3)_3$ | $-NHCH(CH_3)_2$ |
| $OCH_3$ | $OCH_3$ | S | $C(CH_3)_3$ | $-NHCH_2CH_2CH_2CH_3$ |
| $OCH_3$ | $OCH_3$ | S | $C(CH_3)_3$ | $-NHCH_2CH(CH_3)_2$ |
| $OCH_3$ | $OCH_3$ | S | $C(CH_3)_3$ | $-NHCH(CH_3)CH_2CH_3$ |
| $OCH_3$ | $OCH_3$ | S | $C(CH_3)_3$ | $-NHC(CH_3)_3$ |
| $OCH_3$ | $OCH_3$ | S | $C(CH_3)_3$ | $-NH(CH_2)_4CH_3$ |
| $OCH_3$ | $OCH_3$ | S | $C(CH_3)_3$ | 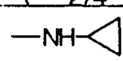 |
| $OCH_3$ | $OCH_3$ | S | $C(CH_3)_3$ | 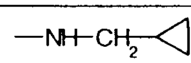 |
| $OCH_3$ | $OCH_3$ | S | $C(CH_3)_3$ | 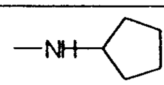 |
| $OCH_3$ | $OCH_3$ | S | $C(CH_3)_3$ | 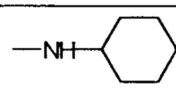 |
| $OCH_3$ | $OCH_3$ | S | $C(CH_3)_3$ | 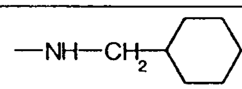 |
| $OCH_3$ | $OCH_3$ | S | $C(CH_3)_3$ | $-NH-CH_2CH=CH_2$ |

Table 1 (continued)

| R1 | R2 | X | R3 | R4 |
|---|---|---|---|---|
| $OCH_3$ | $OCH_3$ | S | $C(CH_3)_3$ | $-NH-CH_2CH_2OCH_3$ |
| $OCH_3$ | $OCH_3$ | S | $C(CH_3)_3$ | $-N(CH_3)_2$ |
| $OCH_3$ | $OCH_3$ | S | $C(CH_3)_3$ | $-N(CH_3)C_2H_5$ |
| $OCH_3$ | $OCH_3$ | S | $C(CH_3)_3$ | $-N(C_2H_5)_2$ |
| $OCH_3$ | $OCH_3$ | S | $C(CH_3)_3$ | $-N(CH_3)CH_2CH_2CH_3$ |
| $OCH_3$ | $OCH_3$ | S | $C(CH_3)_3$ | $-N(CH_3)CH_2CH_2CH_2CH_3$ |
| $OCH_3$ | $OCH_3$ | S | $C(CH_3)_3$ | 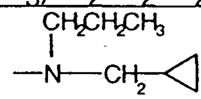 |
| $OCH_3$ | $OCH_3$ | S | $C(CH_3)_3$ | 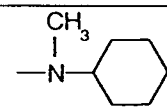 |
| $OCH_3$ | $OCH_3$ | S | $C(CH_3)_3$ | $-N(CH_3)CH_2CH=CH_2$ |
| $OCH_3$ | $OCH_3$ | S | $C(CH_3)_3$ | 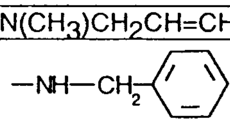 |
| $OCH_3$ | $OCH_3$ | S | $C(CH_3)_3$ | 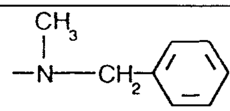 |
| $OCH_3$ | $OCH_3$ | S | $C(CH_3)_3$ | 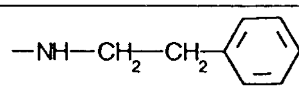 |
| $OCH_3$ | $OCH_3$ | S | $C(CH_3)_3$ | 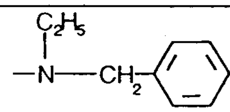 |
| $OCH_3$ | $OCH_3$ | S | $C(CH_3)_3$ | 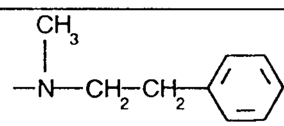 |
| $OCH_3$ | $OCH_3$ | S | $C(CH_3)_3$ | 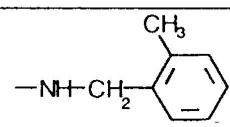 |
| $OCH_3$ | $OCH_3$ | S | $C(CH_3)_3$ | 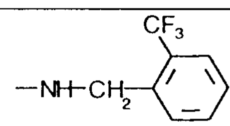 |
| $OCH_3$ | $OCH_3$ | S | $C(CH_3)_3$ | 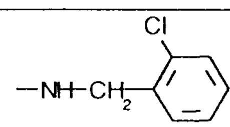 |
| $OCH_3$ | $OCH_3$ | S | $C(CH_3)_3$ | 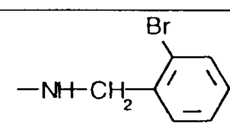 |

Table 1 (continued)

| R1 | R2 | X | R3 | R4 |
|---|---|---|---|---|
| $OCH_3$ | $OCH_3$ | S | $C(CH_3)_3$ | 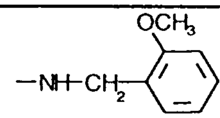 |
| $OCH_3$ | $OCH_3$ | S | $C(CH_3)_3$ | 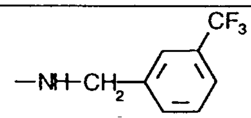 |
| $OCH_3$ | $OCH_3$ | S | $C(CH_3)_3$ | 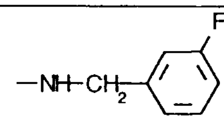 |
| $OCH_3$ | $OCH_3$ | S | $C(CH_3)_3$ | 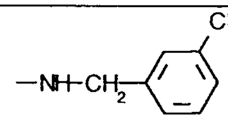 |
| $OCH_3$ | $OCH_3$ | S | $C(CH_3)_3$ | 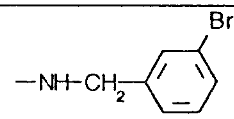 |
| $OCH_3$ | $OCH_3$ | S | $C(CH_3)_3$ | 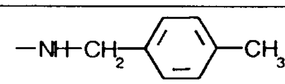 |
| $OCH_3$ | $OCH_3$ | S | $C(CH_3)_3$ | 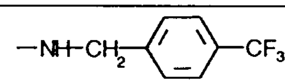 |
| $OCH_3$ | $OCH_3$ | S | $C(CH_3)_3$ | 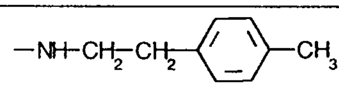 |
| $OCH_3$ | $OCH_3$ | S | $C(CH_3)_3$ | 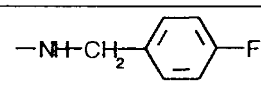 |
| $OCH_3$ | $OCH_3$ | S | $C(CH_3)_3$ | 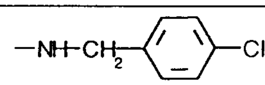 |
| $OCH_3$ | $OCH_3$ | S | $C(CH_3)_3$ | 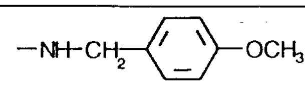 |
| $OCH_3$ | $OCH_3$ | S | $C(CH_3)_3$ | 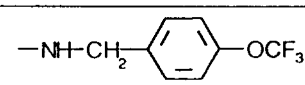 |
| $OCH_3$ | $OCH_3$ | S | $C(CH_3)_3$ | 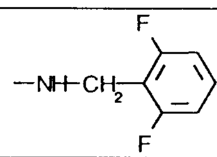 |
| $OCH_3$ | $OCH_3$ | S | $C(CH_3)_3$ | 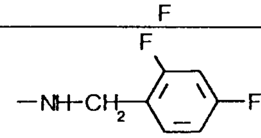 |

Table 1 (continued)

| R1 | R2 | X | R3 | R4 |
|---|---|---|---|---|
| $OCH_3$ | $OCH_3$ | S | $C(CH_3)_3$ | 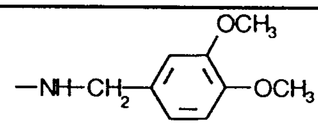 |
| $OCH_3$ | $OCH_3$ | S | $C(CH_3)_3$ | 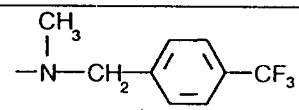 |
| $OCH_3$ | $OCH_3$ | S | $C(CH_3)_3$ | 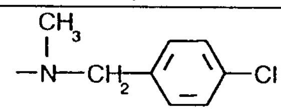 |
| $OCH_3$ | $OCH_3$ | S | $C(CH_3)_3$ | 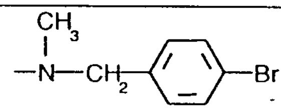 |
| $OCH_3$ | $OCH_3$ | S | $C(CH_3)_3$ | 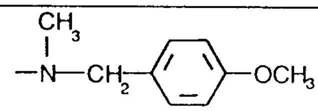 |
| $OCH_3$ | $OCH_3$ | S | $C(CH_3)_3$ | 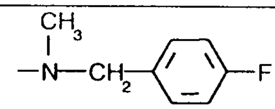 |
| $OCH_3$ | $OCH_3$ | S | $C(CH_3)_3$ | 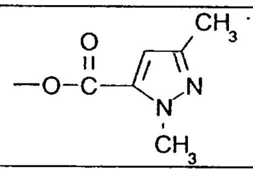 |
| $OCH_3$ | $OCH_3$ | S | $C(CH_3)_3$ | 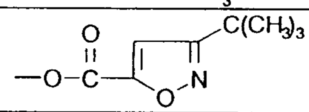 |
| $OCH_3$ | $OCH_3$ | S | $C(CH_3)_3$ | 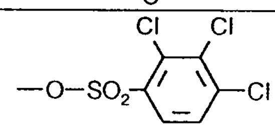 |
| $OCH_3$ | $OCH_3$ | S | $C(CH_3)_3$ | 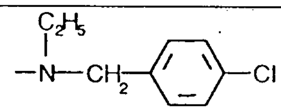 |
| $OCH_3$ | $OCH_3$ | S | $C(CH_3)_3$ | 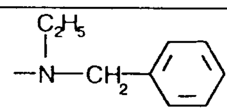 |
| $OCH_3$ | $OCH_3$ | S | $C(CH_3)_3$ | 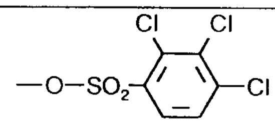 |
| $OCH_3$ | $OCH_3$ | S | $C(CH_3)_3$ | 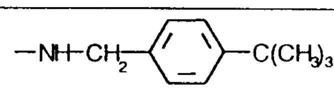 |

Table 1 (continued)

| R1 | R2 | X | R3 | R4 |
|---|---|---|---|---|
| $OCH_3$ | $OCH_3$ | S | $C(CH_3)_3$ | 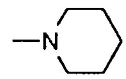 |
| $OCH_3$ | $OCH_3$ | S | $C(CH_3)_3$ | 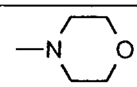 |
| $OCH_3$ | $OCH_3$ | S | $C(CH_3)_3$ | 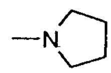 |
| $OCH_3$ | $OCH_3$ | O | $C(CH_3)_3$ | 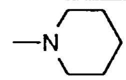 |
| $OCH_3$ | $OCH_3$ | O | $CH(CH_3)_2$ | 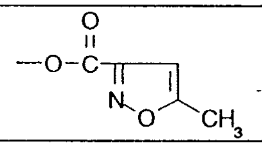 |
| $OCH_3$ | $OCH_3$ | O | $CH(CH_3)_2$ | 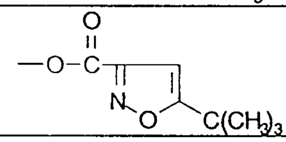 |
| $OCH_3$ | $OCH_3$ | O | $C(CH_3)_3$ | 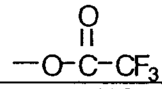 |
| $OCH_3$ | $OCH_3$ | O | $C(CH_3)_3$ | 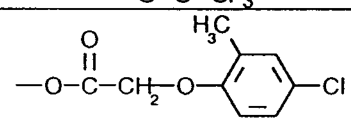 |
| $OCH_3$ | $OCH_3$ | O | $C(CH_3)_3$ | 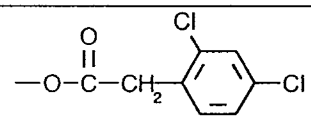 |
| $OCH_3$ | $OCH_3$ | O | $C(CH_3)_3$ | 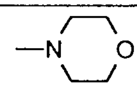 |
| $OCH_3$ | $OCH_3$ | O | $C(CH_3)_3$ | 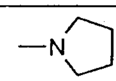 |
| $OCH_3$ | $OCH_3$ | O | $C(CH_3)_3$ | 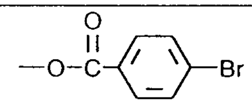 |
| $OCH_3$ | $OCH_3$ | O | $C(CH_3)_3$ | 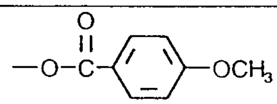 |
| $OCH_3$ | $OCH_3$ | S | $C(CH_3)_3$ | 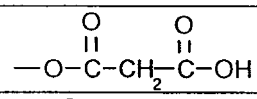 |
| $OCH_3$ | $OCH_3$ | S | $C(CH_3)_3$ | 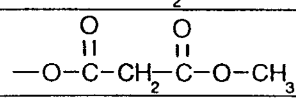 |

Table 1 (continued)

| R1 | R2 | X | R3 | R4 |
|---|---|---|---|---|
| $OCH_3$ | $OCH_3$ | O | $C(CH_3)_3$ | 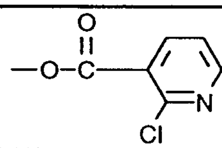 |
| $OCH_3$ | $OCH_3$ | O | $C(CH_3)_3$ | 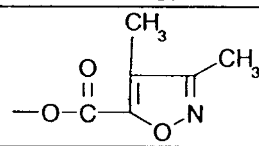 |
| $OCH_3$ | $OCH_3$ | S | $CH(CH_3)_2$ | 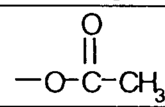 |
| $OCH_3$ | $OCH_3$ | S | $CH(CH_3)_2$ | 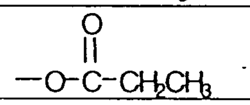 |
| $OCH_3$ | $OCH_3$ | S | $CH(CH_3)_2$ | 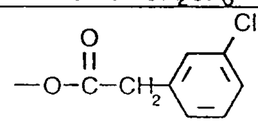 |
| $OCH_3$ | $OCH_3$ | S | $CH(CH_3)_2$ | 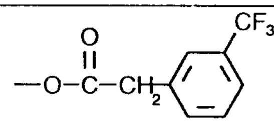 |
| $OCH_3$ | $OCH_3$ | S | $CH(CH_3)_2$ | 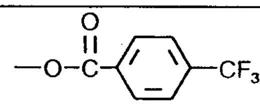 |
| $OCH_3$ | $OCH_3$ | S | $CH(CH_3)_2$ | 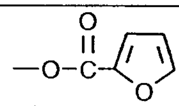 |
| $OCH_3$ | $OCH_3$ | S | $CH(CH_3)_2$ | 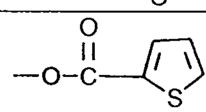 |
| $OCH_3$ | $OCH_3$ | S | $CH(CH_3)_2$ | 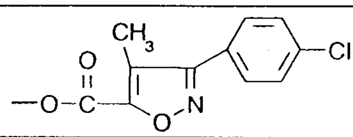 |
| $OCH_3$ | $OCH_3$ | S | $C(CH_3)_3$ | 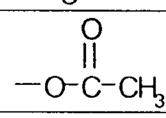 |
| $OCH_3$ | $OCH_3$ | S | $C(CH_3)_3$ | 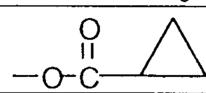 |
| $OCH_3$ | $OCH_3$ | S | $C(CH_3)_3$ | 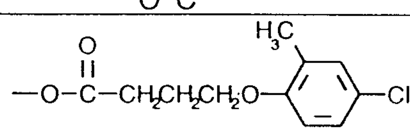 |

Table 1 (continued)

| R1 | R2 | X | R3 | R4 |
|---|---|---|---|---|
| $OCH_3$ | $OCH_3$ | S | $C(CH_3)_3$ | 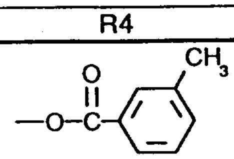 |
| $OCH_3$ | $OCH_3$ | S | $C(CH_3)_3$ | 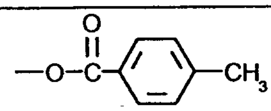 |
| $OCH_3$ | $OCH_3$ | S | $C(CH_3)_3$ | 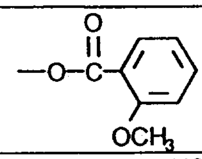 |
| $OCH_3$ | $OCH_3$ | S | $C(CH_3)_3$ | 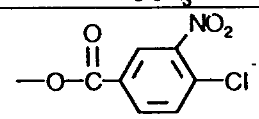 |
| $OCH_3$ | $OCH_3$ | S | $C(CH_3)_3$ | 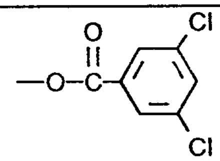 |

When in the process (a), if, for example 2-(4,6-dimethoxy-2-pyrimidinylthio)-3,3-dimethylbutanal, sodium cyanide and ammonium chloride are used as starting materials, the course of the reaction can be represented by the following equation:

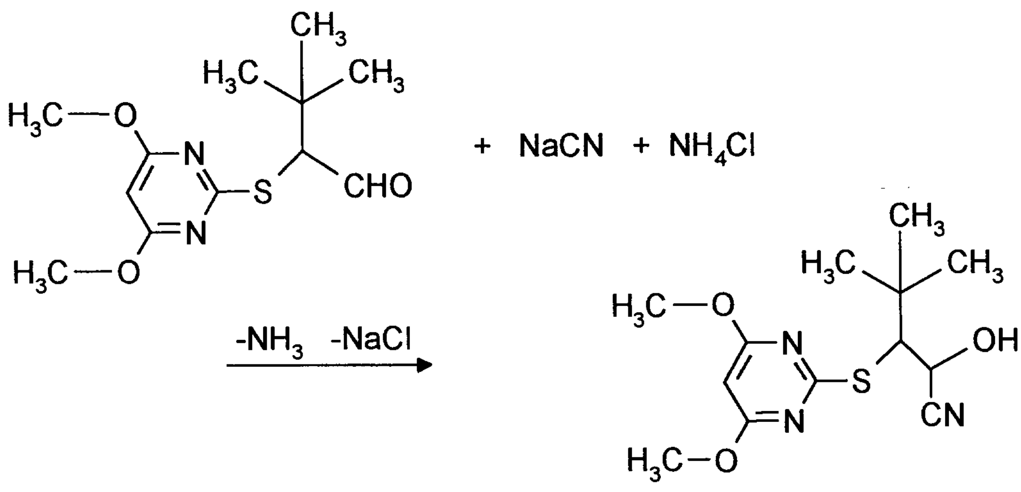


When in the process (b), if, for example, 1-cyano-2-(4,6-dimethoxy-2-pyrimidinylthio)-3,3'-dimethyl-1-butanol and Jones oxidizing agent are used as starting materials, the course of the reaction can be represented by the following equation:

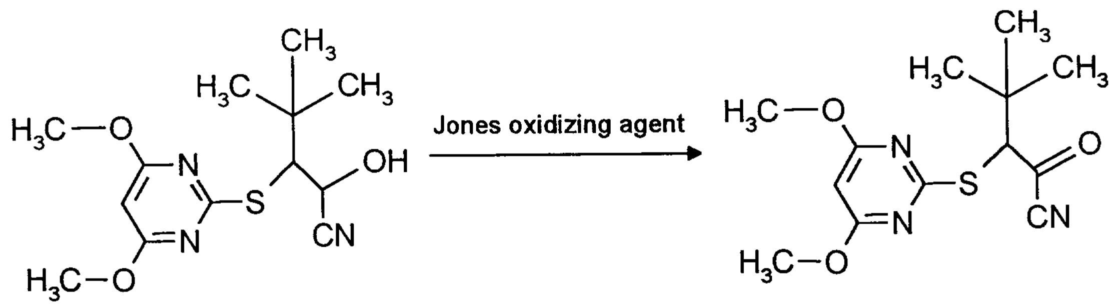


When in the process (c), if, for example, 1-cyano-2-(4,6-dimethoxy-2-pyrimidinylthio)-3,3-dimethyl-1-butanol and ammonia, are used as starting materials, the course of the reaction can be represented by the following equation:

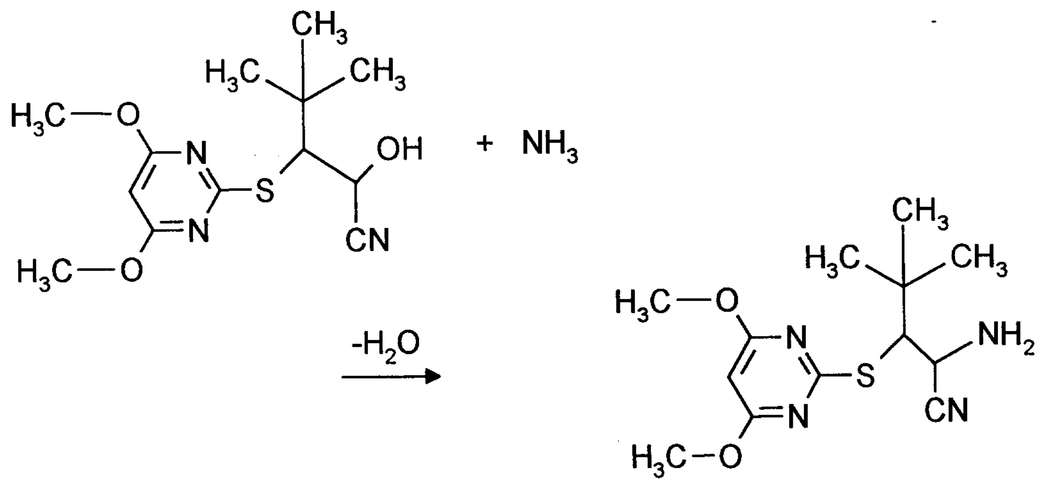


When in the process (d), if, for example, 1-cyano-2-(4,6-dimethoxy-2-pyrimidinylthio)-3,3-dimethyl-1-butanol and methanesulfonyl chloride, are used as starting materials, the course of the reaction can be represented by the following equation:

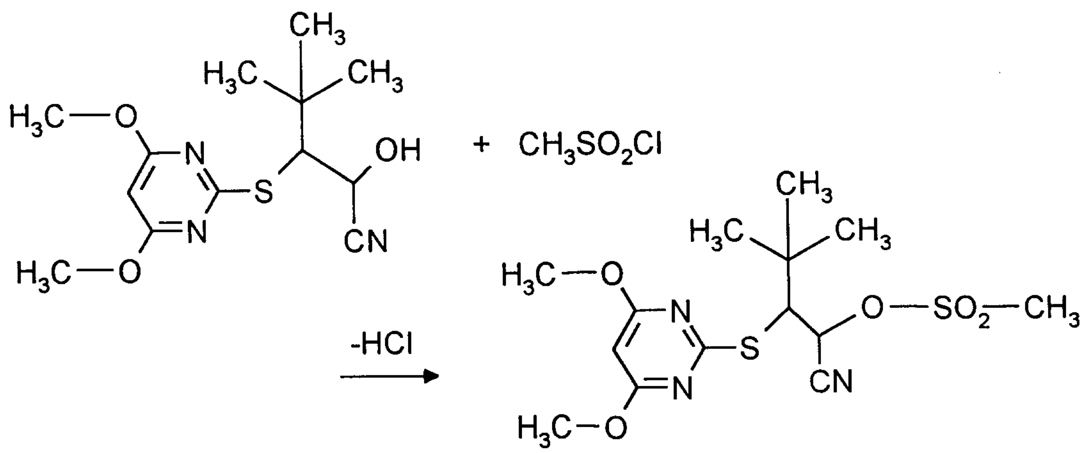


When in the process (e), if, for example, 1-cyano-2-(4,6-dimethoxy-2-pyrimidinylthio)-3,3-dimethyl-1-butanol and acetic anhydride, are used as starting materials, the course of the reaction can be represented by the following equation:

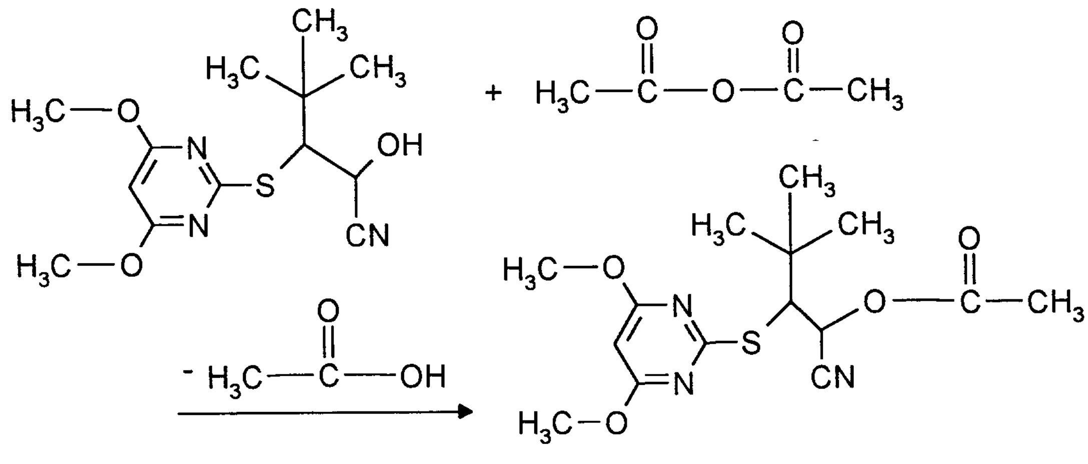


In the process (a), the starting compounds of the formula (II) mean compounds based on the above definitions of $R^1$, $R^2$ and $R^3$, preferably substituents based on the above preferred definitions of $R^1$, $R^2$ and $R^3$.

The compounds of the formula (II) can be obtained in the same way as described in Japanese Patent Application Nos. 118540/1992 and 47110/1993.

As specific examples of the formula (II), there may be mentioned:
2-(4,6-dimethoxy-2-pyrimidinylthio)-3-methylbutanal,
2-(4,6-dimethoxy-2-pyrimidinylthio)-3,3-dimethylbutanal,
2-(4,6-dimethoxy-2-pyrimidinylthio)-3-methylbutanal, and
2-(4,6-dimethoxy-2-pyrimidinyloxy)-3,3-dimethylbutanal.

In the process (b), (c) and (e), the starting compounds of the formula (Ia) mean compounds based on the above definitions of $R^1$, $R^2$ and $R^3$, preferably substituents based on the above preferred definitions of $R^1$, $R^2$ and $R^3$.

The compounds of the formula (Ia) can be obtained in the above mentioned process (a).

As specific examples of the formula (Ia), there may be mentioned:
1-cyano-2-(4,6-dimethoxy-2-pyrimidinylthio)-3-methyl-1-butanol,
1-cyano-2-(4,6-dimethoxy-2-pyrimidinylthio)-3,3-dimethyl-1-butanol,
1-cyano-2-(4,6-dimethoxy-2-pyrimidinylthio)-3-methyl-1-butanol, and
1-cyano-2-(4,6-dimethoxy-2-pyrimidinyloxy)-3,3-dimethyl-1-butanol.

In the process (c), the starting compounds of the formula (III) mean compounds based on the above definitions of $R^5$ and $R^{6}$', preferably substituents based on the above preferred definitions of $R^5$ and $R^6$.

The compounds of the formula (III) are well known in the field of organic chemistry. As specific examples of the formula (III), there may be mentioned: Ammonia, methylamine, dimethylamine, ethylamine, diethylamine, propylamine, benzylamine, pyrrolidine, piperidine and morpholine.

In the process (d), the starting compounds of the formula (IV) mean compounds based on the above definitions of $R^7$, preferably substituents based on the above preferred definitions of $R^7$.

The compounds of the formula (IV) are well known in the field of organic chemistry. As specific examples of the formula (IV), there may be mentioned: Methanesulfonyl chloride, methanesulfonyl bromide, p-toluenesulfonyl chloride, p-chlorophenylsulfonyl chloride, phenylsulfonyl chloride, ethanesulfonyl chloride, propanesulfonyl chloride.

In the process (e), the starting compounds of the formulae (V) and (VI) mean compounds based on the above definitions of $R^8$, preferably substituents based on the above preferred definitions of $R^8$.

The compounds of the formulae (V) and (VI) are well known in the field of organic chemistry. As specific examples of the formulae (V) and (VI), there may be mentioned:
Benzoyl chloride or bromide, cinnamic chloride or bromide, propionic chloride or bromide, phenoxyacetic chloride or bromide, nicotinic chloride or bromide, p-chlorobenzoyl chloride or bromide, chloroacetic chloride or bromide, acetic anhydride, propionic anhydride, acetic formic anhydride.

In carrying out the process (a) mentioned above, use may be made, as suitable diluent, of any inert solvent.

Examples of such diluents are water; aliphatic, cycloaliphatic and aromatic, optionally chlorinated, hydrocarbons such as pentane, hexane, cyclohexane, petroleum ether, ligroin, benzene, toluene, xylene,

dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF), dimethylene glycol dimethyl ether and the like; nitriles such as acetonitrile, propionitrile and the like; alcohols such as methanol, ethanol, isopropanol, butanol, ethylene glycol and the like; esters such as ethyl acetate, amyl acetate and the like, acid amides such as dimethyl formamide (DMF), dimethyl acetamide (DMA), N-methyl-pyrrolidone, 1,3-dimethyl-2-imidazolidinone, hexamethylphosphoric triamide (HMPA) and the like; sulfones and sulfoxides such as dimethyl sulfoxide (DMSO), sulfolane and the like.

The process (a) according to the invention is carried out preferably in the presence of acid.

As examples of such acid may be mentioned: ammonium chloride, sodium hydrogen sulfite and so on.

In the above mentioned process (a), the reaction temperature can be varied within a substantially wide range. In general, the reaction is carried out at a temperature of from about -20 °C about 80 °C, preferably from 5 °C to about 30 °C.

Further, the reaction is carried out under normal pressure, although it is also possible to employ a higher or reduced pressure.

When the above mentioned process (a) according to the present invention is carried out, use is made, for example, about 1.1 to 2.0 moles of sodium cyanide in a diluent such as ether and water, per 1 mol of the compounds represented by the general formula (II) to obtain the desired compounds.

In carrying out the above mentioned process (b), use may be made, as suitable diluent, of any inert solvent.

Examples of such diluents are water; aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxy ethane (DME), tetrahydrofurane (THF), dimethylene glycol dimethyl ether and the like; ketones such as acetone, methylethyl ketone (MEK), methyl-iso-propyl ketone, methyl-iso-butyl ketone (MIBK) and the like; nitriles such as acetonitrile, propionitrile and the like; esters such as ethyl acetate, amyl acetate and the like, acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like.

In the above mentioned process (b), the reaction temperature can be varied within a substantially wide range. In general, the reaction is carried out at a temperature of from about -30 °C about 50 °C, preferably from -5 °C to about 15 °C.

Further, the reaction is carried out under normal pressure, although it is possible to employ a higher or reduced pressure.

When the above mentioned process (b) according to the present invention is carried out, use is made, for example, about 1.0 to 3.0 mols of 8N Jones oxidizing agents in a diluent such as acetone, per 1 mol of the compounds represented by the general formula (Ia) to obtain the desired compounds.

In carrying out the above mentioned process (c), use may be made, as suitable diluent, of any inert solvent.

Examples of such diluents are water; aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxy ethane (DME), tetrahydrofurane (THF), dimethylene glycol dimethyl ether and the like; nitriles such as acetonitrile, propionitrile and the like; alcohols such as methanol, ethanol, isopropanol, butanol, ethylene glycol and the like; esters such as ethyl acetate, amyl acetate and the like, acid amides such as dimethyl formamide (DMF), dimethyl acetamide (DMA), N-methyl-pyrrolidone, 1,3-dimethyl-2-imidazolidinone, hexamethyl phosphoric triamide (HMPA) and the like; sulfones and sulfoxides such as dimethyl sulfoxide (DMSO), sulfolane and the like; and a base such as pyridine.

The process (c) according to the invention is carried out preferably in the presence of ammonium chloride as catalyst.

In the above mentioned process (c), the reaction temperature can be varied within a substantially wide range. In general, the reaction is carried out at a temperature of from about -10 °C about 50 °C, preferably from -5 °C to about 30 °C.

Further, the reaction is carried out under normal pressure, although it is possible to employ a higher or reduced pressure.

When the above mentioned process (c) according to the present invention is carried out, use is made, for example, of an excess of the compound of the formula (III) in a diluent such as methanol, and a catalytic amount of ammonium chloride, per 1 mol of the compounds represented by the formula (Ia) to obtain the desired compounds.

In carrying out the above mentioned process (d), use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzen, dichlorobenzene and the like; ethers such as diethyl ether, methyl ethyl ether, diisopropyl ether, dibutyl ether, dioxane, dimethoxyethane (DME), tetrahydrofurane (THF), dimethylene glycol dimethyl ether and the like; ketones such as acetone, methylethyl ketone (MEK), methyl-iso-propyl ketone, methyl-iso-butyl ketone (MIBK) and the like; nitriles such as acetonitrile, propionitrile and the like; esters such as ethyl acetate, amyl acetate and the like, acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like; and a base such as pyridine.
The process (d) according to the invention is carried out preferably in the presence of acid binder.

As examples of such acid binder may be mentioned: inorganic bases including hydroxide, carbonate, bicarbonate of alkali metals such as, for example, sodium hydrogen carbonate, potassium hydrogen carbonate, sodium carbonate, potassium carbonate, lithium hydroxide, sodium hydroxide, potassium hydroxide, calcium hydroxide and the like; inorganic alkali metal amides including lithium amide, sodium amide, potassium amide, and the like; organic bases including tertiary amines, dialkylaminoanilines, and pyridines such as, for example, triethylamine, 1,1,4,4-tetra-methylenediamine (TMEDA), N,N-dimethylaniline, N,N-diethylaniline, pyridine, 4-dimethylaminopyridine (DMAP), 1,4-diaza-bicyclo[2,2,2]octane (DABCO), 1,8-diazabicyclo[5,4,0]undec-7-ene (DBU) and the like; organic lithiums including methyllithium, n-butyllithium, sec-butyllithium, tert-butyllithium, phenyllithium, dimethyl copper lithium, lithiumdiisopropylamide, lithiumcyclohexylisopropylamide, lithiumdicyclohexylamide, n-butyllithium DABCO, n-butyllithium TMEDA and the like.

In the above mentioned process (d), the reaction temperature can be varied within a substantially wide range. In general, the reaction is carried out at a temperature of from about -10 °C about 80 °C, preferably from 0 °C to about 50 °C.

Further, the reaction is carried out under normal pressure, although it is also possible to employ a higher or reduced pressure.

When the above mentioned process (d) according to the present invention is carried out, use is made, for example, about 1.0 to 2.0 mols of the compound of the formula (IV) in a diluent such as pyridine per 1 mol of the compounds represented by the general formula (Ia) to obtain the desired compound.

In carrying out the process (e) mentioned above, use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF), dimethylene glycol dimethyl ether and the like; ketones such as acetone, methylethyl ketone (MEK), methyl-iso-propyl ketone, methyl-iso-butyl ketone (MIBK) and the like; nitriles such as acetonitrile, propionitrile and the like; esters such as ethyl acetate, amyl acetate and the like, acid amides such as dimethyl formamide (DMF), dimethyl acetamide (DMA), N-methyl-pyrrolidone, 1,3-dimethyl-2-imidazolidinone, hexamethylphosphoric triamide (HMPA) and the like; sulfones and sulfoxides such as dimethyl sulfoxide (DMSO), sulfolane and the like; and bases such as pyridine.

The process (e) according to the invention is carried out preferably in the presence of acid binder.

As examples of such acid binder may be mentioned: inorganic bases including hydroxide, carbonate, bicarbonate of alkali metals such as, for example, sodium hydrogen carbonate, potassium hydrogen carbonate, sodium carbonate, potassium carbonate, lithium hydroxide, sodium hydroxide, potassium hydroxide, calcium hydroxide and the like; inorganic alkali metal amides including lithium amide, sodium amide, potassium amide, and the like; organic bases including tertiary amines, dialkylaminoanilines, and pyridines such as, for example, triethylamine, 1,1,4,4-tetra-methylenediamine (TMEDA), N,N-dimethylaniline, N,N-diethyl-aniline, pyridine, 4-dimethylaminopyridine (DMAP), 1,4-diaza-bicyclo[2,2,2]octane (DABCO), 1,8-diazabicyclo[5,4,0]-undec-7-ene (DBU) and the like; organic lithiums including methyllithium, n-(sec- or tert-)butyllithium, phenyllithium, dimethyl copper lithium, lithiumdiisopropylamide, lithiumcyclohexyl-isopropylamide, lithiumdicyclohexylamide, n-butyllithium DABCO, n-butyllithium TMEDA and the like.

In the above mentioned process (e), the reaction temperature can be varied within a substantially wide range. In general, the reaction is carried out at a temperature of from about -10 ° C about 80 ° C, preferably from 0 ° C to about 50 ° C.

Further, the reaction is carried out under normal pressure, although it is also possible to employ a higher or reduced pressure.

When the above mentioned process (e) according to the present invention is carried out, use is made, for example, about 1.0 to 2.0 mols of the compound of the formula (V) or the formula (VI) in a diluent such as pyridine per 1 mol of the compounds represented by the general formula (Ia) to obtain the desired compounds.

The active compounds according to the invention can be used, for example, in connection with the following plants:

Dicotyledon weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea.

Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.

Monocotyledon weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus and Apera.

Monocotyledon cultures of the genera: Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants. The compounds are suitable, depending on the concentration, for the total combating of weeds, for example on industrial terrain and rail tracks, and on paths and squares with or without tree plantings.

Equally, the compounds can be employed for combating weeds in perennial cultures, for example afforest stations, decorative tree plantings, orchards, vineyards, citrus groves, nut orchards, banana plantations, coffee plantations, tea plantations, rubber plantations, oil palm plantations, cocoa plantations, soft fruit plantings and hop fields, and for the selective combating of weeds in anneal cultures.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in a known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, as as acetone, methyl ethyl ketone, methyl isobutyl-ketone or cyclohexanone, or strongly polar solvents, such as dimethylformamide and dimethylsulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-acid esters, polyoxyethylene-alcohol-ethers, for example alkyl aryl polyglycol ethers, alkyl sulphonates, alkyl sulphates, aryl sulphonates as well as albumin hydrolysis products.

Dispersing agents include, for example, lignin sulphite waste liquors and methyl cellulose. Adhesives such as carboxymethylcellulose and natural and synthetic polymers in the form of powders, granules or lattices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts or iron, manganese boron, copper, cobalt, molybdenum and zinc.

The formulation in general contain from 0.1 to 95 % by weight of active compound, preferably from 0.5 to 90 % by weight.

The active compounds according to the invention, such as in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellents, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomizing or scattering.

The active compounds according to the invention can be applied either before or after emergence of the plants. They can also be incorporated into the soil before sowing. They are used, in particular, after emergence of the plants.

The amount of active compound used can vary within a substantial range. It depends essentially on the nature of the desired effect.

In general, the amounts used are between 0.001 and 10 kg of active compound per hectare of soil surface, preferably between 0.01 and 5 kg per ha.

The preparation and use of the active compounds according to the invention can be seen from the following examples.

**Preparation Examples:**

**Example 1**

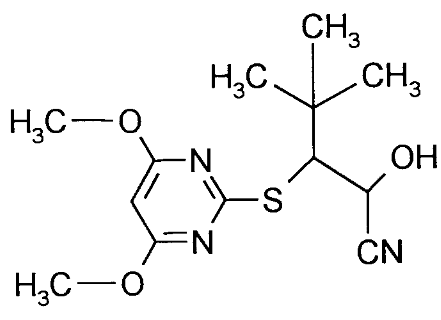

To a solution of 2-(4,6-dimethoxy-2-pyrimidinylthio)-3,3-dimethylbutanal (10 g) in ether (100 ml) was added dropwise at room temperature sodium cyanide (2.8 g) that had been dissolved in water (50 ml), followed by dropwise addition of ammonium chloride (2.8 g) dissolved in water (50 ml) and then vigorous stirring for a period of 4 to 5 hours. After extraction of thus formed organic layer several times with ether, the reaction liquid was dried with anhydrous sodium sulfate, removed from the solvent under reduced pressure distillation, followed by purification of the residue through column chromatography (hexane/ethyl acetate) to give 1-cyano-2-(4,6-dimethoxy-2-pyrimidinylthio)-3,3-dimethyl-1-butanol (10.6 g).
$n_D^{20}$ = 1.5195.

**Example 2**

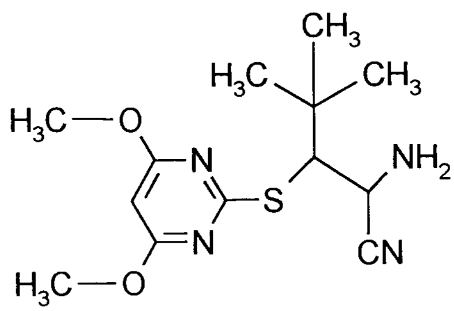

1-Cyano-2-(4,6-dimethoxy-2-pyrimidinylthio)-3,3-dimethyl-1-butanol (3 g) was dissolved in methanol (50 ml), followed by addition of catalytic amount of ammonium chloride thereto, saturation thereof with ammonia gas and a further stirring for a period of 3 to 4 hours at room temperature. From the reaction product was distilled off the solvent under reduced pressure and the resulting residue was purified through column chromatography (hexane/ethyl acetate) to give 1-cyano-2-(4,6-dimethoxy-2-pyrimidinylthio)-3,3-dimethyl-1-butylamine (2.2 g).
mp. 115.5-116.5 °C

**Example 3**

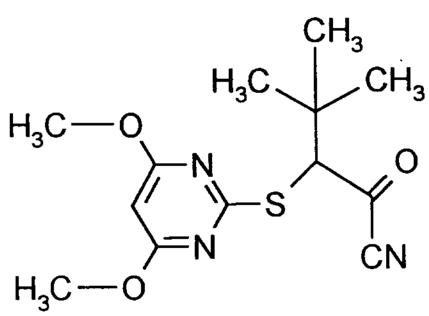

1-Cyano-2-(4,6-dimethoxy-2-pyrimidinylthio)-3,3-dimethyl-1-butanol (4.0 g) was dissolved in acetone (50 ml), followed by slow and dropwise addition of 8N Jones reagent (3.6 ml) at a temperature from 0 to 5 °C and then stirring overnight. To the reaction liquid was added water (100 ml), followed by removal of acetone under reduced pressure distillation, extraction several times with ethyl acetate, drying with anhydrous sodium sulfate and, finally removal of the solvent under distillation. The resulting residue was purified through column chromatography (hexane/ethyl acetate) to give 1-cyano-2-(4,6-dimethoxy-2-pyrimidinylthio)-3,3-dimethyl-1-propionyl cyanide (3.0 g).
mp. 66.5-68 °C

**Example 4**

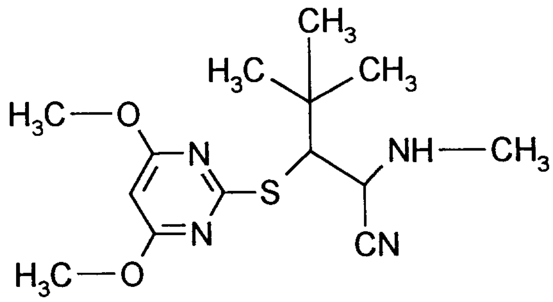

1-Cyano-2-(4,6-dimethoxy-2-pyrimidinylthio)-3,3-dimethyl-1-butanol (5.0 g) was dissolved in methanol (20 ml), followed by addition of a catalytic amount of ammonium chloride thereto, dropwiese addition of methylamine (40% methanol solution, 40 ml) at room temperature. After further stirring for a period of 24 hours, the desired compound crystallized out, which was then separated by filtration, washed with methanol and dried to give 1-cyano-2-(4,6-dimethoxy-2-pyrimidinylthio)-N,3,3-trimethyl-1-butylamine (4.1 g).
mp. 154-155 °C

**Example 5**

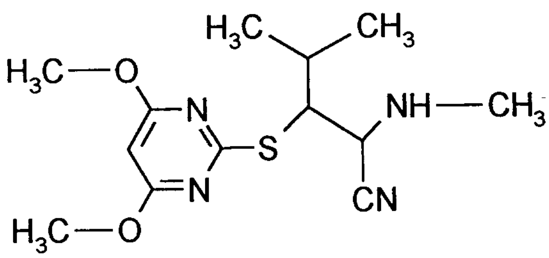

2-(4,6-Dimethoxy-2-pyrimidinylthio)-3-methyl-1-butanol (2.0 g), sodium cyanide (0.5 g) and ammonium chloride (0.5 g) was dissolved in 40% methylamine-methanol (50 ml) and then stirring 15 hours at room temperature. From the reaction product was distilled off the solvent under reduced pressure and the resulting residue was purified through column chromatography (hexane/ethyl acetate) to give 1-cyano-2-(4,6-dimethoxy-1-pyrimidinylthio)-N,3-dimethyl-1-butylamine (1.6 g).
$n_D^{20}$ = 1.5380

**Example 6**

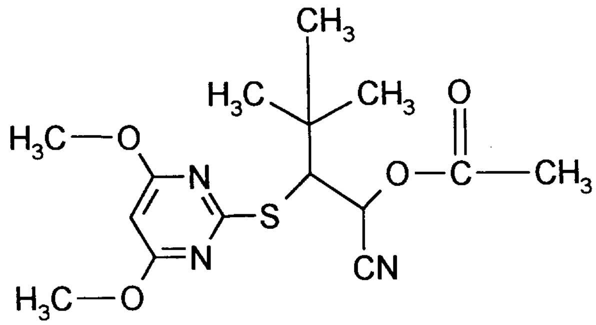

1-Cyano-2-(4,6-dimethoxy-2-pyrimidinylthio)-3,3-dimethyl-butanol (1.0 g) was dissolved in pyridine (20 ml), followed by a dropwise addition of acetic anhydride (10 ml) at room temperature. After 5 hours stirring, the reaction liquid was poured into water (100 ml) and extracted with ethyl acetate. Then the organic layer was washed with 2% hydrochlorid acid, saturated aqueous solution of sodium hydrogen carbonate and water, in this order. After drying with anhydrous sodium sulfate, the reaction liquid was subjected to a reduced pressure distillation to remove the solvent therefrom, and the thus obtained substance was purified by a silica gel column chromatography to give 1-cyano-2-(4,6-dimethoxy-2-pyrimidinylthio)-3,3-dimethyl-1-butyl acetate (0.9 g).
mp. 101-103.5 °C

**Example 7**

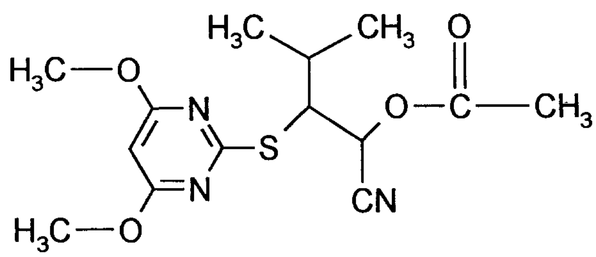

1-Cyano-2-(4,6-dimethoxy-2-pyrimidinylthio)-3-methyl-butanol (1.0 g) was dissolved in pyridine (20 ml), followed by a dropwise addition of acetic anhydride (1 g) at room temperature. After 5 hours stirring, the reaction liquid was poured into water (100 ml) and extracted with ethyl acetate. Then the organic layer was first washed with 10% hydrochloric acid and then with water.

After drying with anhydrous sodium sulfate, the reaction liquid was subjected to a reduced pressure distillation to remove the solvent therefrom, and the thus obtained substance was purified by a silica gel column chromatography to give 1-cyano-2-(4,6-dimethoxy-2-pyrimidinylthio)-3-methyl-1-butyl acetate (0.9 g).
$n_D^{20}$ = 1.5193

**Example 8**

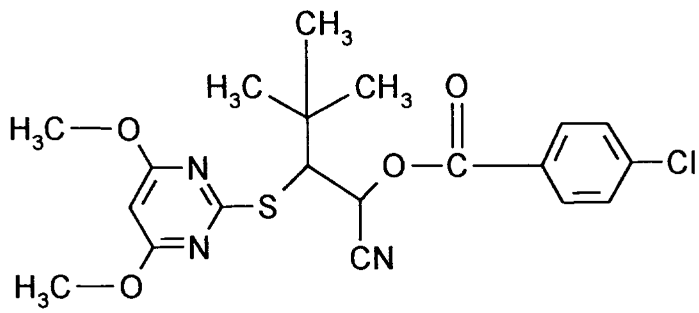

To 1-cyano-2-(4,6-dimethoxy-2-pyrimidinylthio)-3,3-dimethylbutanol (1.0 g) was added dropwise a solution of 4-chlorobenzoyl chloride (0.65 g) in methylene chloride (50 ml), followed by a dropwise addition of triethylamine (0.37 g) at a temperature from 0 to 5 ° C. The resulting solution, after a five-hour stirring, was washed with water. After drying with anhydrous sodium sulfate, the reaction liquid was subjected to a reduced pressure distillation to remove the solvent therefrom, and the thus obtained substance was purified by a silica gel column chromatography (hexane/ethyl acetate) to give 1-cyano-2-(4,6-dimethoxy-2-pyrimidinylthio)-3,3-dimethyl-butyl-4-chlorobenzoate (0.9 g).
mp. 83-87 ° C

**Example 9**

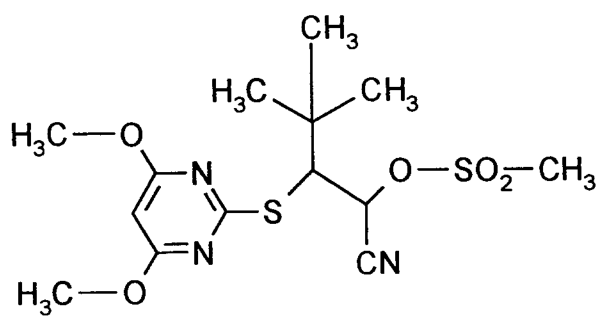

1-Cyano-2-(4,6-dimethoxy-2-pyrimidinylthio)-3,3-dimethylbutanol (1.5 g) was dissolved in pyridine (20 ml), followed by a dropwise addition of methanesulfonyl chloride (1.2 g) thereto at from 0 to 5 ° C. The mixture was stirred for 1 hour while maintaining the above mentioned temperature, and then the mixture was further stirred for 5 to 6 hours at room temperature. After the completion of reaction, water was added to the mixture, followed by extraction with ethyl acetate. The organic layer was washed with 2% hydrochloric acid, a saturated aqueous solution of sodium hydrogen carbonate and water, in that order. The resulting mixture, after drying with anhydrous sodium sulfate, was removed from the solvent under a reduced pressure distillation and the obtained residue was purified through column chromatography (hexane/ethyl acetate) to give 2-methane-sulfonyloxy-3-(4,6-dimethoxy-2-pyrimidinylthio) valeronitrile (1.6 g).
mp. 101-103 ° C

In the following Table 2 are shown the compounds that were prepared likewise the above mentioned Synthesis Examples 1 to 9, together with those which were actually prepared in the same Examples.

Table 2

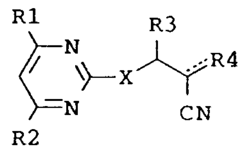

| Compound No. | R1 | R2 | X | R3 | R4 | Physical constant |
|---|---|---|---|---|---|---|
| 1 | $OCH_3$ | $OCH_3$ | S | $C(CH_3)_3$ | OH | $n_D^{20}$ 1.5195 |
| 2 | $OCH_3$ | $OCH_3$ | S | $C(CH_3)_3$ | $NH_2$ | mp. 115.5～116.5℃ |
| 3 | $OCH_3$ | $OCH_3$ | S | $C(CH_3)_3$ | O | mp. 66.5～68℃ |
| 4 | $OCH_3$ | $OCH_3$ | O | $C(CH_3)_3$ | O | mp. 83～86.5℃ |
| 5 | $OCH_3$ | $OCH_3$ | S | $CH(CH_3)_2$ | OH | $n_D^{20}$ 1.5197 |
| 6 | $OCH_3$ | $OCH_3$ | O | $C(CH_3)_3$ | OH | mp. 127～129.5℃ |
| 7 | $OCH_3$ | $OCH_3$ | S | $CH(CH_3)_2$ | $NH_2$ | $n_D^{20}$ 1.5426 |
| 8 | $OCH_3$ | $OCH_3$ | O | $C(CH_3)_3$ | $NH_2$ | mp. 119～121℃ |
| 9 | $OCH_3$ | $OCH_3$ | S | $C(CH_3)_3$ | $NHCH_3$ | mp. 154～155℃ |
| 10 | $OCH_3$ | $OCH_3$ | S | $CH(CH_3)_2$ | $NHCH_3$ | $n_D^{20}$ 1.5380 |
| 11 | $OCH_3$ | $OCH_3$ | S | $C(CH_3)_3$ | $NHCH_2CH_2CH_3$ | $n_D^{20}$ 1.5239 |
| 12 | $OCH_3$ | $OCH_3$ | O | $C(CH_3)_3$ | $NHCH_3$ | mp. 78～81℃ |
| 13 | $OCH_3$ | $OCH_3$ | O | $C(CH_3)_3$ | 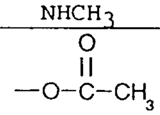 | mp. 101～103.5℃ |
| 14 | $OCH_3$ | $OCH_3$ | O | $C(CH_3)_3$ | 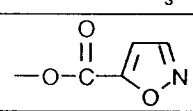 | $n_D^{20}$ 1.4847 |
| 15 | $OCH_3$ | $OCH_3$ | O | $C(CH_3)_3$ | 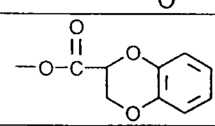 | $n_D^{20}$ 1.5109 |
| 16 | $OCH_3$ | $OCH_3$ | S | $CH(CH_3)_2$ | 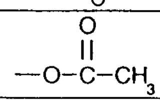 | $n_D^{20}$ 1.5193 |
| 17 | $OCH_3$ | $OCH_3$ | S | $C(CH_3)_3$ | 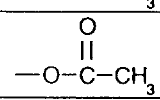 | mp. 84～88℃ |

Table 2 (continued)

| Compound No. | R1 | R2 | X | R3 | R4 | Physical constant |
|---|---|---|---|---|---|---|
| 18 | $OCH_3$ | $OCH_3$ | S | $C(CH_3)_3$ | 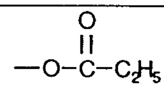 | $n_D^{20}$ 1.5066 |
| 19 | $OCH_3$ | $OCH_3$ | S | $C(CH_3)_3$ | 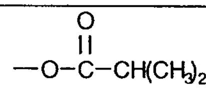 | $n_D^{20}$ 1.5005 |
| 20 | $OCH_3$ | $OCH_3$ | S | $C(CH_3)_3$ | 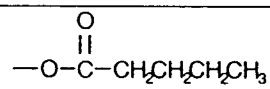 | $n_D^{20}$ 1.5062 |
| 21 | $OCH_3$ | $OCH_3$ | S | $C(CH_3)_3$ | 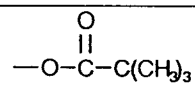 | $n_D^{20}$ 1.5033 |
| 22 | $OCH_3$ | $OCH_3$ | S | $C(CH_3)_3$ | 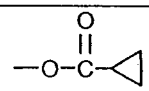 | $n_D^{20}$ 1.5619 |
| 23 | $OCH_3$ | $OCH_3$ | S | $C(CH_3)_3$ | 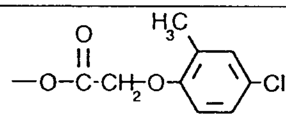 | $n_D^{20}$ 1.5363 |
| 24 | $OCH_3$ | $OCH_3$ | S | $C(CH_3)_3$ | 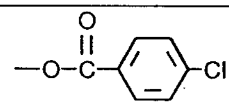 | mp. 83～87℃ |
| 25 | $OCH_3$ | $OCH_3$ | S | $C(CH_3)_3$ | 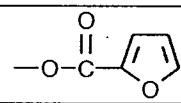 | mp. 95～97℃ |
| 26 | $OCH_3$ | $OCH_3$ | S | $C(CH_3)_3$ | 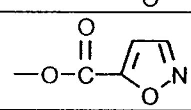 | mp. 144～148℃ |
| 27 | $OCH_3$ | $OCH_3$ | S | $C(CH_3)_3$ | 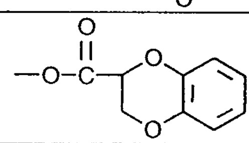 | $n_D^{20}$ 1.5302 |
| 28 | $OCH_3$ | $OCH_3$ | S | $C(CH_3)_3$ | 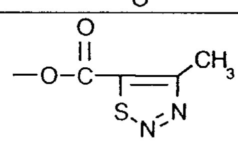 | mp. 131～135℃ |
| 29 | $OCH_3$ | $OCH_3$ | S | $C(CH_3)_3$ | 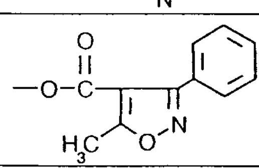 | $n_D^{20}$ 1.5370 |
| 30 | $OCH_3$ | $OCH_3$ | S | $C(CH_3)_3$ | 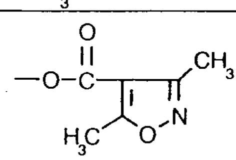 | mp. 153～154℃ |

Table 2 (continued)

| Compound No. | R1 | R2 | X | R3 | R4 | Physical constant |
|---|---|---|---|---|---|---|
| 31 | $OCH_3$ | $OCH_3$ | S | $C(CH_3)_3$ | $-O-SO_2-CH_3$ | mp. 101~103℃ |
| 32 | $OCH_3$ | $OCH_3$ | S | $C(CH_3)_3$ | 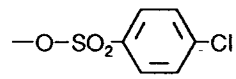 | $n_D^{20}$ 1.5418 |
| 33 | $OCH_3$ | $OCH_3$ | S | $C(CH_3)_3$ | 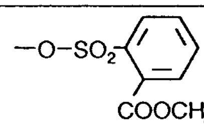 | $n_D^{20}$ 1.5320 |
| 34 | $OCH_3$ | $OCH_3$ | S | $C(CH_3)_3$ | 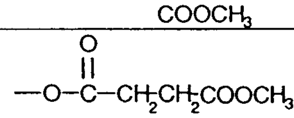 | $n_D^{20}$ 1.5111 |
| 35 | $OCH_3$ | $OCH_3$ | S | $C(CH_3)_3$ | 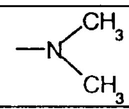 | mp. 109~110℃ |
| 36 | $OCH_3$ | $OCH_3$ | S | $C(CH_3)_3$ | 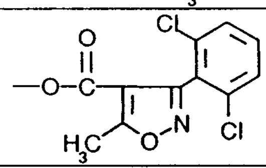 | mp. 62~66℃ |
| 37 | $OCH_3$ | $OCH_3$ | S | $C(CH_3)_3$ | 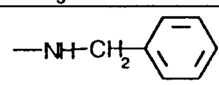 | mp. 111~112℃ |
| 38 | $OCH_3$ | $OCH_3$ | S | $C(CH_3)_3$ | 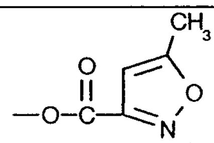 | mp. 126~130℃ |
| 39 | $OCH_3$ | $OCH_3$ | S | $C(CH_3)_3$ | 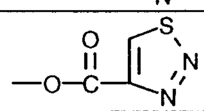 | mp. 151~153℃ |
| 40 | $OCH_3$ | $OCH_3$ | S | $C(CH_3)_3$ | 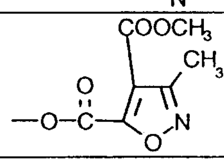 | mp. 112~115℃ |

**Biological test:**

**Test Example 1: Pre-emergence soil treatment test on upland weeds**

Formulation of Active Compounds

| | |
|---|---|
| Carrier: | 5 part by weight of acetone |
| Emulsifier: | 1 part by weight of benzyloxy polyglycol ether |

To produce a suitable formulation of each of the active compounds, 1 part by weight of the active compound was mixed with stated amount of carrier and with the stated amount of emulsifier, and the resulting emulsifiable concentrate was diluted with water to the desired concentration.

Test Procedures

In a greenhouse, a number of test pots each having an area of 120 $cm^2$ were charged with soil taken out from cultivated field. Onto the soil surface in the respective test pots were sown the seeds of barnyard grass (Echinochloa crus-gall) and redroot pigweed (Amaranthus blitum), respectively, followed by soil covering thereon. Thereafter, predetermined dosages of the active compound formulations mentioned above were uniformly sprayed onto the soil surface of the respective test pots.

Four weeks after the application of the active compound formulations, the degrees of herbicidal effect on the weeds were determined. The herbicidal effect was rated as follows:

| | |
|---|---|
| Completely killed weeds | 100% |
| status equivalent to non-treated pots | 0%. |

In the present test, each of the above mentioned compound Nos. 1, 2, 3, 4, 11, 14, 16, 18, 19, 20, 21, 28, 34, 37 and 38 according to the present invention exhibit a 100% herbicidal effect on barnyard grass and redroot pigweed at a dosage of 1.0 kg/ha.

**Test example 2: Post-emergence foliage treatment on upland weeds**

Test Procedures

In a greenhouse, a number of test pots each having an area of 120 $cm^2$ were charged with soil taken out from a cultivated field. Seeds of barnyard grass and redroot pigweed, respectively, were sown onto the soil surface in the respective test pots, followed by cultivation for ten days. Thereafter, predetermined dosages of the active compound formulations, prepared as in the test example 1 mentioned above were uniformly sprayed onto the foliage portions of the test weeds in the respective test pots.

Three weeks after the application of the active compound formulations, the degrees of herbicidal effect on the weeds were determined.

In the present test, each of the above-mentioned compounds 1, 3, 4, 11, 12, 26, 28, 38 and 39 according to the present invention exhibited a 100% herbicidal effect on barnyard grass and redroot pigweed at a dosage of 1.0 kg/ha.

**Claims**

**1.** Pyrimidine derivatives containing a cyano group of the formula (I)

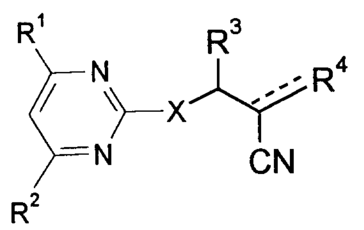

(I)

wherein

| | |
|---|---|
| X | represents oxygen or sulfur, |
| $R^1$ | represents halogen, $C_{1-4}$ alkyl, $C_{1-4}$ alkoxy, halogeno-$C_{1-4}$ alkyl, halogeno-$C_{1-4}$ alkoxy, |
| $R^2$ | represents halogen, $C_{1-4}$ alkoxy, halogeno-$C_{1-4}$ alkyl, halogeno-$C_{1-4}$ alkoxy, |
| $R^3$ | represents in each case optionally $C_{1-4}$ substituted or unsubstituted alkyl, or $C_{3-7}$ cycloalkyl, |
| ---- | represents a single bond or a double bond, |
| $R^4$ | represents hydroxy, oxo or one of the following groups: |

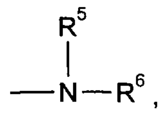

$-O-SO_2-R^7$ or

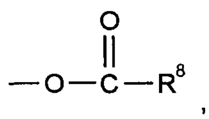

$R^5$ and $R^6$ each represent independently from each other hydrogen, or in each case optionally substituted or unsubstituted $C_{3-8}$ cycloalkyl or $C_{2-8}$ alkenyl or

$R^5$ and $R^6$ together with the nitrogen atom to which they are bonded may form a 5- or 6-membered heterocyclic ring,

$R^7$ represents in each case optionally substituted or unsubstituted $C_{1-8}$ alkyl, $C_{2-8}$ alkenyl, phenyl or a heterocyclic ring,

and

$R^8$ represents hydrogen, or in each case optionally substituted or unsubstituted $C_{1-20}$ alkyl, $C_{2-20}$ alkenyl, $C_{2-20}$ alkynyl, phenyl, a heterocyclic ring, a condensed heterocyclic ring or $C_{3-8}$ cycloalkyl.

**2.** Pyrimidine derivatives containing a cyano group of the formula (I), according to claim 1, wherein

X represents oxygen or sulfur,

$R^1$ represents halogen, $C_{1-3}$ alkyl, $C_{1-3}$ alkoxy, halogeno-$C_{1-3}$ alkyl or halogeno-$C_{1-3}$ alkoxy,

$R^2$ represents halogen, $C_{1-3}$ alkoxy, halogeno-$C_{1-3}$ alkyl or halogeno-$C_{1-3}$ alkoxy, $R^3$ represents $C_{2-4}$ alkyl, or $C_{3-6}$ cycloalkyl which may be substituted by $C_{1-3}$ alkyl,

---- represents a single bond or double bond,

$R^4$ represents hydroxy, oxo or one of the following groups:

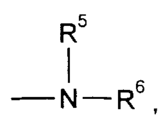

$-O-SO_2-R^7$ or

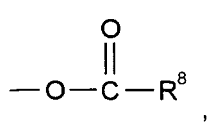

$R^5$ and $R^6$ each represent independently from each other hydrogen, and in each case optionally substituted or unsubstituted $C_{1-6}$ alkyl, $C_{1-4}$ alkoxy-$C_{1-4}$ alkyl, $C_{3-8}$ cycloalkyl-$C_{1-4}$ alkyl, $C_{1-4}$ alkyl-$C_{3-8}$ cycloalkyl-$C_{1-4}$ alkyl, $C_{3-8}$ cycloalkyl, $C_{3-8}$ cycloalkyl-$C_{1-4}$ alkyl, $C_{2-6}$ alkenyl, or phenyl-$C_{1-4}$ alkyl; the substituent(s) being selected from the group consisting of halogen, $C_{1-4}$ alkyl, halogeno-$C_{1-4}$ alkyl and halogeno-$C_{1-4}$ alkoxy, or

$R^5$ and $R^6$ may form a pyrrolidine, piperidine or morpholine together with the nitrogen to which they are bonded,

$R^7$ represents in each case optionally substituted or unsubstituted $C_{1-4}$ alkyl, halogeno-$C_{1-4}$ alkyl, $C_{1-4}$ alkyl-carbonyl-$C_{1-4}$ alkyl, $C_{2-8}$ alkenyl, phenyl, the substituent(s) being selected from the group consisting of halogen, nitro, $C_{1-4}$ alkyl, halogeno-$C_{1-4}$

alkyl, $C_{1-4}$ alkoxy, halogeno-$C_{1-4}$ alkoxy and $C_{1-4}$ alkoxycarbonyl,

$R^7$ further represents an optionally substituted 5- or 6-membered heterocyclic ring, the hetero atom(s) of said heterocyclic ring being selected from the group consisting of oxygen, sulfur and nitrogen, and the substituent(s) being selected from the group consisting of halogen, $C_{1-4}$ alkyl, $C_{1-4}$ alkoxy or $C_{1-4}$ alkoxycarbonyl,

and

$R^8$ represents hydrogen, $C_{1-6}$ alkyl, halogeno-$C_{1-6}$ alkyl, cyano-$C_{1-6}$ alkyl, carboxyl-$C_{1-6}$ alkyl, $C_{1-4}$ alkoxy-$C_{1-4}$ alkyl, $C_{1-4}$ alkyl-carbonyl-$C_{1-4}$ alkyl, or $C_{1-4}$ alkoxycarbonyl-$C_{1-4}$ alkyl,

$R^8$ further represents in each case optionally substituted or unsubstituted phenyl-$C_{1-4}$ alkyl, phenoxy-$C_{1-4}$ alkyl or phenylthio-$C_{1-4}$ alkyl, the substituent(s) being selected from the group consisting of halogen, $C_{1-4}$ alkyl and halogeno-$C_{1-4}$ alkyl,

$R^8$ further represents optionally substituted or unsubstituted $C_{2-6}$ alkenyl, the substituent(s) being selected from the group consisting of halogen, carboxyl or its salt, phenyl halogeno-phenyl and $C_{1-4}$ alkyl-phenyl,

$R^8$ further represents in each case optionally substituted or unsubstituted $C_{2-6}$ alkynyl or phenyl, the substituent(s) being selected from the group consisting of nitro, halogen, $C_{1-4}$ alkyl, $C_{1-4}$ alkoxy, $C_{1-4}$ alkylthio, halogeno-$C_{1-4}$ alkyl and halogeno-$C_{1-4}$ alkylthio,

$R^8$ further represents in each case an optionally substituted 5- or 6-membered heterocyclic ring or 5.6- or 6.6-condensed heterocyclic ring, the hetero atom(s) of said heterocyclic ring(s) being selected from the group consisting of oxygen, sulfur and nitrogen, the substituent(s) being selected from the group consisting of halogen, $C_{1-4}$ alkyl, $C_{1-4}$ alkoxy, $C_{1-4}$ alkylthio, halogeno-$C_{1-4}$ alkyl, phenyl, halogeno-phenyl, $C_{1-4}$ alkyl-phenyl and carboxyl or its salts, or

$R^8$ further represents $C_{3-8}$ cycloalkyl which may optionally be substituted by $C_{1-4}$ alkyl.

**3.** Pyrimidine derivatives containing a cyano group of the formula (I) according to claim 1 wherein

X represents oxygen or sulfur,

$R^1$ represents methoxy,

$R^2$ represents methoxy,

$R^3$ represents $C_{2-4}$ alkyl or $C_{3-6}$ cycloalkyl which may be substituted by methyl,

---- represents single bond or double bond,

$R^4$ represents hydroxy, oxo or one of the following groups:

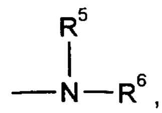

-O-$SO_2$-$R^7$ or

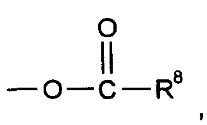

$R^5$ and $R^6$ each represent independently from each other hydrogen, or in each case optionally substituted or unsubstituted $C_{1-4}$ alkyl, methoxy-$C_{1-3}$ alkyl, cyclopropyl-$C_{1-3}$ alkyl, methyl-cyclopropyl-$C_{1-3}$ alkyl, cyclopentyl-$C_{1-3}$ alkyl, methyl-cyclopentyl-$C_{1-3}$ alkyl, cyclohexyl-$C_{1-3}$ alkyl, methyl-cyclohexyl-$C_{1-3}$ alkyl, cyclopropyl, methyl-cyclopropyl, cyclopentyl, methyl-cyclopentyl, cyclohexyl, methyl-cyclohexyl, $C_{2-4}$ alkenyl, or phenyl-$C_{1-3}$ alkyl, the subsitutent(s) being selected from the group consisting of fluoro, chloro, bromo, methyl, butyl, trifluoromethyl, methoxy and trifluoromethoxy, or

$R^5$ and $R^6$ may form a pyrrolidine, piperidine or morpholine together with the nitrogen to which they are bonded,

$R^7$ represents in each case optionally substituted or unsubstituted $C_{1-4}$ alkyl, or $C_{2-8}$ alkenyl, the substituent(s) being selected from the group consisting of fluoro, chloro, bromo and methoxy-carbonyl, or phenyl being optionally substituted by one of the

group consisting of fluoro, chloro, bromo, nitro, $C_{1-4}$ alkyl, halogeno-$C_{1-4}$ alkyl, methoxy, trifluoromethoxy and methoxy-carbonyl,

$R^7$ further represents in each case an optionally substituted thienyl, thiazolyl, imidazolyl, pyrazolyl, furyl, pyridyl, pyrimidinyl, pyridazinyl, pyrazinyl, isoxazolyl and oxazolyl, the substitutent(s) being selected from the group consisting of fluoro, chloro, bromo, methyl and methoxy-carbonyl,

and

$R^8$ represents hydrogen, $C_{1-4}$ alkyl, halogeno-$C_{1-4}$ alkyl, cyano-$C_{1-4}$ alkyl, carboxyl-$C_{1-4}$ alkyl, methoxy-$C_{1-4}$ alkyl, methylcarbonyl-$C_{1-4}$ alkyl, or methoxycarbonyl-$C_{1-4}$ alkyl,

$R^8$ further represents in each case optionally substituted phenyl-$C_{1-4}$ alkyl, phenoxy-$C_{1-4}$ alkyl or phenylthio-$C_{1-4}$ alkyl, the subsitutent(s) of those phenyl rings being selected from the group consisting of fluoro, chloro, bromo, $C_{1-4}$ alkyl and trifluoromethyl,

$R^8$ further represents optionally substituted $C_{2-6}$ alkenyl, the substituent(s) being selected from the group consisting of fluoro, chloro, bromo, carboxyl or its sodium salt and phenyl which may be substituted by fluoro, chloro, bromo or methyl,

$R^8$ further represents $C_{2-6}$ alkynyl, optionally substituted phenyl, the substituent(s) being selected from the group consisting of nitro, fluoro, chloro, bromo, methyl, ethyl, methoxy, methylthio, trifluoromethyl and trifluoromethoxy,

$R^8$ further represents in each case an optionally substituted thienyl, thiazolyl, imidazolyl, pyrazolyl, furyl, pyridyl, pyrimidinyl, pyridazinyl, pyrazinyl, isoxalyl, oxazolyl, quinolyl and indolinyl, the substituent(s) being selected from the group consisting of fluoro, chloro, bromo, methoxy, methylthio, trifluoromethyl, carboxyl, phenyl, halogeno-phenyl and $C_{1-4}$ alkylphenyl,

$R^8$ further represents cyclopropyl, methyl-cyclopropyl, cyclopenthyl, methyl-cyclopenthyl, cyclohexyl, methyl-cyclohexyl.

**4.** Pyrimidine derivatives containing a cyano group of the formula (I)
wherein $R^1$, $R^2$, $R^3$, $R^4$, and X have the meanings as described in claim 1, characterized in that
(a) in the case where $R^4$ represents hydroxy:
compounds of the formula (II)

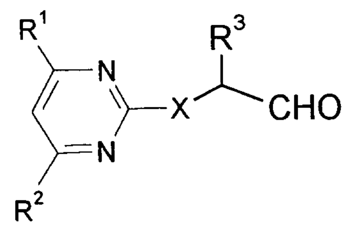

(II)

wherein
$R^1$, $R^2$ and $R^3$ have the above mentioned meanings,
are reacted with sodium cyanide or potassium cyanide,
in the presence of an acid, and if appropriate, in the presence of inert solvents,

or

(b) in the case where $R^4$ represents oxo:
compounds of the formula (Ia)

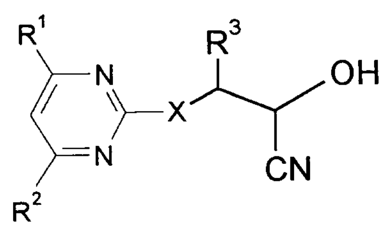

(Ia)

wherein $R^1$, $R^2$ and $R^3$ have the above mentioned meanings,
are reacted with an oxidizing agent,
if appropriate, in the presence of inert solvents,

or

(c) in the case where $R^4$ represents

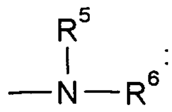

the compounds of the formula (Ia)
are reacted with a compound of the following formula (III)

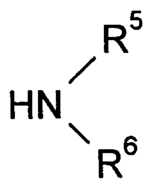 (III)

wherein $R^5$ and $R^6$ have the above mentioned meanings,
if appropriate, in the presence of inert solvents,

or

(d) in the case where $R^4$ represents $-O-SO_2-R^7$:
the compounds of the formula (Ia)
are reacted with a compound of the formula (IV)

$R^7-SO_2-Y$ (IV)

wherein $R^7$ represents the same meanings as mentioned above, and Y represents chlorine or bromine,
if appropriate, in the presence of an acid binder, and if appropriate, in the presence of inert solvents,

or

(e) in the case where $R^4$ represents

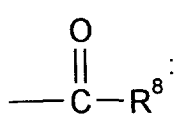

the compounds of the formula (Ia)
are reacted with the compound of the following formula (V)

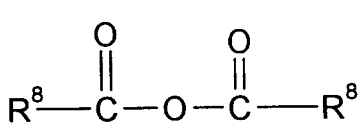 (V)

wherein $R^8$ has the same meanings as mentioned above,
or are reacted with the compounds of the following formula (VI)

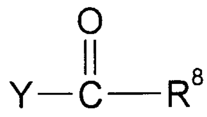 (VI)

wherein $R^8$ and Y represent the same meanings as mentioned above,
if appropriate, in the presence of an acid binder, and if appropriate, in the presence of inert solvents.

**5.** Herbicidal compositions, characterized in that they contain at least one pyrimidine derivative containing a cyano group of the formula (I).

**6.** Process for combating weeds, characterized in that pyrimidine derivatives containing a cyano group of the formula (I) are allowed to act on weeds and/or their habitat.

**7.** Use of pyrimidine derivatives containing a cyano group of the formula (I) for combating weeds.

**8.** Process for the preparation of herbicidal compositions, characterized in that pyrimidine derivatives containing a cyano group of the formula (I) are mixed with extenders and/or surface active agents.